(19) Europäisches Patentamt
European Patent Office
Office européen des brevets

(11) **EP 2 282 770 B1**

(12) **EUROPEAN PATENT SPECIFICATION**

(45) Date of publication and mention
of the grant of the patent:
**07.03.2018 Bulletin 2018/10**

(21) Application number: **09814929.7**

(22) Date of filing: **04.06.2009**

(51) Int Cl.:
*C07K 16/00* (2006.01)   *A61K 39/395* (2006.01)

(86) International application number:
**PCT/US2009/046214**

(87) International publication number:
**WO 2010/033279 (25.03.2010 Gazette 2010/12)**

(54) **ANTIBODIES WITH ALTERED BINDING TO FCRN AND METHODS OF USING SAME**

ANTIKÖRPER MIT VERÄNDERTER BINDUNG AN FCRN UND VERWENDUNGSVERFAHREN DAFÜR

ANTICORPS À LIAISON ALTEREE À FCRN ET LEURS PROCEDES D'UTILISATION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **04.06.2008 US 58658 P**

(43) Date of publication of application:
**16.02.2011 Bulletin 2011/07**

(73) Proprietor: **MacroGenics, Inc.**
**Rockville, MD 20850 (US)**

(72) Inventor: **GORLATOV, Sergey**
**Gaithersburg, MD 20877 (US)**

(74) Representative: **Atkinson, Jennifer et al**
**Barker Brettell LLP**
**100 Hagley Road**
**Edgbaston**
**Birmingham B16 8QQ (GB)**

(56) References cited:
WO-A1-2007/080277   WO-A2-2006/053301
CN-A- 1 958 615   FR-A1- 2 894 982
US-A1- 2006 134 105   US-A1- 2007 041 907
US-A1- 2007 148 164   US-A1- 2008 112 961

- SHIELDS R L ET AL: "High resolution mapping of the binding site on human IgG1 for Fc gamma RI, Fc gamma RII, Fc gamma RIII and FcRn and design of IgG1 variants with improved binding to the Fc gamma R", JOURNAL OF BIOLOGICAL CHEMISTRY, AMERICAN SOCIETY OF BIOLOGICAL CHEMISTS, BALTIMORE, MD, US,, vol. 276, no. 9, 2 March 2001 (2001-03-02), pages 6591-6604, XP002638208,
- VACCARO CARLOS ET AL: "Divergent activities of an engineered antibody in murine and human systems have implications for therapeutic antibodies", PROCEEDINGS OF THE NATIONAL ACADEMY OF SCIENCES, NATIONAL ACADEMY OF SCIENCES, WASHINGTON, DC; US, vol. 103, no. 49, 5 December 2006 (2006-12-05), pages 18709-18714, XP002518923, ISSN: 0027-8424, DOI: 10.1073/PNAS.0606304103
- STAVENHAGEN J B ET AL: "Enhancing the potency of therapeutic monoclonal antibodies via Fc optimization", ADVANCES IN ENZYME REGULATION, PERGAMON PRESS, OXFORD, GB, vol. 48, no. 1, 1 January 2008 (2008-01-01), pages 152-164, XP025407108, ISSN: 0065-2571, DOI: 10.1016/J.ADVENZREG.2007.11.011 [retrieved on 2007-12-03]
- WEST A P ET AL: "Crystal structure and immunoglobulin G binding properties of the human major histocompatibility complex-related Fc receptor", BIOCHEMISTRY, AMERICAN CHEMICAL SOCIETY, US, vol. 39, no. 32, 15 August 2000 (2000-08-15), pages 9698-9708, XP002300287, ISSN: 0006-2960, DOI: 10.1021/BI000749M

**(Cont. next page)**

- MEDESAN C ET AL: "Delineation of the amino acid residues involved in transcytosis and catabolism of mouse IgG1", THE JOURNAL OF IMMUNOLOGY, THE AMERICAN ASSOCIATION OF IMMUNOLOGISTS, US, vol. 158, no. 5, 1 March 1997 (1997-03-01) , pages 2211-2217, XP002532767, ISSN: 0022-1767
- PAUL R HINTON ET AL: "An engineered human IgG1 antibody with longer serum half-life", THE JOURNAL OF IMMUNOLOGY, THE AMERICAN ASSOCIATION OF IMMUNOLOGISTS, US, vol. 176, no. 1, 1 January 2006 (2006-01-01), pages 346-356, XP002484005, ISSN: 0022-1767
- GESTUR VIDARSSON ET AL: "IgG Subclasses and Allotypes: From Structure to Effector Functions", FRONTIERS IN IMMUNOLOGY, vol. 5, 20 October 2014 (2014-10-20), XP055166978, DOI: 10.3389/fimmu.2014.00520

## Description

## Background of the Invention:

### Field of the Invention:

This invention relates to antibodies with altered binding to FcRn, and particularly antibodies having enhanced binding to FcRn and/or enhanced serum half-lives.

### Description of Related Art:

The interaction of antibody-antigen complexes with cells of the immune system results in a wide array of responses, ranging from effector functions such as antibody-dependent cytotoxicity, mast cell degranulation, and phagocytosis to immunomodulatory signals such as regulating lymphocyte proliferation and antibody secretion. All these interactions are initiated through the binding of the Fc domain of antibodies or immune complexes to Fc receptors, which are specialized cell surface receptors on hematopoietic cells. The diversity of cellular responses triggered by antibodies and immune complexes results from the structural heterogeneity of Fc receptors. Fc receptors share structurally related ligand binding domains which presumably mediate intracellular signaling.

### I. FcRn

FcRn was first identified in the neonatal rat gut where it functions to mediate the absorption of IgG antibody from the mother's milk and facilitates its transport to the circulatory system. (Leach et al. (1996) J. Immunology 157:3317). FcRn has also been isolated from the human placenta where it mediates absorption and transport of maternal IgG to the fetal circulation. In adults, FcRn is expressed in epithelial tissue such as the pulmonary airways and nasal surfaces (Israel et al. (1997) Immunology 92:69), intestinal and renal proximal tubular epithelium (Kobayashi et al. (2002) Renal Physiol. 282:F358), as well as vaginal, colonic, rectal, and biliary tree surfaces. FcRn is functionally active in adult epithelial cells of diverse origin such as intestinal, bronchial and kidney epithelium, and is capable of transporting IgG and their bound antigens across bronchial epithelium and the intestinal wall. (Spiekerman et al. (2002) J. Exp. Med. 196(3):303-310; Yoshida et al. (2004) Immunity 20:769-783; Bitonti et al. (2004) Proc. Natl. Acad. Sci. USA 101:9763-9768). The ubiquitous expression of FcRn on endothelial cells is suggestive of its importance in IgG homeostasis. (Ward et al. (2002) International Immunology 15(2):187; Ghetie et al. (1996) Eur. J. Immunology 26:690).

FcRn is able to carry out these diverse roles through the transport and recycling of bound IgG within and across cells. Antibodies are normally internalized from circulation by endothelial cells (via pinocytosis) and are targeted to the acidic endosomes and lysosomes of the cells for degradation. FcRn is capable of binding the Fc region of an antibody at the acidic pH of an endosome (<6.5), fusing with the endothelial cell membrane, and releasing the antibody at the neutral pH of the bloodstream ($\sim$7.3-7.5), thereby salvaging the antibody for further use. (Junghans and Anderson (1996) Proc. Natl. Acad. Sci. USA 93:5512; Roopenian et al. (2003) J. Immunology 170:3528). When serum antibody levels decrease, more FcRn molecules are available for IgG binding so that an increased amount of IgG is salvaged. Conversely, if serum IgG levels rise, FcRn becomes saturated, thereby increasing the proportion of antibody that is internalized and degraded (Ghetie and Ward (2000) Annu. Rev. Immunol. 18:739-66). Trans-intestinal transport mediated by FcRn works similarly, in that FcRn on the lumenal side of the intestinal epithelium binds IgG at acidic pH (6.0-6.5), transports IgG across the cell to the basolateral surface, and releases IgG into the neutral bloodstream ($\sim$pH 7.3-7.5). (Raghavan et al. (1994) Immunity 1(4):303-15; Raghavan et al. (1995) Biochemistry 34(45):14649-57).

The FcRn receptor has been isolated from several mammalian species including humans. The sequences of the human FcRn, monkey FcRn rat FcRn, and mouse FcRn are known (Story et al. (1994) J. Exp. Med. 180:2377). FcRn structurally resembles polypeptides of Major Histocompatibility Complex (MHC) Class I. (Ghetie and Ward (1997) Immunology Today 18(12):592-8). FcRn is a heterodimer composed of two polypeptide chains: a light $\beta_2$microglobulin ($\beta_2$m) chain, and a non-covalently bound heavy $\alpha$ chain. The $\beta_2$m chain of FcRn is also a component of MHC I. The FcRn $\alpha$ chain is a 46 kD protein composed of an extracellular domain divided into three subdomains ($\alpha$1, $\alpha$2, and $\alpha$3), a transmembrane region, and a relatively short cytoplasmic tail. (Burmeister et al. (1994) Nature 372:336). FcRn has a version of the MHC peptide binding groove, but the groove is occluded and does not function in antibody-FcRn binding. (Simister and Mostov (1989) Nature 337:184-7).

FcRn binds to antibodies via interaction of the FcRn extracellular domain with sites in the Fc region of antibodies, particularly near the interface of the CH2 and CH3 regions. (Raghavan et al. (1994) Immunity 1:303-15). The FcRn-binding site of the Fc region is also the site at which the bacterial proteins A and G bind. (Martin et al. (2001) Mol Cell 7:867-877; Sauer-Eriksson et al. (1995) Structure 3:265-278; Tashiro et al. (1995) Curr Opin Struct Biol 5:471-481). Crystallographic studies have confirmed that each Fc region of an antibody chain can bind to an FcRn molecule, and therefore a whole IgG antibody is able to complex with two FcRn molecules at a time. (Burmeister et al. (1994) Nature 372:336-379).

Various site-specific mutagenesis experiments in the Fc region of mouse IgGs have led to identification of certain critical amino acid residues involved in the interaction between IgG and FcRn. (Kim et al. (1994) Eur. J. Immunol. 24:2429-2434; Medesan et al. (1996) Eur. J. Immunol. 26:2533; Medesan et al. (1997) J. Immunol. 158:2211-2217). These studies and sequence comparison studies found that isoleucine at position 253, histidine at position 310, and histidine at position 435 (all Kabat numbering) are highly conserved in human and rodent IgGs, and that variation in these residues disrupts Fc-FcRn binding, and thus results in antibodies with much shorter serum half-lives than wild-type IgG. Other studies have shown that increased binding affinity for FcRn leads to increases in the serum half-life of the molecule. (Kim et al. (1994) Eur. J. Immunol. 24:2429-2434; Popov et al. (1996) Mol. Immunol. 33:493-502; Ghetie et al. (1996) Eur. J. Immunol. 26:690-696; Junghans et al. (1996) Proc. Natl. Acad. Sci. USA 93:5512-55166; Israel et al. (1996) Immunol. 89:573-578).

The role of FcRn affinity in affecting serum half-life has particular promise for medicine. Many therapeutics, in particular biologicals (i.e. peptide or polypeptide drugs, polynucleotides, etc.) suffer from inadequate serum half-lives in vivo. This necessitates the administration of such therapeutics at high frequencies and/or higher doses, or the use of sustained release formulations, in order to maintain the serum levels necessary for therapeutic effects. Frequent systemic administration of therapeutics, however, is expensive, inconvenient for the patient and the medical practitioners, and associated with considerable negative side effects such as tissue scarring, vascular pathologies, and increased risk of infection. Such drawbacks lead to decreased patient compliance and increased costs for the health system.

## II. Fcγ Receptors

The Fc receptors, members of the immunoglobulin gene superfamily of proteins, are surface glycoproteins that can bind the Fc portion of immunoglobulin molecules. Each member of the family recognizes immunoglobulins of one or more isotypes through a recognition domain on the α chain of the Fc receptor. Fc receptors are defined by their specificity for immunoglobulin subtypes. Fc receptors for IgG are referred to as "FcγR," for IgE as "FεR," and for IgA as "FcαR." Different accessory cells bear Fc receptors for antibodies of different isotype, and the isotype of the antibody determines which accessory cells will be engaged in a given response (Billadeau et al. (2002) J. Clin. Investigat. 2(109):161-81; Gerber et al. (2001) Microbes Infection 3:131-139; Ravetch et al. (2001) Annu. Rev. Immunol. 19:275-90; Ravetch et al. (2000) Science 290:84-89; Ravetch (1994) Cell 78(4):553-560; Ravetch et al. (1991) Annu. Rev. Immunol. 9:457-492; see also, Immunobiology: The Immune System in Health and Disease (4th ed. 1999), Elsevier Science Ltd/Garland Publishing, New York). An overview of various receptors is presented in **Table 1.**

| TABLE 1 Receptors for the Fc Regions of Immunoglobulin Isotypes | | | |
|---|---|---|---|
| **Receptor** | **Binding** | **Cell Type** | **Effect of Ligation** |
| FcγRI (CD64) | IgG1 $10^8$ M$^{-1}$ | Macrophages Neutrophils Eosinophils Dendritic cells | Uptake Stimulation Activation of respiratory burst Induction of killing |
| FcγRII-A (CD32) | IgG1 $2 \times 10^6$ M$^{-1}$ | Macrophages Neutrophils Eosinophils Dendritic cells Platelets Langerhan cells | Uptake Granule release |
| FcγRII-B1 (CD32) | IgG1 $2 \times 10^6$ M$^{-1}$ | B cells Mast cells | No uptake Inhibition of Stimulation |
| FcγRII-B2 (CD32) | IgG1 $2 \times 10^6$ M$^{-1}$ | Macrophages Neutrophils Eosinophils | Uptake Inhibition of Stimulation |

(continued)

| TABLE 1 | | | |
|---|---|---|---|
| Receptors for the Fc Regions of Immunoglobulin Isotypes | | | |
| Receptor | Binding | Cell Type | Effect of Ligation |
| FcγRIII (CD16) | IgG1 $5 \times 10^5$ M$^{-1}$ | NK cells Eosinophils Macrophages Neutrophils Mast Cells | Induction of Killing |
| FcεRI | IgE 1010 M$^{-1}$ | Mast cells Eosinophil Basophils | Secretion of granules |
| FcαRI (CD89) | IgA1, IgA2 $10^7$ M$^{-1}$ | Macrophages Neutrophils Eosinophils | Uptake Induction of killing |

Each Fcγ receptor ("FcγR") is an integral membrane glycoprotein, possessing extracellular domains related to a C2-set of immunoglobulin-related domains, a single membrane spanning domain and an intracytoplasmic domain of variable length. There are four known FcγRs, designated FcγRI (CD64), FcγRII (CD32), FcγRIII (CD16), and FcγRIV. The receptors are encoded by distinct genes; however, the extensive homology between the family members suggest they arose from a common progenitor perhaps by gene duplication.

Both activating and inhibitory signals are transduced through the FcγRs following ligation. These diametrically opposing functions result from structural differences among the different receptor isoforms. Two distinct domains within the cytoplasmic signaling domains of the receptor called immunoreceptor tyrosine based activation motifs (ITAMs) or immunoreceptor tyrosine based inhibitory motifs (ITIMS) account for the different responses. The recruitment of different cytoplasmic enzymes to these structures dictates the outcome of the FcγR-mediated cellular responses. ITAM-containing FcγR complexes include FcγRI, FcγRIIA, FcγRIIIA, and FcγRIV, whereas ITIM-containing complexes only include FcγRIIB.

FcγRI displays high affinity for the antibody constant region and restricted isotype specificity (Hulett and Hogarth (1994) Adv Immunol 57:1-127). FcγRII proteins are 40 KDa integral membrane glycoproteins which bind only the complexed IgG due to a low affinity for monomeric Ig ($10^6$ M$^{-1}$). This receptor is the most widely expressed FcγR, present on all hematopoietic cells, including monocytes, macrophages, B cells, NK cells, neutrophils, mast cells, and platelets. FcγRII has only two immunoglobulin-like regions in its immunoglobulin binding chain and hence a much lower affinity for IgG than FcγRI. There are three known human FcγRII genes (FcγRII-A, FcγRII-B, FcγRII-C), all of which bind IgG in aggregates or immune complexes. Human neutrophils express the FcγRIIA gene. The FcγRIIB gene is expressed on B lymphocytes; its extracellular domain is 96% identical to FcγRIIA and binds IgG complexes in an indistinguishable manner.

Distinct differences within the cytoplasmic domains of FcγRII-A and FcγRII-B create two functionally heterogenous responses to receptor ligation. The FcγRII-A isoform initiates intracellular signaling leading to cell activation such as phagocytosis and respiratory burst, whereas the FcγRII-B isoform initiates inhibitory signals, e.g., inhibiting B-cell activation. FcγRIIA clustering via immune complexes or specific antibody cross-linking serves to aggregate ITAMs along with receptor-associated kinases which facilitate ITAM phosphorylation. ITAM phosphorylation serves as a docking site for Syk kinase, activation of which results in activation of downstream substrates (e.g., PI$_3$K). Cellular activation leads to release of proinflammatory mediators. When co-ligated or co-aggregated along with an activating FcγR having an ITAM, such as FcγRIIA or FcεRI, the ITIM in FcγRIIB becomes phosphorylated and recruits the SH2 domain of the src homology 2-containing inositol phosphatase (SHIP), which in turn is phosphorylated and associates with Shc (Ott (2002) J. Immunol. 162(9):4430-4439; Yamanshi et al. (1997) Cell 88:205; Carpino et al. (1997) Cell 88:197). SHIP hydrolyzes phosphoinositol messengers released as a consequence of ITAM-containing FcγR-mediated tyrosine kinase activation, consequently preventing the influx of intracellular Ca$^{++}$, and dampening cellular responsiveness to FcγR ligation. Thus, B cell activation, B cell proliferation and antibody secretion is aborted, and FcγR-mediated phagocytosis is down-regulated (Tridandapani et al. (2002) J. Biol. Chem. 277(7):5082-89).

Specifically, coaggregation of FcγRIIA with FcγRIIB results in down-regulation of phosphorylation of Akt, which is a serine-threonine kinase that is involved in cellular regulation and serves to suppress apoptosis, and coaggregation of FcγRIIB with the high affinity IgE receptor FcεRI in mast cells leads to inhibition of antigen-induced degranulation, calcium mobilization, and cytokine production (Long (1999) Annu Rev. Immunol 17:875; Metcalfe et al. (1997) Physiol.

Rev. 77:1033). Coaggregation of FcγRIIB and the B-cell receptor (BCR) leads to inhibition of BCR-mediated signaling, and inhibition of cell cycle progression and cellular survival. Although numerous effector functions of FcγRIIB-mediated inhibition of BCR signaling are mediated through SHIP, recently it has been demonstrated that lipopolysaccharide (LPS)-activated B cells from SHIP deficient mice exhibit significant FcγRIIB-mediated inhibition of calcium mobilization, Ins(1,4,5)P$_3$ production, and Erk and Akt phosphorylation (Brauweiler et al. (2001) Journal of Immunology 167(1): 204-211).

The size of FcγRIII ranges between 40 and 80 kDa in mouse and man, due to heterogeneity within this class. Two human genes encode two transcripts, FcγRIIIA, an integral membrane glycoprotein, and FcγRIIIB, a glycosylphosphatidyl-inositol (GPI)-linked version. One murine gene encodes an FcγRIII homologous to the membrane spanning human FcγRIIIA. The FcγRIII shares structural characteristics with each of the other two FcγRs. Like FcγRII, FcγRIII binds IgG with low affinity and contains the corresponding two extracellular Ig-like domains. FcγRIIIA is expressed in macrophages, mast cells, and is the lone FcγR in NK cells. The GPI-linked FcγRIIIB is currently known to be expressed only in human neutrophils.

FcγRIV (also known as mFcRIV) requires association of the FcR gamma-chain for optimal expression and function on myeloid cells; its signaling potential is also enhanced by a cytoplasmic "YEEP" motif that recruits the adaptor molecule Crk-L and phosphatidylinositol-3-OH kinase. FcγRIV preferentially binds immunoglobulin E antibodies of the b allotype (IgEb) as well as IgG2a and IgG2b antibodies. Ligation of FcγRIV by antigen-IgEb immune complexes promotes macrophage-mediated phagocytosis, presentation of antigen to T cells, production of proinflammatory cytokines and the late phase of cutaneous allergic reactions (Hirano et al. (2007) Nature Immunology 8:762-771). FcγRIV is a recently identified receptor, conserved in all mammalian species with intermediate affinity and restricted subclass specificity (Nimmerjahn et al. (2005) Immunity 23:41-51; Mechetina et al. (2002) Immunogenetics 54:463-468; Davis et al. (2002) Immunol Rev 190:23-36). FcγRIII and FcγRIV are physiologically important activation FcγRs for mediating inflammatory disease triggered by cytotoxic antibodies or pathogenic immune complexes. FcγRIV is found on dendritic cells, macrophages, monocytes and neutrophils.

CN 1 958 615 A (China Antibody Pharmaceutical; 2007) is stated to disclose a humanized anti-CD20 antibody, compositions containing the disclosed antibody, and uses thereof in the treatment of diseases with the high-expression of CD20.

FR 2 894 982 A1 (Lab Francais du Fractionnement; 2007) is stated to disclose methods of producing antibodies that bind to Fc receptors and include particular amino acid modifications at positions 310 and 435.

Shields et al. (2001) "High resolution mapping of the binding site on human IgG1 for FcgammaRI, FcgammaRII, FcgammaRIII and FcRn and design of IgG1 variants with improved binding to the FcgammaR" J. Biol. Chem., Am. Soc. Biochem. Biologist, 276(9):6591-6604, relates to mapping of human IgG1 for human Fc receptors, and is stated to disclose select variants with binding to particular Fc receptors and enhanced antibody-dependent cell cytotoxicity.

Despite all such advances, a need remains for antibodies that possess therapeutic uses, for example in the treatment of autoimmunity, cancer, inflammatory disease, and/or transplantation, which have increased serum half-lives, particularly in humans. It is also desirable that such antibodies exhibit improved ability to mediate effector function from the Fc receptors. The present invention is directed to this and other needs.

## Summary of the Invention:

According to a first aspect of the present invention, there is provided a polypeptide comprising a variant Fc domain in accordance with claim 1 herein. Embodiments of the invention provide polypeptides comprising immunoglobulin Fc domains having a lysine at Kabat residue 435. The polypeptides may be antibodies, may specifically bind human FcRn, and may exhibit enhanced binding to FcRn and/or enhanced serum half-life. The polypeptides may comprise a variant Fc domain, which comprises one or more modifications, which modifications confer a phenotype alteration on the polypeptide, including altered effector function, increased or decreased binding to an FcγR, etc. The embodiments of the invention also provide polynucleotides encoding the polypeptides

The invention further concerns the embodiments of all such polypeptides of the first aspect which exhibit enhanced binding to FcRn at a pH below 6.5, as compared to a wild-type Fc domain, and particularly which exhibit enhanced binding to FcRn at pH 6.0, as compared to a wild-type Fc domain.

The invention further concerns the embodiments of all such polypeptides of the first aspect which exhibit higher binding affinity to FcRn, as compared to a wild-type Fc domain and/or enhanced serum half-life (preferably at least 1.5 times, more preferably at least 2 times, still more preferably at least 3 times greater than that of the polypeptide having a wild-type Fc domain).

The invention particularly concerns such polypeptides wherein the variant immunoglobulin Fc domain of the polypeptide:

(A) exhibits enhanced binding to FcRn at a pH below 6.5, as compared to a wild-type Fc domain;

(B) exhibits higher binding affinity to FcRn, as compared to a wild-type Fc domain; or

(C) enhances the serum half-life of the polypeptide.

The invention particularly concerns such polypeptides wherein the variant immunoglobulin Fc domain is a variant immunoglobulin G (IgG) Fc domain, and particularly wherein the IgG is selected from the group consisting of immunoglobulin G class 1 ($IgG_1$), immunoglobulin G class 2 ($IgG_2$), immunoglobulin G class 3 ($IgG_3$), and immunoglobulin G class 4 ($IgG_4$), and particularly wherein the polypeptide is an IgG heavy chain or a portion thereof.

The invention particularly concerns such polypeptides wherein the polypeptide binds a tumor antigen or a pathogen-related antigen or a human antigen.

The invention particularly concerns such polypeptides wherein the polypeptide is:

(A) a single chain antibody;

(B) a diabody; or

(C) a polypeptide chain of an antibody, or of an F(ab')2 fragment or F(ab) fragment of an antibody (including a monoclonal antibody)

and especially wherein the polypeptide is linked to a heterologous polypeptide (*e.g.*, a therapeutic agent such as a cytotoxin, .

The invention particularly concerns the embodiment wherein such polypeptides are an antibody comprising an Fc domain having a lysine at Kabat residue 435, and exhibiting at least one of:

(A) enhanced binding to FcRn at a pH below 6.5, as compared to a wild-type antibody;

(B) higher binding affinity to FcRn, as compared to a wild-type antibody; or

(C) enhanced serum half-life (preferably at least 1.5 times, more preferably at least 2 times, still more preferably at least 3 times greater than that of the polypeptide having a wild-type Fc domain) as compared to a wild-type antibody.

The invention further concerns the embodiment wherein the above-described polypeptides comprise at least one amino acid modification in addition to a lysine at Kabat residue 435 and an aspartic acid at Kabat residue 288. The invention particularly concerns such an embodiment wherein the additional modification comprises:

(A) at least one substitution selected from the group consisting of F243L, D270E, R292P, S298N, Y300L, V305I, A330V, and P396L;

(B) at least two substitutions selected from the group consisting of F243L and P396L; F243L and R292P; and R292P and V305I;

(C) at least three substitutions selected from the group consisting of F243L, R292P and Y300L; F243L, R292P and V305I; F243L, R292P and P396L; and R292P, V305I and P396L;

(D) at least four substitutions selected from the group consisting of F243L, R292P, Y300L and P396L; and F243L, R292P, V305I and P396L; or

(E) at least F243L, R292P, Y300L, V305I and P396 substitutions.

The invention further concerns the embodiment wherein the above-described polypeptides wherein the FcRn-binding domain is an Fc domain.

The invention further concerns the embodiment wherein the above-described polypeptides wherein the amino acid modifications of the variant Fc domain alter effector function (especially antibody-dependent cell-mediated cytotoxicity (ADCC) function or complement-dependent cytotoxicity (CDC) function) mediated by the Fc domain.

The invention further concerns the embodiment wherein the amino acid modifications of the variant Fc domain of the first aspect:

(A) increase binding of the Fc domain to an activating FcγR

(B) increase binding of the Fc domain to FcγRIIB

(C) decrease binding of the Fc domain to FcγRIIB.

The invention particularly concerns such polypeptides wherein the polypeptide binds a tumor antigen or a pathogen-related antigen (especially a pathogen-related antigen selected from the group consisting of bacterial antigens, viral antigens, fungal antigens, and protozoan antigens, for example, a smallpox antigen, a West Nile Virus antigen, an anthrax antigen, a bacterial meningitis antigen, a cholera antigen, a Clostridium difficile antigen, a Lyme disease antigen, a Pateurella pestis antigen, a pneumococcal antigen , a streptococcal antigen, a Clostridium tetani antigen, a micrococcal antigen, or a tularemia antigen) or a human antigen (especially wherein the tumor antigen is selected from the group consisting of 17-1A, αvβ3, AFP, BCR complex, CA125, CD3, CD18, CD20, CD22, CD33, CD44, CD52, CEA, CTLA-4, DNA-associated proteins, EGF receptor, Ep-CAM, GD2-ganglioside, gp IIIb/IIIa, gp72, HER2/neu, HLA-DR 10 beta, HLA-DR antigen, IgE, ganglioside GD3, MUC-1, nuC242, PEM antigen, SK-1 antigen, tumor antigen CA125, tumor antigen MUC1, VEGF, and VEGF-receptor).

The invention further concerns a polynucleotide encoding any of the above-described polypeptides of the invention..

The invention additionally provides the use of the polypeptide of the invention for the manufacture of a medicament for the treatment of cancer, wherein said polypeptide binds a tumor antigen.

The invention additionally provides the polypeptide of the invention for use in treating cancer, wherein said polypeptide binds a tumor antigen.

The use may be alone, or with a therapeutic agent (for example, an anti-angiogenic agent, an anti-neoplastic agent, a chemotherapeutic agent, or a cytotoxic agent) administered simultaneously or sequentially with the antibody.

The invention additionally provides the polypeptide of the invention for use in treating or preventing an infectious disease, wherein said polypeptide binds a pathogen-related antigen. The invention additionally provides the use of the polypeptide of the invention for the manufacture of a medicament for the treatment of an infectious disease, wherein said polypeptide binds a pathogen-related antigen. The infectious disease may be especially anthrax, bacterial meningitis, cholera, Clostridium difficile infection, Lyme disease, plague, pneumonia, streptococcus, tetanus, tuberculosis, tularemia, dengue fever, encephalitis, hemorrhagic fever, hepatitis, herpes, human papillomavirus, influenza, polio, rabies, smallpox, viral meningitis, West Nile fever, or yellow fever). The use may be alone, or with a therapeutic agent (for example, an anti-bacterial agent, anti-fungal agent, anti-protozoan agent and an anti-viral agent) administered simultaneously or sequentially with the antibody.

The invention additionally concerns a pharmaceutical composition comprising any of the above-described polypeptides of the invention, and a pharmaceutically acceptable carrier.

Additional advantages and features of the present invention will be apparent from the following detailed description, drawings and examples, which illustrate preferred embodiments of the invention.

**Brief Description of the Drawings:**

**Figure 1** depicts a Biacore analysis of wild-type and variant antibody binding to FcRn.

**Figures 2** and **3** depict Biacore analyses of wild-type and variant antibody binding to FcRn, in a pH dependent fashion. **Figure 2** shows binding at pH 6.0, and **Figure 3** shows binding at pH 7.4.

**Figure 4** depicts an SPR analysis of binding of soluble hFcRn (400nM) to mutant Fc ch4420 captured on the surface with immobilized mIgG1-Flrscn.

**Figure 5, Panels A-E** depict an SPR analysis of wild-type and variant antibody binding to soluble human FcγRIIIA (Panel A; V158; **Panel B**; F158), FcγRIIB **(Panel C)** and FcγRIIA (**Panels D** and **E**).

**Figure 6, Panels A-E** depict an SPR analysis of wild-type and variant antibody binding to soluble human FcγRIIIA (**Panel A;** V158; **Panel B;** F158), FcγRIIB (**Panel C**) and FcγRIIA (**Panels D** and **E**). Tested are ch4420 mutants MGFc316 (a 4D5 Fc variant having substitutions F243L, R292P, Y300L, V305I, and P396L in addition to H435K); MGFc317 (a 4D5 Fc variant having substitutions F243L, R292P, Y300L, V305I, and P396L in addition to both K288D and H435K); and MGFc318 (a 4D5 Fc variant having substitutions F243L, R292P and Y300L in addition to both K288D and H435K).

**Figure 7** depicts an SPR analysis of binding of FcRn to ch4420 mutants captured on a surface with immobilized protein-Flrscn; the SPR responses at pH 6.0 are normalized to same level of antibody. Tested are ch4420 mutants MGFc315 (K288V/H435D); MGFc316 (a 4D5 Fc variant having substitutions F243L, R292P, Y300L, V305I, and P396L in addition to H435K); MGFc317 (a 4D5 Fc variant having substitutions F243L, R292P, Y300L, V305I, and P396L in addition to both K288D and H435K); and MGFc318 (a 4D5 Fc variant having substitutions F243L, R292P and Y300L in addition to both K288D and H435K).

**Figures 8A-8E** show the results of investigations of the kinetics of binding of Fc variants to FcRn. The results of this investigation are shown for wild-type in **Figure 8A** (Langmuir 1:1 model) and **Figure 8B** (steady state affinity model), for ch4420 variant N434A in **Figure 8C** (Langmuir 1:1 model) and **Figure 8D** (steady state affinity model), and for MGFc315 in **Figure 8E** (Langmuir 1:1 model) and **Figure 8F** (steady state affinity model). The results show that the ch4420 variant MGFc315 had a lower KD than either N434A or wild-type Fc.

**Figures 9A-9B** show the results of investigations of the ability of different K288 ch4420 variants and of N286K, H435K and wild-type to bind to FcRn.

**Detailed Description of the Invention:**

The present invention provides polypeptides that exhibit altered binding to FcRn, and particularly antibodies that exhibit enhanced binding to human FcRn. The invention also provides methods of using the antibodies and compositions comprising them in diseases such as cancer and infectious disease.

Reference will now be made in detail to the presently preferred embodiments of the invention, which, together with the drawings and the following examples, serve to explain the principles of the invention. Unless otherwise defined, all technical and scientific terms used herein have the same meaning as commonly understood by one of ordinary skill in the art to which this invention belongs. One skilled in the art may refer to general reference texts for such definitions or for detailed descriptions of techniques discussed herein. These texts include Current Protocols in Molecular Biology (Ausubel et al., eds., John Wiley & Sons, and supplements through March 2008), Molecular Cloning: A Laboratory Manual (Sambrook and Russell, 3rd ed., 2001); Single-Molecule Techniques: A Laboratory Manual (Selvin & Ha, eds., Cold Spring Harbor Press, 2008); Current Protocols in Nucleic Acid Chemistry (Beaucage et al., eds., John Wiley & Sons, Inc., 2000); Current Protocols in Immunology (Coligan et al., eds., John Wiley & Sons, N.Y., and supplements through March 2008), Making and Using Antibodies: A Practical Handbook (Howard & Kaser, eds., CRC, 2006); Using Antibodies: A Laboratory Manual (Harlow & Lane, Cold Spring Harbor Press, 1999); Binding and Kinetics for Molecular Biologists (Goodrich & Kugel, Cold Spring Harbor Press, 2007); Current Protocols in Pharmacology (Enna et al., eds., John Wiley & Sons, N.Y., and supplements through March 2008), The Pharmacological Basis of Therapeutics (Goodman & Gilman, 11th ed., 2006), and Remington: The Science and Practice of Pharmacy (Lippincott Williams & Wilkins, 21 st edition (2005), for example.

**A. DEFINITIONS**

As used herein, the term "ADCC" refers to Antibody Dependent Cellular Cytotoxicity, an *in vitro* cell-mediated reaction in which nonspecific cytotoxic cells that express Fc$\gamma$Rs (e.g., monocytic cells such as Natural Killer (NK) cells and macrophages) recognize bound antibody on a target cell and subsequently cause lysis of the target cell.

As used herein, the term "antibody" refers to monoclonal antibodies, multispecific antibodies, human antibodies, humanized antibodies, synthetic antibodies, chimeric antibodies, polyclonal antibodies, camelized antibodies, single-chain Fvs (scFv), single chain antibodies, immunologically active antibody fragments (e.g., antibody fragments capable of binding to an epitope, *e.g.,* Fab fragments, Fab' fragments, F(ab')$_2$ fragments, Fv fragments, fragments containing either a VL or VH domain or a complementary determining region (CDR) that immunospecifically binds an antigen, etc.), bi-functional or multi-functional antibodies, disulfide-linked bispecific Fvs (sdFv), intrabodies, and diabodies, and epitope-binding fragments of any of the above. In particular, the term antibodies is intended to encompass immunoglobulin molecules, immunologically active fragments of immunoglobulin molecules, i.e., molecules that contain an antigen binding site, and FcRn-binding fragments of immunoglobulin molecules, e.g., Fc domain or hinge domain-Fc domain fragments. Immunoglobulin molecules can be of any type (e.g., IgG, IgE, IgM, IgD, IgA and IgY), class (e.g., IgG$_1$, IgG$_2$, IgG$_3$, IgG$_4$, IgA$_1$ and IgA$_2$) or subclass.

Reference to a "B cell antigen receptor" or "BCR" is intended to reference the B cell antigen receptor, which includes a membrane immunoglobulin (mIg) antigen binding component, or a biologically active portion thereof (i.e, a portion capable of binding a ligand and/or capable of associating with a transducer component). The term "BCR complex" is intended to reference the complex of BCR with transducer CD79a and CD79b components, or biologically active portions thereof (i.e., a portion capable of transducing an intracellular signal and/or capable of associating with an extracellular ligand binding portion).

As used herein, the term "cancer" refers to a neoplasm or tumor resulting from abnormal uncontrolled growth of cells. As used herein, cancer explicitly includes, leukemias and lymphomas. In some embodiments, cancer refers to a benign tumor, which has remained localized. In other embodiments, cancer refers to a malignant tumor, which has invaded and destroyed neighboring body structures and spread to distant sites. In some embodiments, the cancer is associated with a specific cancer antigen.

The terms "cell proliferative disorder" and "proliferative disorder" refer to disorders that are associated with some degree of abnormal cell proliferation. In one embodiment, the cell proliferative disorder is cancer.

The term "chimeric," when referring to antibodies, refers to an antibody in which a portion of a heavy and/or light chain is identical to or homologous with an antibody from one species (e.g., mouse) or antibody class or subclass, while the remaining portion is identical to or homologous with an antibody of another species (e.g., human) or antibody class or subclass, so long as they exhibit the desired biological activity. Chimeric antibodies of interest herein include "primatized" antibodies comprising variable domain antigen-binding sequences derived from a non-human primate (e.g., Old World Monkey, Ape, etc.) and human constant region sequences.

As used herein, the term "Complementarity Determining Region" or "CDR" refers to the amino acid residues of an antibody variable domain that are necessary for antigen binding. Each variable domain typically has three CDR regions identified as $CDR_1$, $CDR_2$ and $CDR_3$.

As used herein, the term "diabody molecule" refers to a complex of two or more polypeptide chains or proteins, each comprising at least one $V_L$ and one $V_H$ domain or fragment thereof, wherein both domains are comprised within a single polypeptide chain. In certain embodiments a "diabody molecule" includes molecules comprising an Fc or a hinge-Fc domain. Said polypeptide chains in the complex may be the same or different, i.e., the diabody molecule may be a homo-multimer or a hetero-multimer. In specific aspects, a "diabody molecule" includes dimers or tetramers or said polypeptide chains containing both a $V_L$ and $V_H$ domain. The individual polypeptide chains comprising the multimeric proteins may be covalently joined to at least one other peptide of the multimer by interchain disulfide bonds.

As used herein, the terms "disorder" and "disease" are used interchangeably to refer to a condition in a subject. In particular, the term "autoimmune disease" is used interchangeably with the term "autoimmune disorder" to refer to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, preferably chronic inflammation. Autoimmune disorders may or may not be associated with inflammation. Moreover, inflammation may or may not be caused by an autoimmune disorder. Thus, certain disorders may be characterized as both autoimmune and inflammatory disorders.

The term "effector cell" as used herein refers to a cell of the immune system that expresses one or more Fc receptors and mediates one or more effector functions. Effector cells include but are not limited to monocytes, macrophages, neutrophils, dendritic cells, eosinophils, mast cells, platelets, B cells, large granular lymphocytes, Langerhans' cells, natural killer (NK) cells, and may be from any organism including but not limited to humans, mice, rats, rabbits, and monkeys.

The term "effector function" refers to biological activities attributable to the interaction of an antibody Fc region with an Fc receptor or ligand. An antibody may have one or more effector functions. Non-limiting examples of antibody effector functions include antibody-dependent cell-mediated cytotoxicity (ADCC), C1q binding, complement dependent cytotoxicity (CDC), down regulation of cell surface receptors (e.g., B-cell receptor; BCR), opsonization, opsonophagocytosis, cell binding, and rosetting. Effector functions include both those that operate after the binding of an antigen and those that operate independent of antigen binding.

As used herein, the term "epitope" refers to that portion of a polypeptide or protein or a non-protein molecule that is immunospecifically bound by an antibody. An epitope may have immunogenic activity, such that it elicits an antibody production response in an animal. The ability of an epitope to immunospecifically bind an antibody may be determined by for example, an immunoassay. Epitopes need not necessarily be immunogenic.

The terms "Fc receptor" or "FcR" are used herein to describe a receptor that binds to the Fc region of an antibody. An exemplary FcR is a native sequence human FcR. An FcR may be one which binds an IgG antibody (a gamma receptor) and includes receptors of the FcγRI, FcγRII, FcγRIII, and FcγRIV subclasses, including allelic variants and alternatively spliced forms of these receptors, e.g., there are at least two known FcγRII receptors, FcγRIIA and FcγRIIB. The term FcR also includes the neonatal receptor, FcRn, which is responsible for the transfer of maternal IgGs to the fetus.

As used herein, the term "Fc region" or "Fc domain" is used to define a C-terminal region of an IgG heavy chain. Although the boundaries may vary slightly, the human IgG heavy chain Fc region is defined to stretch from Cys226 to the carboxy terminus. The Fc region of an IgG comprises two constant domains, $C_{H2}$ and $C_{H3}$. The $C_{H2}$ domain of a human IgG Fc region (also referred to as "Cγ2" domain) usually extends from amino acid 231 to amino acid 338, and the $C_{H3}$ domain of a human IgG Fc region usually extends from amino acids 342 to 447.

The term "glycosylation site" refers to an amino acid residue or residues that is recognized by a mammalian cell as a location for the attachment of sugar residues. Amino acid residues to which carbohydrates, such as oligosaccharides, are attached are usually asparagine (N-linkage), serine (O-linkage), and threonine (O-linkage) residues. The specific sites of attachment usually have a characteristic sequence of amino acids, referred to as a "glycosylation site sequence." The glycosylation site sequence for N-linked glycosylation is: Asn-X-Ser/Thr, where X can be any of the conventional amino acids other than proline. The Fc region of human IgG has two N-linked glycosylation sites, one in each of the $C_{H2}$ domains, at the asparagine at position 297 (Asn 297).

As used herein, the term "HAMA response" refers to the Human Anti-Mouse Antibody response, which is a deleterious immunogenic response that occurs when a human immune system recognizes a murine antibody as foreign and attacks it. A HAMA response can cause toxic shock or death. Chimeric and humanized antibodies reduce the likelihood of a HAMA response by decreasing the non-human portions of administered antibodies, but there is still potential for a Human Anti-Human Antibody response ("HAHA response") immune response to such antibodies.

The terms "heavy chain," "light chain" ("$C_L$"), "light chain variable region" ("$V_L$"), "heavy chain variable region" ("$V_H$"), "framework region" ("FR"), "heavy chain constant domain ("$C_H$"), "light chain constant domain ("$C_L$") refer to domains in naturally occurring immunoglobulins and the corresponding domains of synthetic (e.g., recombinant) binding proteins (e.g., humanized antibodies). The basic structural unit of naturally occurring immunoglobulins (e.g., IgG) is a tetramer having two light chains and two heavy chains. Usually naturally occurring immunoglobulin is expressed as a glycoprotein of about 150 KDa, although IgG can also be produced in a non-glycosylated form. The amino-terminal ("N") portion of each chain includes a variable region of about 100 to 110 or more amino acids primarily responsible for antigen recognition. The carboxy-terminal ("C") portion of each chain defines a constant region, with light chains having a single constant domain and heavy chains usually having three constant domains and a hinge region. Thus, the structure of the light chains of a naturally occurring IgG molecule is N-$V_L$-$C_L$-C and the structure of IgG heavy chains is N-$V_H$-$C_{H1}$-H-$C_{H2}$-$C_{H3}$-C (where H is the hinge region). The variable regions of an IgG molecule consists of the complementarity determining regions (CDRs), which contain the residues in contact with antigen and non-CDR segments, referred to as framework segments, which maintain the structure and determine the positioning of the CDR loops. Thus, the $V_L$ and $V_H$ domains have the structure N-$FR_1$-$CDR_1$-$FR_2$-$CDR_2$-$FR_3$-$CDR_3$-$FR_4$-C.

As used herein, the term "heterologous" nucleic acid denotes DNA, RNA, etc. that is introduced into a host cell. The nucleic acid may be derived from any of a variety of sources including genomic DNA, mRNA, cDNA, synthetic DNA and fusions or combinations of these. The nucleic acid may include a polynucelotide from the same cell or cell type as the host or recipient cell or a polynucleotide from a different cell type, for example, from a mammal or plant, and may, optionally, include marker or selection genes, for example, antibiotic resistance genes, temperature resistance genes, etc.

The term "hinge region" is generally defined as stretching from Glu216 to Pro230 of human IgG1. Hinge regions of other IgG isotypes may be aligned with the IgG1 sequence by placing the first and last cysteine residues forming inter-heavy chain S-S bonds in the same positions.

As used herein, the term "humanized" has its usual meaning in the art. In general terms, humanization of a non-human antibody involves substituting the CDR sequences from non-human immunoglobulin $V_L$ and $V_H$ regions into human framework regions. Further, as used herein, "humanized" antibodies may comprise additional substitutions and mutations in the CDR and/or framework regions introduced to increase affinity or for other purposes. For example, substitution of nonhuman framework residues in the human sequence can increase affinity. The resulting variable domains have non-human CDR sequences and framework sequences derived from human antibody framework sequence(s) or a human consensus sequence. A variety of different human framework regions may be used singly or in combination as a basis for a humanized antibody.

As used herein, the term "immunomodulatory agent" and variations thereof refer to an agent that modulates a host's immune system. In certain embodiments, an immunomodulatory agent is an immunosuppressant agent. In certain other embodiments, an immunomodulatory agent is an immunostimulatory agent. Immunomodulatory agents include, but are not limited to, small molecules, peptides, polypeptides, fusion proteins, antibodies, inorganic molecules, mimetic agents, and organic molecules.

As used herein, the term "immunospecifically binds," refers to the specific binding exhibited between an antibody and the epitope that it recognizes. Such binding will typically exhibit a $K_D$ of at least about 0.1 mM, more usually at least about 1 $\mu$M, preferably at least about 0.1 $\mu$M or less, and most preferably, 0.01 $\mu$M or less. Preferably, the antibodies of the invention immunospecifically bind to proteins with high affinity (e.g., low $K_D$).

An antibody that immunospecifically binds to an antigen may bind to other peptides or polypeptides with lower affinity as determined by, e.g., immunoassays, BIAcore, or other assays known in the art. Preferably, molecules that specifically bind an antigen do not cross react with other proteins. Molecules that specifically bind an antigen can be identified, for example, by immunoassays, BIAcore, or other techniques known to those of skill in the art.

The term "Antibody Engineering Technology Art" as used herein refers to technology disclosed in U.S. Patent Application No. 11/952,568; U.S. Patent Application Publication Nos. 20040185045, 20040197347, 20040197866, 20050037000, 20050064514, 20050215767, 20050260213, 20060013810, 20060134709, 20060177439, 20070004909, 20070036799, 20070037216, 20070077246, 20070244303, 20080044417, 20080044429, 20080050371, 20080095766, and 20080112961; U.S. Patent Nos. 7,112,439; 7,351,803; and 7,355,008; International Application Publication Nos. WO 05/115452, WO 05/110474, WO 06/113665, WO 04/063351, WO 06/088494, WO 06/066078, WO 04/016750, WO 05/018669, WO 07/021841, WO 08/019199, WO 08/002933, WO2007106707, WO2008105886, and WO2008140603.

The term "monoclonal antibody" as used herein refers to an antibody obtained from a population of substantially homogeneous antibodies, i.e., the individual antibodies comprising the population are identical except for possible naturally occurring mutations that may be present in minor amounts, and the term "polyclonal antibody" as used herein refers to an antibody obtained from a population of heterogenous antibodies. Monoclonal antibodies are highly specific, being directed against a single epitope. In addition to their specificity, monoclonal antibodies are advantageous in that they may be synthesized without contamination by other antibodies. The term "monoclonal" indicates the character of the antibody as being obtained from a substantially homogeneous population of antibodies, and is not to be construed as requiring production of the antibody by any particular method.

As used herein, the terms "nucleic acids" and "nucleotide sequences" include DNA molecules (e.g., cDNA or genomic DNA), RNA molecules (e.g., mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

"Serum half-life," as used herein, refers to the time taken for the serum concentration of the polypeptide to be reduced by 50%, in vivo, for example due to degradation of the sequence or compound and/or clearance or sequestration of the sequence or compound by natural mechanisms. The serum half-life of a polypeptide can be determined, for example, by pharmacokinetic analysis.

"Substantial sequence identity," as used herein, refers to two or more sequences or subsequences (e.g., domains) that have at least about 80% amino acid residue identity, preferably at least about 90%, or at least about 95% identity when compared and aligned for maximum correspondence. Sequence identity between two similar sequences (e.g., antibody variable regions) can be measured by algorithms such as that of Smith & Waterman, 1981, Adv. Appl. Math. 2:482 [local homology algorithm], Needleman & Wunsch, 1970, J. Mol. Biol. 48:443 [homology alignment algorithm], Pearson & Lipman, 1988, Proc. Natl. Acad. Sci. (U.S.A.) 85:2444 [search for similarity method], or Altschul et al., 1990, J. Mol. Biol. 215:403-10 [BLAST algorithm]. When using any of the aforementioned algorithms, the default parameters (for Window length, gap penalty, etc.) are used. An amino acid sequence is said to be "substantially similar to" a second sequence when the degree of sequence identity is at least about 70% identical, preferably at least about 80%, or at least about 90%, or even at least about 95%, identical. A nucleic acid sequence is said to be "substantially similar to" a second sequence when either: (1) the degree of sequence identity is at least about 70% identical, preferably at least about 80%, or at least about 90%, or even at least about 95%, identical, or the nucleic acid sequence encodes a polypeptide that is at least about 70% identical, preferably at least about 80%, or at least about 90%, or even at least about 95%, identical to the polypeptide encoded by the second sequence. Sequences that are substantially identical are also substantially similar.

When referring to antibodies, the assignment of amino acids to each domain is in accordance with Kabat, Sequences Of Proteins Of Immunological Interest (National Institutes of Health, Bethesda, Md., 1987 and 1991) Throughout the present specification, the numbering of the residues in an IgG heavy chain is that of the EU index as in Kabat, and refers to the numbering of the human IgG1 EU antibody.

## B. ANTIBODIES

The present invention particularly relates to polypeptides with altered binding to FcRn, and more preferably to human FcRn. The polypeptides have enhanced binding affinity for FcRn and/or enhanced serum half-lives, and more preferably also have enhanced effector function, all as compared to a native antibody. In particular, the polypeptide comprises an immunoglobulin FcRn-binding or Fc domain having a lysine at Kabat residue 435. Preferably, the immunoglobulin domain is human, fully human, humanized or chimeric. Preferably the polypeptide is an antibody. The antibody may be a variant antibody, for example the antibody may comprise a variant Fc domain.

The immunoglobulin is an IgG, for example an IgG selected from the group consisting of $IgG_1$, $IgG_2$, $IgG_3$, and $IgG_4$, or a fragment thereof that has been modified to contain an FcRn-binding domain or an IgG Fc domain.

The FcRn-binding or Fc domain comprising a lysine at Kabat residue 435 has an unexpected enhanced binding affinity for FcRn, and particularly to human FcRn. The polypeptides having this modification exhibit enhanced binding to FcRn in a pH-dependent manner, e.g., enhanced binding at a pH below 6.5, enhanced binding at a pH below 6.0, etc., as compared to binding at neutral pHs (e.g., about 7.0 to 7.5). This enhanced binding to FcRn results in an increased bioavailability of the polypeptides, in particular by increasing transport of the polypeptides to a target tissue, increasing release of the polypeptides into the bloodstream, and the like. For example, FcRn is expressed in the mucous membranes of adults such as the intestinal epithelia, and thus polypeptides having enhanced binding to FcRn are expected to exhibit improved bioavailability after oral administration because of improved transport across the intestinal wall. Likewise, the presence of FcRn in the lung is expected to similarly promote transport of the polypeptides of the invention across the pulmonary mucosa into the bloodstream, thus increasing the bioavailability of polypeptides which are administered via intrapulmonary or intranasal means.

The polypeptides having this 435K modification also exhibit enhanced serum half-lives, as compared to a wild-type FcRn-binding or Fc domain. In one embodiment, the polypeptides exhibit a serum half-life in mammals (especially humans) of about 3, 4, 5, 6, 7, 8, 9, 10, 11, 12, 13, 14, 15, 16, 17, 18, 19, 20, 21, 22, 23, 24, 25, 26, 27, 28, 29, or 30 days, or at least about 3, 4, 5, 6, 7, 8, 9, 10, 11, 12, 13, 14, 15, 16, 17, 18, 19, 20, 21, 22, 23, 24, 25, 26, 27, 28, 29, or 30 days. In another embodiment, the polypeptides exhibit a serum half-life in mammals (especially humans) of more than 3, 4, 5, 6, 7, 8, 9, 10, 11, 12, 13, 14, 15, 16, 17, 18, 19, 20, 21, 22, 23, 24, 25, 26, 27, 28, 29, 30, or more than 30 days. In a preferred embodiment, the serum half-life of the polypeptides is at least 4 days, at least 7 days, at least 9 days, or at least 12 days.

In a different embodiment, the polypeptides exhibit a serum half-life in mammals (especially humans) that is enhanced, as compared to a polypeptide having a wild-type Fc domain. The enhanced serum half-life is at least 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, or more than 10 times the serum half-life of a wild-type Fc domain. In another embodiment, the enhanced serum half-life is more than 1.5, 2, 2.5, 3, 3.5, 4, 4.5, or 5 times the serum half-life of a wild-type Fc domain. In yet another embodiment, the enhanced serum half-life is more than 1.5, 3, 5, 7.5, or 10 times the serum half-life of a wild-type Fc domain.

The polypeptides (especially antibodies) may bind an antigen, preferably a human antigen. In a preferred embodiment, the antigen is an antigen related to a disease or disorder to be treated with the polypeptides, for example a tumor antigen or a pathogen-related antigen. Non-limiting examples of suitable tumor antigens include 17-1A, $\alpha v \beta 3$, AFP, BCR complex, CA125, CD3, CD18, CD20, CD22, CD33, CD44, CD52, CEA, CTLA-4, DNA-associated proteins, EGF receptor, Ep-CAM, GD2-ganglioside, gp IIIb/IIIa, gp72, HER2/neu, HLA-DR 10 beta, HLA-DR antigen, IgE, ganglioside GD3, MUC-1, nuC242, PEM antigen, SK-1 antigen, tumor antigen CA125, tumor antigen MUC1, VEGF, and VEGF-receptor. Non-limiting examples of pathogen-related antigens include viral antigens (e.g., coat proteins, capsid protein, surface antigens, etc.), bacterial antigens (e.g., endotoxins, exotoxins, fimbrial antigens, glycolipids, O antigens, surface antigens, etc.), fungal antigens (e.g., gp43, mycotoxins, etc.), and protozoan antigens (e.g., amoeba antigen, histidine rich protein-2, p30, etc.).

The polypeptides (especially antibodies) contemplated by the present invention may be in a complex with one another or with other non-immunoglobulin polypeptides (e.g., enzymes, hormones, structural proteins, etc.). For example, an embodiment may provide a polypeptide complex comprising two polypeptides, wherein one of said polypeptides comprises a heavy chain, and the other polypeptide comprises a variant light chain, or wherein both polypeptides comprise the same sequences. Complexing can be mediated by any suitable technique, including by dimerization/multimerization at a dimerization/multimerization domain such as those described herein or covalent interactions (such as through a disulfide linkage) (which in some contexts is part of a dimerization domain, for example a dimerization domain may contain a leucine zipper sequence and a cysteine). In another embodiment, a composition may comprise polypeptides and/or polynucleotides of the invention, for example a composition may comprise a plurality of any of the polypeptides described herein. A composition comprising a polynucleotide or polypeptide may be in the form of a kit or an article of manufacture (optionally packaged with instructions, buffers, etc.).

It is also contemplated that polypeptide variants (and in particular antibody variants) can be prepared. The polypeptide variants may possess sequence modifications (e.g., substitutions, deletions and/or additions) at desired positions within their amino acid sequences relative to the native amino acid sequence. Those skilled in the art will appreciate that amino acid changes may alter post-translational processes of the antibody or polypeptide, such as changing the number or position of glycosylation sites or altering the membrane anchoring characteristics. In a preferred embodiment, the antibody and polypeptide variants are Fc region variants.

Variants may have the same or altered activity as compared to a native antibody or polypeptide. For example, it may be desirable that the variant have the same activity, but be modified in a manner so that it is more stable or has a longer half-life in vivo, for example by conjugating the antibody with albumin or a salvage receptor binding epitope, as described, e.g., in U.S. Patent No. 5,739,277. Or, for example, it may be desirable that an antibody have an increased binding affinity to antigen, but the same effector function as a native antibody, or it may be desirable that an antibody have the same binding affinity to antigen, but a decreased effector function. Activity may be tested by, e.g., using in vitro assays such as ELISA assays, surface plasmon resonance assays, radiolabeled protein binding assays (RIA), or immunoprecipitation assays.

Substantial modifications in function or immunological identity may be accomplished by selecting modifications that differ significantly in their effect on maintaining (a) the structure of the polypeptide backbone in the area of the modification, for example, as a sheet or helical conformation, (b) the charge or hydrophobicity of the molecule at the target site, or (c) the bulk of the side chain. Scanning amino acid analysis can also be employed to identify one or more amino acids along a contiguous sequence, for example as described by Cunningham and Wells (1989) Science 244:1081-1085. Among the preferred scanning amino acids are relatively small, neutral amino acids, such as alanine, glycine, serine, and cysteine. Alanine is typically a preferred scanning amino acid among this group because it is the most common amino acid, is frequently found in both buried and exposed positions, and because it eliminates the side-chain beyond the beta-carbon and is less likely to alter the main-chain conformation of the variant. If alanine substitution does not yield adequate amounts of variant, an isoteric amino acid can be used. Further, any cysteine residue not involved in maintaining the proper conformation of the antibody or polypeptide may be substituted, generally with serine, to improve the oxidative stability of the molecule and prevent aberrant crosslinking. However, in certain circumstances, particularly where the antibody is an antibody fragment such as an Fv fragment, cysteine bond(s) may be added to the antibody or polypeptide to improve its stability.

**B1. Fc Domain Variants**

The polypeptides of the present invention have variant Fc domains. Modification of the Fc domain normally leads to an altered phenotype, for example altered serum half-life, altered stability, altered susceptibility to cellular enzymes or altered effector function. It may be desirable to modify the antibody of the invention with respect to effector function, so as to enhance the effectiveness of the antibody in treating cancer, for example. Reduction or elimination of effector function is desirable in certain cases, for example in the case of antibodies whose mechanism of action involves blocking or antagonism, but not killing of the cells bearing a target antigen. Increased effector function is generally desirable when directed to undesirable cells, such as tumor and foreign cells, where the FcγRs are expressed at low levels, for example, tumor specific B cells with low levels of FcγRIIB (e.g., non-Hodgkins lymphoma, CLL, and Burkitt's lymphoma). In said embodiments, molecules of the invention with conferred or altered effector function activity are useful for the treatment and/or prevention of a disease, disorder or infection where an enhanced efficacy of effector function activity is desired.

In certain embodiments, the molecules of the invention comprise one or more modifications to the amino acids of the Fc domain, which reduce the affinity and avidity of the Fc region and, thus, the molecule of the invention, for one or more FcγR receptors. In other embodiments, the molecules of the invention comprise one or more modifications to the amino acids of the Fc region, which increase the affinity and avidity of the Fc region and, thus, the molecule of the invention, for one or more FcγR receptors. In other embodiments, the molecules comprise a variant Fc domain wherein said variant confers or mediates increased ADCC activity and/or an increased binding to FcγRIIA, relative to a molecule comprising no Fc domain or comprising a wild-type Fc domain. In alternate embodiments, the molecules comprise a variant Fc domain wherein said variant confers or mediates decreased ADCC activity (or other effector function) and/or an increased binding to FcγRIIB, relative to a molecule comprising no Fc domain or comprising a wild-type Fc domain.

In some embodiments, the invention encompasses molecules comprising a variant Fc region, which variant Fc region does not show a detectable binding to any FcγR, relative to a comparable molecule comprising the wild-type Fc region. In other embodiments, the invention encompasses molecules comprising a variant Fc region, which variant Fc region only binds a single FcγR, preferably one of FcγRIIA, FcγRIIB, or FcγRIIIA.

The polypeptides of the present invention may comprise altered affinities for an activating and/or inhibitory Fcγ receptor. In one embodiment, the antibody or polypeptide comprises a variant Fc region that has increased affinity for FcγRIIB and decreased affinity for FcγRIIIA and/or FcγRIIA, relative to a comparable molecule with a wild-type Fc region. In another embodiment, the polypeptides of the present invention compris a variant Fc region, which has decreased affinity for FcγRIIB and increased affinity for FcγRIIIA and/or FcγRIIA, relative to a comparable molecule with a wild-type Fc region. In yet another embodiment, the polypeptides of the present invention comprise a variant Fc region that has decreased affinity for FcγRIIB and decreased affinity for FcγRIIIA and/or FcγRIIA, relative to a comparable molecule with a wild-type Fc region. In still another embodiment, the polypeptides of the present invention comprise a variant Fc region, which has unchanged affinity for FcγRIIB and decreased (or increased) affinity for FcγRIIIA and/or FcγRIIA, relative to a comparable molecule with a wild-type Fc region.

In certain embodiments, the invention encompasses immunoglobulins comprising a variant Fc region with an altered affinity for FcγRIIIA and/or FcγRIIA such that the immunoglobulin has an enhanced effector function, e.g., antibody dependent cell mediated cytotoxicity. Non-limiting examples of effector cell functions include antibody-dependent cell mediated cytotoxicity (ADCC), antibody-dependent phagocytosis, phagocytosis, opsonization, opsonophagocytosis, cell binding, rosetting, C1q binding, and complement dependent cell mediated cytotoxicity.

In a preferred embodiment, the alteration in affinity or effector function is at least 2-fold, preferably at least 4-fold, at least 5-fold, at least 6-fold, at least 7-fold, at least 8-fold, at least 9-fold, at least 10-fold, at least 50-fold, or at least 100-fold, relative to a comparable molecule comprising a wild-type Fc region. In other embodiments of the invention, the variant Fc region immunospecifically binds one or more FcRs with at least 65%, preferably at least 70%, 75%, 80%, 85%, 90%, 95%, 100%, 125%, 150%, 175%, 200%, 225%, or 250% greater affinity relative to a molecule comprising a wild-type Fc region. Such measurements can be in vivo or in vitro assays, and in a preferred embodiment are in vitro assays such as ELISA or surface plasmon resonance assays.

In different embodiments, the molecules comprise a variant Fc domain wherein said variant agonizes at least one activity of an FcγR receptor, or antagonizes at least one activity of an FcγR receptor. In a preferred embodiment, the molecules comprise a variant that agonizes (or antagonizes) one or more activities of FcγRIIB, for example, B cell receptor-mediated signaling, activation of B cells, B cell proliferation, antibody production, intracellular calcium influx of B cells, cell cycle progression, FcγRIIB-mediated inhibition of FcεRI signaling, phosphorylation of FcγRIIB, SHIP recruitment, SHIP phosphorylation and association with Shc, or activity of one or more downstream molecules (e.g., MAP kinase, JNK, p38, or Akt) in the FcγRIIB signal transduction pathway. In another embodiment, the molecules comprise a variant that agonizes (or antagonizes) one or more activities of FcεRI, for example, mast cell activation, calcium mobilization, degranulation, cytokine production, or serotonin release.

In certain embodiments, the molecules comprise an Fc domain comprising domains or regions from two or more IgG isotypes (e.g., IgG1, IgG2, IgG3 and IgG4). The various IgG isotypes exhibit differing physical and functional properties including serum half-life, complement fixation, FcγR binding affinities and effector function activities (e.g., ADCC, CDC, etc.) due to differences in the amino acid sequences of their hinge and/or Fc domains, for example as described in Flesch and Neppert (1999) J. Clin. Lab. Anal. 14:141-156; Chappel et al. (1993) J. Biol. Chem. 33:25124-25131; Chappel et al. (1991) Proc. Natl. Acad. Sci. (U.S.A.) 88:9036-9040; or Brüggemann et al. (1987) J. Exp. Med 166:1351-1361. This type of variant Fc domain may be used alone, or in combination with an amino acid modification, to affect Fc-mediated effector function and/or binding activity. In combination, the amino acid modification and IgG hinge/Fc region may display similar functionality (e.g., increased affinity for FcγRIIA) and may act additively or, more preferably, synergistically to modify the effector functionality in the molecule of the invention, relative to a molecule of the invention comprising a wild-type Fc region. In other embodiments, the amino acid modification and IgG Fc region may display opposite functionality (e.g., increased and decreased affinity for FcγRIIA, respectively) and may act to selectively temper or reduce a specific functionality in the molecule of the invention, relative to a molecule of the invention not comprising an Fc region or comprising a wild-type Fc region of the same isotype.

In a preferred specific embodiment, the molecules comprise a variant Fc region, wherein said variant Fc region comprises at least one amino acid modification relative to a wild-type Fc region, such that said molecule has an altered affinity for an FcR, provided that said variant Fc region does not have a substitution at positions that make a direct contact with FcγR based on crystallographic and structural analysis of Fc-FcR interactions such as those disclosed by Sondermann et al. (2000) Nature 406:267-73. Examples of positions within the Fc region that make a direct contact with FcγR are amino acid residues 234-239 (hinge region), amino acid residues 265-269 (B/C loop), amino acid residues 297-299 (C'/E loop), and amino acid residues 327-332 (F/G loop). In some embodiments, the molecules of the invention comprise variant Fc regions comprise modification of at least one residue that does not make a direct contact with an FcγR based on structural and crystallographic analysis, e.g., is not within the Fc-FcγR binding site.

Variant Fc domains are well known in the art, and any known Fc variant may be used in the present invention to confer or modify the effector function exhibited by a molecule of the invention comprising an Fc domain (or portion thereof) as functionally assayed, e.g., in an NK dependent or macrophage dependent assay. For example, Fc domain variants identified as altering effector function are disclosed in the Antibody Engineering Technology Art, and any suitable variant disclosed therein may be used in the present molecules.

In certain embodiments, the molecules comprise a variant Fc region, having one or more amino acid modifications in one or more regions, which modification(s) alter (relative to a wild-type Fc region) the Ratio of Affinities of the variant Fc region to an activating FcγR (such as FcγRIIA or FcγRIIIA) relative to an inhibiting FcγR (such as FcγRIIB):

$$\text{Ratio of Affinities} = \frac{\text{Wild-Type to Variant Change in Affinity to Fc}\gamma\text{R}_{\text{Activating}}}{\text{Wild-Type to Variant Change in Affinity to Fc}\gamma\text{R}_{\text{Inhibiting}}}$$

Where an Fc variant has a Ratio of Affinities greater than 1, the methods of the invention have particular use in providing a therapeutic or prophylactic treatment of a disease, disorder, or infection, or the amelioration of a symptom thereof, where an enhanced efficacy of effector cell function (e.g., ADCC) mediated by FcγR is desired, e.g., cancer or infectious disease. Where an Fc variant has a Ratio of Affinities less than 1, the methods of the invention have particular use in providing a therapeutic or prophylactic treatment of a disease or disorder, or the amelioration of a symptom thereof, where a decreased efficacy of effector cell function mediated by FcγR is desired, e.g., autoimmune or inflammatory disorders. **Table 2** lists exemplary single, double, triple, quadruple and quintuple mutations by whether their Ratio of Affinities is greater than or less than 1. Specific binding data for various mutations is listed in **Table 3,** and more information concerning these mutations may be found in the Antibody Engineering Technology Art.

| Table 2: Exemplary Single and Multiple Mutations Listed by Ratio of Affinities | | | | | |
|---|---|---|---|---|---|
| Ratio | Single | Double | Triple | Quadruple | Quintuple |
| > 1 | F243L | F243L & R292P | F243L, P247L & N421K | L234F, F243L, R292P & Y300L | L235V, F243L, R292P, Y300L & P396L |
| | D270E | F243L & Y300L | F243L, R292P & Y300L | L235I, F243L, R292P & Y300L | |
| | R292G | | F243L, R292P & V305I | | |
| | R292P | F243L & P396L | F243L, R292P & P396L | L235Q, F243L, R292P & Y300L | L235P, F243L, R292P, Y300L & P396L |
| | | | F243L, Y300L & P396L | | |
| | | D270E & P396L | P247L, D270E & N421K | F243L, P247L, D270E & N421K | |
| | | R292P & Y300L | R255L, D270E & P396L | F243L, R255L, D270E & P396L | F243L, R292P, V305I, Y300L & P396L |
| | | | D270E, G316D & R416G | | |
| | | R292P & V305I | D270E, K392T & P396L | F243L, D270E, G316D & R416G | |
| | | | D270E, P396L & Q419H | | |
| | | R292P & P396L | V284M, R292L & K370N | F243L, D270E, K392T & P396L | |
| | | Y300L & | R292P, Y300L & P396L | F243L, D270E, P396L | |
| | | P396L | | & Q419H | |
| | | P396L & Q419H | | F243L, R292P, Y300L, & P396L | |
| | | | | F243L, R292P, V305I & P396L | |
| | | | | P247L, D270E, Y300L & N421K | |
| | | | | R255L, D270E, R292G & P396L | |
| | | | | R255L, D270E, Y300L & P396L | |
| | | | | D270E, G316D, P396L & R416G | |
| < 1 | Y300L | F243L & P396L | F243L, R292P & V305I | | |
| | P396L | P247L & N421K | | | |
| | | R255L & P396L | | | |
| | | R292P & V305I | | | |
| | | K392T & P396L | | | |
| | | P396L & Q419H | | | |

| Table 3: Detailed Binding Information for Exemplary Fc Variants | | | | | |
|---|---|---|---|---|---|
| | | | | Ratio of Affinities | |
| Fc sequence | CD16A V158 | CD16A F158 | CD32B | CD16A/CD32B | |
| | | | | V158 | F158 |
| Ratio of Affinities > 1 | | | | | |
| Class I: Increased Binding to CD16; Decreased Binding to CD32B | | | | | |
| F243L | 4.79 | 3.44 | 0.84 | 5.70 | 4.10 |
| F243L P247L D270E N421K | 2.30 | 3.45 | 0.32 | 7.19 | 10.78 |
| F243L P247L N421K | 1.89 | 1.71 | 0.17 | 11.12 | 10.06 |
| F243L R255L D270E P396L | 1.75 | 1.64 | 0.38 | 4.61 | 4.32 |
| F243L D270E G316D R416G | 1.50 | 1.34 | 0.20 | 7.50 | 6.70 |
| F243L D270E K392T P396L | 3.16 | 2.44 | 0.44 | 7.18 | 5.55 |
| F243L D270E P396L Q419H | 1.46 | 1.15 | 0.26 | 5.62 | 4.42 |
| F243L R292P | 4.73 | | 0.12 | 39.4 | |
| F243L R292P | 4 | 1.67 | 0.16 | 25 | 10.44 |
| F243L R292P P300L | 6.69 | 2.3 | 0.32 | 20.9 | 7.19 |
| F243L R292P V305I | 2.56 | 1.43 | ND | >25 | >25 |
| F243L R292P V305I P396L | 5.37 | 2.53 | 0.40 | 13.43 | 6.33 |
| P247L D270E N421K | 1.89 | 2.46 | 0.58 | 3.26 | 4.24 |
| R255L D270E R292G P396L | 1.39 | 1.30 | 0.65 | 2.14 | 2.00 |
| R255L D270E Y300L P396L | 1.52 | 1.74 | 0.87 | 1.75 | 2.00 |
| R255L D270E P396L | 1.34 | 1.65 | 0.87 | 1.54 | 1.90 |
| D270E | 1.25 | 1.48 | 0.39 | 3.21 | 3.79 |
| D270E G316D R416G | 2.18 | 2.49 | 0.78 | 2.79 | 3.19 |
| D270E K392T P396L | 1.81 | 2.28 | 0.79 | 2.29 | 2.89 |
| D270E P396L | 1.38 | 1.65 | 0.89 | 1.55 | 1.85 |
| D270E P396L G316D R416G | 1.22 | | 1.07 | 1.14 | |
| D270E P396L Q419H | 1.64 | 2.00 | 0.68 | 2.41 | 2.94 |
| V284M R292P K370N | 1.14 | 1.37 | 0.37 | 3.1 | 3.7 |
| R292G | 1.54 | | 0.25 | 6.2 | |
| R292P | 2.90 | | 0.25 | 11.60 | |
| R292P V305I | 1.32 | 1.28 | 0.37 | 3.6 | 3.46 |
| Class II: Decreased Binding to CD16; Greatly Decreased Binding to CD32B | | | | | |
| R292P | | 0.64 | 0.25 | | 2.56 |
| R292P F243L | | 0.6 | 0.12 | | 5.00 |
| Class III: Increased Binding to CD16; Unchanged Binding to CD32B | | | | | |
| F243I R292P Y300L V305I P396L | 10.9 | 3.12 | 1.05 | 10.4 | 2.97 |
| F243L R292P Y300L P396L | 10.06 | 5.62 | 1.07 | 9.40 | 5.25 |
| R292P V305I P396L | 1.85 | 1.90 | 0.92 | 2.01 | 2.07 |

(continued)

| Class IV: Greatly Increased Binding to CD16; Increased Binding to CD32B | | | | | |
|---|---|---|---|---|---|
| F243L R292P Y300L V305I P396L | 10.06 | 8.25 | 1.38 | 7.29 | 5.98 |
| D270E G316D P396L R416G | 1.22 | | 1.07 | 1.14 | |
| **Ratio of Affinities < 1** | | | | | |
| **Class V: Unchanged Binding to CD16; Increased Binding to CD32B** | | | | | |
| R255L P396L | 1.09 | | 2.22 | 0.49 | |
| Y300L | 1.01 | | 1.18 | | 0.99 |
| **Class VI: Increased Binding to CD16; Greatly Increased Binding to CD32B** | | | | | |
| F243L P396L | 1.49 | 1.60 | 2.22 | 0.67 | 0.72 |
| P247L N421K | 1.29 | 1.73 | 2.00 | 0.65 | 0.87 |
| R255L P396L | | 1.39 | 2.22 | 0.49 | 0.63 |
| R292P V305I | 1.59 | 2.11 | 2.67 | 0.60 | 0.79 |
| K392T P396L | 1.49 | 1.81 | 2.35 | 0.63 | 0.77 |
| P396L | 1.27 | 1.73 | 2.58 | 0.49 | 0.67 |
| P396L Q419H | 1.19 | 1.19 | 1.33 | 0.89 | 0.89 |
| **Class VII: Decreased Binding to CD16; Increased / Unchanged Binding to CD32B** | | | | | |
| D270E G316D P396L R416G | | 0.94 | 1.07 | | 0.88 |

In other embodiments, the molecules comprise a variant Fc region having one or more amino acid substitutions, which substitutions alter (relative to a wild-type Fc region) the binding of the variant Fc region, e.g., enhance the binding to an activating FcγR (such as FcγRIIA or FcγRIIIA) and/or reduce the binding to an inhibiting FcγR (such as FcγRIIB). Various Fc mutations having one or more amino acid changes were engineered and analyzed by surface plasmon resonance for $k_{off}$, as shown in **Table 4.** Dissociation rate constants for binding the various FcγR were determined by BIAcore analysis and directly compared with those for the wild-type Fc, with the ratio (x = WT $k_{off}$/mutant $k_{off}$) indicated in the right-hand columns of Table 4 with respect to each FcγR tested.

**Table 4: Comparison Of $k_{off}$ Of Fc Mutants to Wild-Type Fc**

| Mutant | Amino Acid Changes | | | | | CD16A$^V$ | CD16A$^F$ | CD32A$^H$ | CD32B |
|---|---|---|---|---|---|---|---|---|---|
| **One Amino Acid** | | | | | | | | | |
| 1 | F243L | | | | | **4.8** | **3.4** | **0.6** | 0.8 |
| 2 | D270E | | | | | 1.3 | 1.5 | **2.2** | **0.4** |
| 3 | R292P | | | | | **2.4** | 1.6 | 0.7 | **0.3** |
| 4 | S298N | | | | | nd | nd | nt | **0.2** |
| 5 | Y300L | | | | | 1.0 | 1.2 | **2.9** | 1.2 |
| 6 | V305I | | | | | 0.9 | 0.6 | 1.3 | 1.2 |
| 7 | A330V | | | | | 0.6 | 1.2 | **0.4** | **0.3** |
| 8 | P396L | | | | | 1.3 | 1.7 | 1.6 | **2.6** |
| **Two Amino Acids** | | | | | | | | | |
| 9 | F243L | | | | P396L | **2.2** | **2.0** | 1.5 | 1.6 |
| 10 | F243L | R292P | | | | **4.0** | 1.7 | **0.5** | **0.2** |
| 11 | | R292P | | V305I | | 1.3 | 1.3 | **0.8** | **0.4** |
| **Three Amino Acids** | | | | | | | | | |
| 12 | F243L | R292P | Y300L | | | **7.4** | **4.6** | 1.0 | 0.6 |
| 13 | F243L | R292P | | V305I | | **2.6** | 1.4 | **0.2** | **0.1** |
| 14 | F243L | R292P | | | P396L | **6.3** | **3.4** | 1.4 | **0.4** |
| 15 | | R292P | | V305I | P396L | **1.9** | **1.9** | 1.5 | 0.9 |
| **Four Amino Acids** | | | | | | | | | |
| 16 | F243L | R292P | Y300L | | P396L | **10.1** | **5.6** | 1.7 | 1.1 |
| 17 | F243L | R292P | | V305I | P396L | **4.0** | **2.3** | 0.8 | **0.4** |
| **Five Amino Acids** | | | | | | | | | |
| 18 | F243L | R292P | Y300L | V305I | P396L | **10.1** | **8.3** | **3.2** | 1.4 |

Abbreviations: nd, no detectable binding; nt, not tested. Values with ≥ 80% difference (≥ 0.8 fold) from wild-type in either direction are in bold. Shading denotes Fc mutants identified directly by yeast display; all other mutants were constructed by site-directed mutagenesis.

There is also extensive guidance in the Antibody Engineering Technology Art concerning desirable modifications. Exemplary modifications that may be desirable in certain circumstances are listed below:

- In a specific embodiment, in variant Fc regions, any amino acid modifications (e.g., substitutions) at any of positions 235, 240, 241, 243, 244, 247, 262, 263, 269, 298, 328, or 330 and preferably one or more of the following residues: A240, I240, L241, L243, H244, N298, I328, V330. In a different specific embodiment, in variant Fc regions, any amino acid modifications (e.g., substitutions) at any of positions 268, 269, 270, 272, 276, 278, 283, 285, 286, 289, 292, 293, 301, 303, 305, 307, 309, 331, 333, 334, 335, 337, 338, 340, 360, 373, 376, 416, 419, 430, 434, 435, 437, 438 or 439 and preferably one or more of the following residues: H280, Q280, Y280, G290, S290, T290, Y290, N294, K295, P296, D298, N298, P298, V298, I300, L300.

- In a preferred embodiment, in variant Fc regions that bind an FcγR with an altered affinity, any amino acid modifications (e.g., substitutions) at any of positions 255, 256, 258, 267, 268, 269, 270, 272, 276, 278, 280, 283, 285, 286, 289, 290, 292, 293, 294, 295, 296, 298, 300, 301, 303, 305, 307, 309, 312, 320, 322, 326, 329, 330, 332, 331, 333, 334, 335, 337, 338, 339, 340, 359, 360, 373, 376, 416, 419, 430, 434, 435, 437, 438, 439. Preferably, the variant Fc region has any of the following residues: A256, N268, Q272, D286, Q286, S286, A290, S290, A298, M301, A312, E320, M320, Q320, R320, E322, A326, D326, E326, N326, S326, K330, T339, A333, A334, E334, H334, L334, M334, Q334, V334, K335, Q335, A359, A360, A430.

- In a different embodiment, in variant Fc regions that bind an FcγR (via its Fc region) with a reduced affinity, any amino acid modifications (e.g., substitutions) at any of positions 252, 254, 265, 268, 269, 270, 278, 289, 292, 293, 294, 295, 296, 298, 300, 301, 303, 322, 324, 327, 329, 333, 335, 338, 340, 373, 376, 382, 388, 389, 414, 416, 419, 434, 435, 437, 438, or 439.

- In a different embodiment, in variant Fc regions that bind an FcγR (via its Fc region) with an enhanced affinity, any amino acid modifications (e.g., substitutions) at any of positions 280, 283, 285, 286, 290, 294, 295, 298, 300, 301, 305, 307, 309, 312, 315, 331, 333, 334, 337, 340, 360, 378, 398, or 430. In a different embodiment, in variant Fc regions that binds FcγRIIA with an enhanced affinity, any of the following residues: A255, A256, A258, A267, A268, N268, A272, Q272, A276, A280, A283, A285, A286, D286, Q286, S286, A290, S290, M301, E320, M320, Q320, R320, E322, A326, D326, E326, S326, K330, A331, Q335, A337, A430.

In other embodiments, the invention encompasses the use of any Fc variant known in the art, such as those disclosed in Jefferis et al. (2002) Immunol Lett 82:57-65; Presta et al. (2002) Biochem Soc Trans 30:487-90; Idusogie et al. (2001) J Immunol 166:2571-75; Shields et al. (2001) J Biol Chem 276:6591-6604; Idusogie et al. (2000) J Immunol 164:4178-84; Reddy et al. (2000) J Immunol 164:1925-33; Xu et al. (2000) Cell Immunol 200:16-26; Armour et al. (1999) Eur J Immunol 29:2613-24; Jefferis et al. (1996) Immunol Lett 54:101-04; Lund et al. (1996) J Immunol 157:4963-69; Hutchins et al. (1995) Proc. Natl. Acad. Sci. (U.S.A.) 92:11980-84; Jefferis et al. (1995) Immunol Lett. 44:111-17; Lund et al. (1995) FASEB J 9:115-19; Alegre et al. (1994) Transplantation 57:1537-43; Lund et al. (1992) Mol Immunol 29:53-59; Lund et al. (1991) J. Immunol 147:2657-62; Duncan et al. (1988) Nature 332:563-64; US Patent Nos. 5,624,821; 5,885,573; 6,194,551; 7,276,586; and 7,317,091; and PCT Publications WO 00/42072 and PCT WO 99/58572.

Effector function can be modified by techniques such as those described in the Antibody Engineering Technology Art, or by other means. For example, cysteine residue(s) may be introduced in the Fc region, thereby allowing interchain disulfide bond formation in this region, resulting in the generation of a homodimeric antibody that may have improved internalization capability and/or increased complement-mediated cell killing and ADCC. See Caron et al. (1992) J. Exp Med. 176:1191-1195; and B. Shopes (1992) J. Immunol. 148:2918-2922. Homodimeric antibodies with enhanced anti-tumor activity may also be prepared using heterobifunctional crosslinkers as described in Wolff et al. (1993) Cancer Research 53:2560-65. Alternatively, an antibody can be engineered which has dual Fc regions and may thereby have enhanced complement lysis and ADCC capabilities. Stevenson et al. (1989) Anti-Cancer Drug Design 3:219-230.

## B2. Sequence Modifications

Generally, sequence modifications may be the substitution, deletion, or addition of one or more residues in the antibody or polypeptide that results in a change in the amino acid sequence as compared to the native sequence. Guidance in determining which amino acid residue may be inserted, substituted or deleted without adversely affecting the desired activity may be found by comparing the sequence of the antibody or polypeptide with that of homologous known protein molecules and minimizing the number of amino acid sequence changes made in regions of high homology. The variation allowed may be determined by systematically making insertions, deletions or substitutions of amino acids in the sequence and testing the resulting variants for activity exhibited by the full-length or mature native sequence.

Amino acid substitutions may involve the conservative or non-conservative substitution of one or more residues. Such substitutions are well-known in the art, for example a conservative substitution entails replacing an amino acid with another amino acid having similar structural and/or chemical properties, such as the replacement of a leucine with a serine. Non-conservative substitutions generally entail replacing an amino acid with another amino acid having different structural and/or chemical properties, for example an acidic amino acid (e.g., Glu) may be replaced with a basic amino acid (e.g., Asn).

A particularly preferred type of substitutional variant involves substituting one or more hypervariable region residues of a parent antibody (e.g., a humanized or human antibody), in order to obtain a variant antibody having improved biological properties relative to the parent antibody. A convenient way for generating such substitutional variants involves affinity maturation using phage display. Briefly, several hypervariable region sites are mutated to generate all possible amino substitutions at each site, the antibody variants thus generated are displayed on phage, and the phage-displayed variants are then screened for their biological activity (e.g., binding affinity). In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and its antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

The modification may also involve the incorporation (e.g., by substitution or addition) of unnatural amino acids, for example by methods such as those described in, e.g., Wang et al. (2002) Chem. Comm. 1:1-11; Wang et al. (2001) Science 292:498-500; and van Hest et al. (2001) Chem. Comm. 19:1897-1904. Alternative strategies focus on the enzymes responsible for the biosynthesis of amino acyl-tRNA, as described in, e.g., Tang et al. (2001) J. Am. Chem. 123(44):11089-11090; and Kiick et al. (2001) FEBS Lett. 505(3):465.

In a preferred embodiment, 2, 3, 4, 5, 6, 7, 8, 9, 10, 11, 12, 13, 14, or 15 amino acid residues have been modified. Additionally or alternatively, such modifications may be characterized as having no more than 2, 3, 4, 5, 6, 7, 8, 9, 10, 11, 12, 13, 14, or 15 modified amino acid residues. Additionally or alternatively, such modifications may be characterized as having no more than 15, 14, 13, 12, 11, 10, 9, 8, 7, 6, 5, 4, 3 or 2 modified amino acid residues. The modifications may be all substitutions, or any combination of substitutions, deletions, or additions.

Nucleic acid molecules encoding amino acid sequence variants may be prepared by a variety of methods known in the art. These methods include, but are not limited to, isolation from a natural source (in the case of naturally occurring amino acid sequence variants) or preparation by oligonucleotide-mediated (or site-directed) mutagenesis, restriction selection mutagenesis, PCR mutagenesis, and cassette mutagenesis of an earlier prepared variant or a non-variant version of the antibody.

### B3. Other Modifications

The polypeptide variants (and especially antibody variants) of the present invention include analogs and derivatives that are modified, e.g., by the covalent attachment of any type of molecule as long as such covalent attachment permits the antibody to retain its epitope binding immunospecificity. For example, but not by way of limitation, the derivatives and analogs of the antibodies include those that have been further modified, e.g., by glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques, including, but not limited to specific chemical cleavage, acetylation, formylation, metabolic synthesis in the presence of tunicamycin, etc. Additionally, the analog or derivative can contain one or more unnatural amino acids.

The antibodies and polypeptides may be modified by introducing one or more glycosylation sites into the antibodies, deleting one or more glycosylation sites from the antibodies, or shifting an existing glycosylation site on the antibodies, preferably without altering the desired functionality of the antibodies, e.g., binding activity. Glycosylation sites may be introduced into, or deleted from, the variable and/or constant region of the antibodies, by methods known in the art. For example, a glycosylation site may be introduced into an antibody of the invention by modifying or mutating an amino acid sequence of the antibody so that the desired sequence (e.g., Asn-X-Thr/Ser) is obtained, and a glycosylation site may be shifted by modifying position 296 in the Fc region, so that position 296 and not position 297 is glycosylated. Methods of modifying the carbohydrate content (glycosylation) of proteins are well known in the art, for example as described in U.S. Patent Nos. 6,472,511 and 6,218,149; U.S. Patent Publication Nos. 20030115614 and 20020028486; EP 0359096 B1; and WO 03/035835.

In some embodiments, molecules of the invention are engineered to comprise an altered glycosylation pattern or an altered glycoform. Engineered glycoforms may be useful for a variety of purposes, including, but not limited to, enhancing effector function. Engineered glycoforms may be generated by any method known to one skilled in the art, for example by using engineered or variant expression strains, by co-expression with one or more enzymes, for example, N-acetylglucosaminyltransferase III (GnT-III), by expressing an antibody of the invention in various organisms or cell lines from various organisms, or by modifying carbohydrate(s) after the antibody has been expressed and purified. Methods for generating engineered glycoforms are known in the art, and include but are not limited to those described in, e.g., Okazaki et al. (2004) JMB 336:1239-1249; Shinkawa et al. (2003) J Biol Chem 278:3466-3473; Shields et al. (2002) J Biol Chem 277:26733-26740; Davies et al. (2001) Biotechnol Bioeng 74:288-294; Umana et al. (1999) Nat. Biotechnol 17:176-180; U.S. Patent No. 6,602,684; U.S. Patent Publication Nos. 20030157108, 20030115614, and 20030003097; WO 02/311140; WO 02/30954; WO 01/292246; WO 00/61739; Potillegent™ technology available from Biowa, Inc. (Princeton, NJ); and GlycoMAb™ glycosylation engineering technology available from GLYCART biotechnology AG (Zurich, Switzerland).

### B4. Polypeptide Conjugates

The polypeptides of the present invention may be recombinantly fused or chemically conjugated (including both covalent and non-covalent conjugations) to heterologous polypeptides or portions thereof to generate fusion proteins. Preferably, the polypeptide of the present invention (especially an antibody) is fused to at least 10, at least 15, at least 20, at least 25, at least 30, at least 40, at least 50, at least 60, at least 70, at least 80, at least 90 or at least 100 amino acids of the heterologous polypeptide to generate a desired fusion protein. The fusion does not necessarily need to be direct, but may occur through linker sequences. Polypeptides of the present invention may also be attached to solid supports or semi-solid matrices, which are particularly useful for immunoassays or purification of the target antigen. Such supports and matrices include, but are not limited to, glass, cellulose, polyacrylamide, agarose beads, acrylamide beads, nylon, polystyrene, polyvinyl chloride or polypropylene. Attachment may be accomplished, for example, by methods described in Methods in Enzymology, 44 (1976).

The antibodies and polypeptides may be conjugated to a therapeutic agent in order to modify a given biological response, affect (e.g., increase) the serum half-life of the therapeutic agent, or target the therapeutic agent to a particular subset of cells. They may also be fused to marker sequences (e.g., a hexa-histidine peptide or a "flag" tag) to faciliate purification. Techniques for conjugating such therapeutic moieties to antibodies are well known; see, e.g., Hellstrom et al., "Antibodies For Drug Delivery", in Controlled Drug Delivery (2nd ed., Robinson et al. (eds.), 1987, pp. 623-53, Marcel Dekker, Inc.).

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of molecules of the invention (e.g., antibodies with higher affinities and lower dissociation rates). Antibodies and polypeptides of the invention, or their encoding nucleic acids, may be further altered by being subjected to random mutagenesis by error-prone PCR, random nucleotide insertion or other methods prior to recombination. One or more portions of a polynucleotide encoding a molecule of the invention, may be recombined with one or more components, motifs, sections, parts, domains, fragments, etc. of one or more heterologous molecules.

## B5. Fragments

The invention additionally provides antibody and other polypeptide fragments. Such fragments may be truncated at the N-terminus or C-terminus, or may lack internal residues, for example, when compared with a full length native antibody or protein. Certain fragments may lack amino acid residues that are not essential for a desired biological activity. These fragments may be prepared by any of a number of conventional techniques. Desired peptide fragments may be chemically synthesized. An alternative approach involves generating antibody or polypeptide fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired antibody or polypeptide fragment, by polymerase chain reaction (PCR). Oligonucleotides that define the desired termini of the DNA fragment are employed at the 5' and 3' primers in the PCR. Preferably, antibody and polypeptide fragments share at least one biological and/or immunological activity with the native antibody or polypeptide disclosed herein.

In some embodiments, a polypeptide of the invention further comprises a dimerization domain, which can comprise a dimerization sequence, and/or sequence comprising one or more cysteine residues. In some embodiments, the dimerization domain will be located between an antibody heavy chain or light chain variable domain and at least a portion of a viral coat protein, and one or more disulfide bond and/or a single dimerization sequence may be present in the dimerization domain to provide for bivalent display. In some embodiments, heavy chains of an $F(ab)_2$ will dimerize at a dimerization domain not including a hinge region. The dimerization domain may comprise a leucine zipper sequence.

In another embodiment, the polypeptide fragments of the present invention comprise an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 30 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide. In a specific embodiment, a fragment of a polypeptide retains at least one function of the polypeptide.

## B6. Diabodies and DARTs

Diabodies and dual affinity retargeting reagents ("DARTs") are also provided by the present invention. The diabodies and DARTs comprise antigen binding domains generally derived from the antibodies and polypeptides of the invention. The design and construction of diabodies and DARTs is described in, for example, U.S. Patent Application No. 20070004909 published on January 4, 2007; Marvin et al. (2005) Acta Pharmacol. Sin. 26:649-658; Olafsen et al. (2004) Prot. Engr. Des. Sel. 17:21-27; Holliger et al. (1993) Proc. Natl. Acad. Sci. (U.S.A.) 90:6444-6448. Each polypeptide chain of a diabody molecule comprises a $V_L$ domain and a $V_H$ domain, from the same or different antibodies, which are covalently linked such that the domains are constrained from self assembly. Interaction of two of the polypeptide chains will produce two $V_L$-$V_H$ pairings, forming two epitope binding sites, i.e., a bivalent molecule. Neither the $V_H$ or $V_L$ domain is constrained to any position within the polypeptide chain, nor are the domains restricted in their relative positions to one another; the only restriction is that a complementary polypeptide chain be available in order to form functional diabody. The domains may be separated by a peptide linker, and the polypeptide chains may be engineered to comprise at least one cysteine residue on each chain, so that interchain disulfide bonds may be formed to stabilize the diabody.

Where the $V_L$ and $V_H$ domains are derived from the same antibody, the two complementary polypeptide chains may be identical, resulting in a bivalent monospecific antibody, or may be different, resulting in a bivalent bispecific antibody (e.g., one that binds ton two different epitopes on the same antigen). Where the $V_L$ and $V_H$ domains are derived from antibodies specific for different antigens, formation of a functional bispecific diabody requires the interaction of two different polypeptide chains, i.e., formation of a heterodimer. In a particular embodiment, at least one epitope binding site of the diabody is specific for an antigen on a particular cell, such as a B-cell or T-cell, a phagocytotic cell, a natural killer (NK) cell or a dendritic cell.

In various embodiments, one or more of the polypeptide chains of the diabody comprises an Fc domain. Fc domains in the polypeptide chains of the diabody molecules preferentially dimerize, resulting in the formation of a diabody molecule that exhibits immunoglobulin-like properties, e.g., Fc-FcγR interactions. Fc comprising diabodies may be dimers, e.g., comprised of two polypeptide chains, each comprising a $V_H$ domain, a $V_L$ domain and an Fc domain. In various embodiments, one or more of the polypeptide chains of the diabody comprises a hinge domain, which may be derived from any immunoglobulin isotype or allotype including IgA, IgD, IgG, IgE and IgM. In preferred embodiments, the hinge domain is derived from IgG, wherein the IgG isotype is IgG1, IgG2, IgG3 or IgG4, or an allotype thereof. The hinge domain may be engineered into a polypeptide chain in any position relative to other domains or portions of the chain, and in certain circumstances may be engineered together with an Fc domain such that the diabody molecule comprises a hinge-Fc domain.

In other embodiments, diabody molecules comprising Fc domains may be tetramers, which may comprise two "heavier" polypeptide chains (i.e. a polypeptide chain comprising a $V_L$, a $V_H$ and an Fc domain), and two "lighter" polypeptide chains (i.e., a polypeptide chain comprising a $V_L$ and a $V_H$). Such lighter and heavier chains may interact to form a monomer, and interact via their unpaired Fc domains to form an Ig-like molecule, which may be a DART molecule. Such an Ig-like diabody is tetravalent and may be monospecific, bispecific or tetraspecific. The Ig-like DART species has unique properties, because its domains may be designed to bind to the same epitope (so as to form a tetravalent, mono-epitope specific Ig-like DART capable of binding four identical antigen molecules), or to different epitopes or antigens. For example, its domains may be designed to bind to two epitopes of the same antigen (so as to form a tetravalent, mono-antigen specific, bi-epitope specific Ig-like DART), or to epitopes of different antigen molecules so as to form a tetravalent Ig-like DART having a pair of binding sites specific for a first antigen and a second pair of binding sites specific for a second antigen). Hybrid molecules having combinations of such attributes can be readily produced.

Although not intending to be bound by a particular mechanism of action, the diabody molecules of the invention exhibit enhanced therapeutic efficacy relative to therapeutic antibodies known in the art, in part, due to the ability of diabody to immunospecifically bind a target cell which expresses a particular antigen (e.g., FcγR) at reduced levels, for example, by virtue of the ability of the diabody to remain on the target cell longer due to an improved avidity of the diabody-epitope interaction. Thus, the diabodies of the invention have particular utility in treatment, prevention or management of a disease or disorder, such as cancer, in a sub-population, wherein the target antigen is expressed at low levels in the target cell population.

Due to their increased valency, low dissociation rates and rapid clearance from the circulation (for diabodies of small size, at or below ~50 kDa), diabody molecules known in the art have also shown particular use in the field of tumor imaging (Fitzgerald et al. (1997) Protein Eng. 10:1221). Of particular importance is the cross linking of differing cells, for example the cross linking of cytotoxic T cells to tumor cells (Staerz et al. (1985) Nature 314:628-31; Holliger et al. (1996) Protein Eng. 9:299-305). Diabody epitope binding domains may also be directed to a surface determinant of any immune effector cell such as CD3, CD16, CD32, or CD64, which are expressed on T lymphocytes, natural killer (NK) cells or other mononuclear cells. In many studies, diabody binding to effector cell determinants, e.g., Fcγ receptors (FcγR), was also found to activate the effector cell (Holliger et al. (1996) Protein Eng. 9:299-305; Holliger et al. (1999) Cancer Res. 59:2909-2916). Normally, effector cell activation is triggered by the binding of an antigen bound antibody to an effector cell via Fc-FcγR interaction; thus, in this regard, diabody molecules of the invention may exhibit Ig-like functionality independent of whether they comprise an Fc domain. By cross-linking tumor and effector cells, the diabody not only brings the effector cell within the proximity of the tumor cells but leads to effective tumor killing. Cao and Lam (2003) Adv. Drug. Deliv. Rev. 55:171-97.

The diabody molecules of the present invention can be produced using a variety of methods, including de novo protein synthesis and recombinant expression of nucleic acids encoding the binding proteins. The desired nucleic acid sequences can be produced by recombinant methods (e.g., PCR mutagenesis of an earlier prepared variant of the desired polynucleotide) or by solid-phase DNA synthesis. Preferably recombinant expression methods are used. In one aspect, the invention provides a polynucleotide that comprises a sequence encoding a CD16A $V_H$ and/or $V_L$; in another aspect, the invention provides a polynucleotide that comprises a sequence encoding a CD32B $V_H$ and/or $V_L$. Because of the degeneracy of the genetic code, a variety of nucleic acid sequences encode each immunoglobulin amino acid sequence, and the present invention includes all nucleic acids encoding the binding proteins described herein.

## B7. Production of Antibodies

Antibodies may be produced or obtained in any of a variety of ways. For example, such antibodies may be obtained from plasma, synthetically, recombinantly or transgenically, via cell (e.g., hybridoma culture), etc. The production of synthetic proteins has been described in, e.g., Dawson et al. (2000) Ann. Rev Biochem. 69:923-960; Wilken et al. (1998) Curr. Opin. Biotechnol. 9(4):412-426; and Kochendoerfer et al. (1999) Curr. Opin. Chem. Biol. 3(6):665-671.

Production of recombinant and transgenic antibodies has been described in, e.g., Wang et al. (2007) IDrugs 10(8):562-565; Hagemeyer et al. (2007) Semin. Thromb. Hemost. 33(2):185-195; Rasmussen et al. (2007) Biotechnol. Lett. 29(6):845-852; Gasser et al. (2007) Biotechnol. Lett. 29(2):201-212; Aubrey et al. (2006) J. Soc. Biol. 200(4):345-354; Laffly et al. (2006) J. Soc. Biol. 200(4):325-343; Jefferis (2005) Biotechnol Prog. 21(1):11-16; Smith et al. (2004) J. Clin. Pathol. 57(9):912-917; Kipriyanov et al. (2004) Mol Biotechnol. 26(1):39-60; Fischer et al. (2003) Vaccine 21(7-8):820-825; Maynard et al. (2000) Ann. Rev. Biomed. Eng. 2:339-376; Young et al. (1998) Res. Immunol. 149(6):609-610; and Hudson (1998) Curr. Opin. Biotechnol. 9(4):395-402.

Production of antibodies via cell (e.g., hybridoma) culture has been described in, e.g., Laffly *et al.* (2006), *supra*; Aldington et al. (2007) J. Chromatogr. B Analyt. Technol. Biomed. Life Sci. 848(1):64-78; S.S. Farid (2006) J. Chromatogr. B Analyt. Technol. Biomed. Life Sci. 848(1):8-18; Birch et al. (2006) Adv. Drug Deliv. Rev. 58(5-6):671-685; Even et al. (2006) Trends Biotechnol. 24(3):105-108; Graumann et al. (2006) Biotechnol. J. 1(2):164-86; U.S. Patent No. 7,112,439; and U.S. Patent Publications Nos. 20070037216 and 20040197866.

Antibodies may be produced via phage display methods, such as those disclosed in, e.g., Brinkman et al. (1995) J. Immunol. Methods 182:41-50; Ames et al. (1995) J. Immunol. Methods 184:177-86; Kettleborough et al. (1994) Eur. J. Immunol. 24:952-58; Persic et al. (1997) Gene 187:9-18; Burton et al. (1994) Advances in Immunology 57:191-280; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. Phage display technology can also be used to increase the affinity of an antibody for its antigen. The technology, referred to as affinity maturation, employs mutagenesis or CDR walking and re-selection using the cognate antigen to identify antibodies that bind with higher affinity to the antigen when compared with the initial or parental antibody. See, e.g., Glaser et al. (1992) J. Immunology 149:3903; Wu et al. (1998) Proc. Natl. Acad. Sci. (U.S.A.) 95:6037; Yelton et al. (1995) J. Immunology 155:1994; Schier et al. (1996) J. Mol. Bio. 263:551.

Monoclonal antibodies may be made by a variety of methods known to those skilled in the art, for example, hybridoma methods as described in, e.g., Kohler et al. (1975) Nature 256:495, Kozbor et al. (1983) Immunology Today 4:72, or Cole et al. (1985) Monoclonal Antibodies and Cancer Therapy, Alan R. Liss, Inc., pp. 77-96, or recombinant DNA methods as described in, e.g., U.S. Pat. No. 4,816,567, or the antibodies may be isolated from phage antibody libraries using the techniques described in Clackson et al. (1991) Nature 352:624-628 and Marks et al. (1991) J. Mol. Biol. 222:581-597, for example. Various procedures well known in the art may be used for the production of polyclonal antibodies to an antigen of interest. For example, various host animals can be immunized by injection with an antigen of interest or derivative thereof, including but not limited to rabbits, sheep, goats, dogs, mice, rats, and guinea pigs, and after allowing for an immunological response, the antibodies can be identified from the sera of the immunized animals.

Bispecific antibodies may also be made, for example through the co-expression of two immunoglobulin heavy chain-light chain pairs, where the two chains have different specificities, followed by purification of the desired molecule using affinity chromatography, as described by Milstein et al. (1983) Nature 305:537-39, WO 93/08829, Traunecker et al. (1991) EMBO J. 10:3655-59. In a different approach, antibody variable domains with the desired binding specificities (antibody-antigen combining sites) are fused to immunoglobulin constant domain sequences, for example to a heavy chain constant domain, comprising at least part of the hinge, $C_{H2}$, and $C_{H3}$ regions. The nucleic acids encoding these fusions may be inserted into the same or different expression vectors, and are expressed in a suitable host organism.

Fully human antibodies (also referred to as completely human antibodies) may be produced using transgenic mice that are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. An overview of this technology for producing human antibodies is described in, for example, Lonberg and Huszar (1995) Int. Rev. Immunol. 13:65-93, and U.S. Patent No. 5,633,425. Fully human antibodies can also be produced using other techniques known in the art, including phage display libraries, as described by Hoogenboom and Winter (1991) J. Mol. Biol. 227:381 and Marks et al. (1991) J. Mol. Biol. 222:581. Fully human antibodies may also be obtained commercially from, for example, Abgenix, Inc. (Freemont, Calif.) and Genpharm (San Jose, Calif.). Fully human antibodies that recognize a selected epitope may be generated using a technique referred to as "guided selection." In this approach a selected non-human monoclonal antibody, e.g., a mouse antibody, is used to guide the selection of a completely human antibody recognizing the same epitope, as described by, e.g., Jespers et al. (1994) Biotechnology 12:899-903.

The present invention also includes polynucleotides that encode the polypeptide molecules of the invention. The polynucleotides encoding the molecules of the invention may be obtained, and the nucleotide sequence of the polynucleotides determined, by any method known in the art, for example, recombinant DNA techniques, site directed mutagenesis, PCR, etc. In one embodiment, human libraries or any other libraries available in the art, can be screened by standard techniques known in the art, to clone the nucleic acids encoding the molecules of the invention.

**B8. Characterization of Antibodies**

The antibodies of the present invention may be characterized in a variety of ways. In particular, antibodies of the invention may be assayed for the ability to immunospecifically bind to an antigen, e.g., HER2/neu, or, where the molecule comprises an Fc domain (or portion thereof) for the ability to exhibit Fc-FcγR interactions, i.e. specific binding of an Fc domain (or portion thereof) to an FcγR. Such an assay may be performed in solution (e.g., Houghten (1992) Bio/Techniques 13:412-421), on beads (Lam (1991) Nature 354:82-84), on chips (Fodor (1993) Nature 364:555-556), on bacteria (U.S. Patent No. 5,223,409), on spores (U.S. Patent Nos. 5,571,698; 5,403,484; and 5,223,409), on plasmids (Cull et al. (1992) Proc. Natl. Acad. Sci. (U.S.A.) 89:1865-1869) or on phage (Scott and Smith (1990) Science 249:386-390; Devlin (1990) Science 249:404-406; Cwirla et al. (1990) Proc. Natl. Acad. Sci. (U.S.A.) 87:6378-6382; and Felici (1991) J. Mol. Biol. 222:301-310). Molecules that have been identified to immunospecifically bind to an antigen can then be assayed for their specificity and affinity for the antigen.

Immunoassays which can be used to analyze immunospecific binding, cross-reactivity, and Fc-FcγR interactions include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunochromatographic assays, immunodiffusion assays, agglutination assays, complement-fixation assays, immunoradiometric assays, fluorescent immunoassays, and protein A immunoassays, etc. (see, e.g., Ausubel et al., 2008, Current Protocols in Molecular Biology).

Binding affinity for a target antigen is typically measured or determined by standard antibody-antigen assays, such as Biacore competitive assays, saturation assays, or immunoassays such as ELISA or RIA.

Preferably, fluorescence activated cell sorting (FACS), using any of the techniques known to those skilled in the art, is used for immunological or functional based assays to characterize molecules of the invention. Flow sorters are capable of rapidly examining a large number of individual cells that have been bound, e.g., opsonized, by molecules of the invention (e.g., 10-100 million cells per hour). Additionally, specific parameters used for optimization of antibody behavior, include but are not limited to, antigen concentration, kinetic competition time, or FACS stringency, each of which may be varied in order to select for antibody molecules which exhibit specific binding properties. Flow cytometers for sorting and examining biological cells are well known in the art. Known flow cytometers are described, for example, in U.S. Patent Nos. 4,347,935; 5,464,581; 5,483,469; 5,602,039; 5,643,796; and 6,211,477. Other known flow cytometers are the FACS Vantage™ system sold by Becton Dickinson and Company, and the COPAS™ system sold by Union Biometrica.

Surface plasmon resonance-based assays may be used to characterize the kinetic parameters of an antigen-binding domain or Fc-FcγR binding. Any method known to those skilled in the art may be used, for example the technology described in, e.g., Dong et al. (2002) Review in Mol. Biotech. 82:303-323; Mullet et al. (2000) Methods 22:77-91; Rich et al. (2000) Current Opinion in Biotechnology 11:54-61; Fivash et al. (1998) Current Opinion in Biotechnology 9:97-101; and U.S. Patent Nos. 6,373,577; 6,289,286; 5,322,798; 5,341,215; and 6,268,125. The data is used to plot binding curves and determine rate constants, for example, $K_{on}$, $K_{off}$, and the apparent equilibrium binding constant $K_d$, for example as described in, e.g., Myszka (1997) Current Opinion in Biotechnology 8:50-57; O'Shannessy et al. (1996) Analytical Biochemistry 236:275-283; Morton et al. (1995) Analytical Biochemistry 227:176-185; Fisher et al. (1994) Current Opinion in Biotechnology 5:389-95; O'Shannessy (1994) Current Opinion in Biotechnology 5:65-71; and Chaiken et al. (1992) Analytical Biochemistry 201:197-210. In preferred embodiments, the kinetic parameters determined using an SPR analysis may be used as a predictive measure of how a molecule will function in a functional assay, e.g., ADCC.

Characterization of binding to FcγR by molecules comprising an Fc domain (or portion thereof) and/or comprising epitope binding domain specific for an FcγR may be performed according to the methods described in the Antibody Engineering Technology Art. Assays for effector cell functions are well-known, for example as described in Abdul-Majid et al. (2002) Scand. J. Immunol. 55:70-81; Perussia et al. (2000) Methods Mol. Biol. 121:179-192; Lehmann et al. (2000) J. Immunol. Methods 243(1-2):229-242; Ding et al. (1998) Immunity 8:403-411; Baggiolini et al. (1998) Experientia 44(10):841-848; Brown (1994) Methods Cell Biol. 45:147-164; and Munn et al. (1990) J. Exp. Med. 172:231-237.

For example, assays for FcγR-mediated phagocytosis may be conducted using human monocytes, by measuring the ability of THP-1 cells to phagocytose fluoresceinated IgG-opsonized sheep red blood cells (SRBC) by methods previously described in Tridandapani et al. (2000) J. Biol. Chem. 275:20480-20487, or using an antibody-dependent opsonophagocytosis assay (ADCP) as described by Bedzyk et al. (1989) J. Biol. Chem. 264(3):1565-1569. Standard methods known to those skilled in the art may be used to characterize the binding of C1q and mediation of complement dependent cytotoxicity (CDC) by molecules of the invention comprising Fc domains (or portions thereof). For example, to determine C1q binding, a C1q binding ELISA may be performed, and to assess complement activation, a complement dependent cytotoxicity (CDC) assay may be performed, e.g., as described in Gazzano-Santoro et al. (1996) J. Immunol. Methods 202:163.

In another embodiment, the molecules of the invention can be assayed for FcγR-mediated ADCC activity in effector cells, e.g., natural killer cells, using any of the standard methods known to those skilled in the art and described in, e.g., Weng et al. (2003) J. Clin. Oncol. 21:3940-3947; Perussia et al. (2000) Methods Mol. Biol. 121:179-192; Ding et al. (1998) Immunity 8:403-411. In a specific preferred embodiment, a time resolved fluorimetric assay is used for measuring ADCC activity against fluorescently-labeled target cells, as described in, e.g., Blomberg et al. (1996) Journal of Immunological Methods 193:199-206. Target cells used in the ADCC assays of the invention include, but are not limited to, breast cancer cell lines, e.g., SK-BR-3 with ATCC accession number HTB-30 (Tremp et al. (1976) Cancer Res. 33-41); B-lymphocytes; cells derived from Burkitts lymphoma, e.g., Raji cells with ATCC accession number CCL-86 (Epstein et al. (1965) J. Natl. Cancer Inst. 34:231-240), and Daudi cells with ATCC accession number CCL-213 (Klein et al. (1968) Cancer Res. 28:1300-1310). The target cells must be recognized by the antigen binding site of the molecule to be assayed. Preferably, the effector cells used in the ADCC assays of the invention are peripheral blood mononuclear cells (PBMC) that are preferably purified from normal human blood, using standard methods known to one skilled in the art, e.g., using Ficoll-Paque density gradient centrifugation.

## C. METHODS OF TREATMENT & PHARMACEUTICAL COMPOSITIONS

The administration of the compositions (e.g., antibodies and polypeptides) of the present invention may be for a "prophylactic" or "therapeutic" purpose, or alternatively can be used for diagnostic purposes. The compositions of the present invention are said to be administered for a "therapeutic" purpose if the amount administered is physiologically significant to provide a therapy for an actual manifestation of the disease. When provided therapeutically, the compound is preferably provided at (or shortly after) the identification of a symptom of actual disease. The therapeutic administration of the compound serves to attenuate the severity of such disease or to reverse its progress. The compositions of the present invention are said to be administered for a "prophylactic" purpose if the amount administered is physiologically significant to provide a therapy for a potential disease or condition. When provided prophylactically, the compound is preferably provided in advance of any symptom thereof. The prophylactic administration of the compound serves to prevent or attenuate any subsequent advance or recurrence of the disease.

Providing a therapy or "treating" refers to any indicia of success in the treatment or amelioration of an injury, pathology or condition, including any objective or subjective parameter such as abatement, remission, diminishing of symptoms or making the injury, pathology or condition more tolerable to the patient, slowing in the rate of degeneration or decline, making the final point of degeneration less debilitating, or improving a patient's physical or mental well-being. The treatment or amelioration of symptoms can be based on objective or subjective parameters, including the results of a physical examination, neuropsychiatric exams, and/or a psychiatric evaluation.

Preferred subjects for treatment include animals, most preferably mammalian species such as humans or other primates, and domestic animals such as dogs, cats and the like, subject to disease and other pathological conditions. A "patient" refers to a subject, preferably mammalian (including human).

The term "therapeutic agent" refers to any agent having a therapeutic effect to prophylactically or therapeutically treat a disorder. Exemplary therapeutic agents include the antibodies and polypeptides of the present invention, as well as other therapeutic agents that may be administered in combination with, or conjugated to, an antibody or polypeptide. In a preferred embodiment, the therapeutic agent is an antibody of the present invention, and preferably is an antibody fragment, a diabody, an Ig-like DART, or a fusion protein.

The molecules of the invention are particularly useful for the treatment and/or prevention of a disease, disorder or infection where enhanced binding to FcRn and/or increased serum half-life mediated by FcRn is desired (e.g., cancer, infectious disease, allergies, etc.). For example, molecules of the invention exhibiting enhanced binding to FcRn, particularly enhanced pH dependent-binding to FcRn, exhibit increased bioavailability following administration, particularly gastrointestinal, genital, nasal, ocular, oral, pulmonary, and rectal administration. Further, molecules of the invention exhibiting increased serum half-lives are particularly useful in the treatment of disease, disorders or infections where increased and sustained exposure to the therapeutic agent is beneficial, because the longer serum half-life generally enables the therapeutic to be administered less frequently, and at a lower dose.

In some embodiment, the molecules of the invention are also particularly useful for the treatment and/or prevention of a disease, disorder or infection where an effector cell function (e.g., ADCC) mediated by FcγR is desired (e.g., cancer, infectious disease). For example, molecules of the invention may bind a cell surface antigen and an FcγR (e.g., FcγRIIIA) on an immune effector cell (e.g., NK cell), stimulating an effector function (e.g., ADCC, CDC, phagocytosis, opsonization, etc.) against said cell. In some embodiments, the antibodies and polypeptides of the invention are especially suited for the treatment of cancers. The efficacy of standard monoclonal antibody therapy depends on the FcγR polymorphism of the subject. (Carton et al. (2002) Blood 99:754-758; Weng et al. (2003) J Clin Oncol. 21(21):3940-3947). These receptors are expressed on the surface of the effector cells and mediate ADCC. High affinity alleles improve the effector cells' ability to mediate ADCC. The antibodies and polypeptides of the invention may comprise a variant Fc domain that exhibits enhanced affinity to FcγR (relative to a wild type Fc domain) on effector cells, thus providing better immunotherapy reagents for patients regardless of their FcγR polymorphism.

For diagnostic purposes, the antibodies or polypeptides may be coupled to a detectable substance, so that they can be used, for example, to monitor the development or progression of a disease, disorder or infection. Examples of detectable substances include various enzymes (e.g., horseradish peroxidase, beta-galactosidase, etc.), prosthetic groups (e.g., avidin/biotin), fluorescent materials (e.g., umbelliferone, fluorescein, or phycoerythrin), luminescent materials (e.g., luminol), bioluminescent materials (e.g., luciferase or aequorin), radioactive materials (e.g., carbon-14, manganese-54, strontium-85 or zinc-65), positron emitting metals, and nonradioactive paramagnetic metal ions. The detectable substance may be coupled or conjugated either directly to the molecules of the invention or indirectly through an intermediate (e.g., a linker), using techniques known in the art.

**C1. Treatable Disorders**

Exemplary disorders that may be treated by various embodiments of the present invention include, but are not limited to, proliferative disorders, cell proliferative disorders, and cancer, autoimmune diseases, inflammatory disorders, and infectious diseases. In various embodiments, the invention encompasses methods and compositions for treatment, prevention or management of a disease or disorder in a subject, comprising administering to the subject a therapeutically effective amount of one or more molecules (antibodies or polypeptides) which bind to a disease antigen. For example, molecules of the invention are particularly useful for the prevention, inhibition, reduction of growth or regression of primary tumors, metastasis of cancer cells, and infectious diseases. Although not intending to be bound by a particular mechanism of action, molecules of the invention mediate effector function resulting in tumor clearance, tumor reduction or a combination thereof. In alternate embodiments, diabodies of the invention mediate therapeutic activity by cross-linking of cell surface antigens and/or receptors and enhanced apoptosis or negative growth regulatory signaling.

Antibodies with a decreased affinity for FcγRIIB and an increased affinity for FcγRIIIA and/or FcγRIIA may lead to an enhanced activating response upon FcγR binding and thus have therapeutic efficacy for treating and/or preventing cancer. Non-limiting examples of cancers treatable by the methods herein include acute myeloid lymphoma, adrenal carcinoma, adenocarcinoma, basal cancer, bladder cancer, bone cancer, bone and connective tissue sarcoma, brain cancer, breast cancer, bronchial cancer, cervical cancer, choriocarcinoma, chronic lymphocytic leukemia, chronic myelogenous leukemia, colon cancer, colorectal cancer, endometrial cancer, esophageal cancer, eye cancer, fallopian tube cancer, gall bladder cancer, gastrointestinal cancer, glioma, hairy cell leukemia, hepatoma, Hodgkin's disease, intrahepatic bile duct cancer, joint cancer, Kaposi's sarcoma, kidney cancer, larynx cancer, liver cancer, leukemia, lung cancer, lymphoblastic leukemia, lymphoma, malignant mesothelioma, medulloblastoma, melanoma, mesothelioma, middle ear cancer, multiple myeloma, myeloma, myxosarcoma, nasal cavity cancer, nasopharynx cancer, neuroblastoma, Non-Hodgkin's lymphoma, non-small cell lung cancer, nose cancer, oral cavity cancer, ovarian cancer, pancreatic cancer, penal cancer, peritoneum cancer, pharynx cancer, pituitary gland cancer, prostate cancer, rectal cancer, renal cancer, salivary gland cancer, skin cancer, soft tissue sarcoma, squamous cell carcinoma, stomach cancer, testicular cancer, thyroid cancer, urinary cancer, uterine cancer, vaginal cancer, vesticular cancer, vulval cancer, and Wilm's tumor.

In some embodiments, the cancer is a hematopoietic cancer or blood-related cancer, such as lymphoma, leukemia, myeloma, lymphoid malignancy, cancer of the spleen, and cancer of the lymph nodes. In a preferred embodiment, the cancer is a B-cell associated cancer, such as, for example, high, intermediate or low grade lymphoma (including B cell lymphoma such as, for example, Burkitt's lymphoma, diffuse large cell lymphoma, follicular lymphoma, Hodgkin's lymphoma, mantle cell lymphoma, marginal zone lymphoma, mucosa-associated-lymphoid tissue B cell lymphoma, non-Hodgkin's lymphoma, small lymphocytic lymphoma, and T cell lymphomas) and leukemias (including chronic lymphocytic leukemia, such as B cell leukemia (CD5+ B lymphocytes), chronic myeloid leukemia, lymphoid leukemia, such as acute lymphoblastic leukemia, myelodysplasia, myeloid leukemia, such as acute myeloid leukemia, and secondary leukemia), multiple myeloma, such as plasma cell malignancy, and other hematological and/or B cell- or T-cell-associated cancers. Other exemplary cancers are cancers of additional hematopoietic cells, including polymorphonuclear leukocytes, such as basophils, eosinophils, neutrophils and monocytes, dendritic cells, platelets, erythrocytes and natural killer cells.

In some embodiments, the cancer to be treated is breast cancer, prostate cancer, uterine cancer, ovarian cancer, colon cancer, endometrial cancer, adrenal carcinoma, or non-small cell lung cancer. In some embodiments, the cancer is breast cancer or prostate cancer. In some embodiments, the cancer is a cancer in which HER2/neu is overexpressed. In a specific embodiment, an antibody or polypeptide of the invention inhibits or reduces the growth of cancer cells by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth of cancer cells in the absence of the antibody or polypeptide of the invention.

Antibodies with an increased affinity for FcγRIIB and a decreased affinity for FcγRIIIA and/or FcγRIIA may lead to a diminished activating response upon FcγR binding and thus have therapeutic efficacy for treating and/or preventing inflammation and autoimmune disease. Examples of autoimmune diseases or autoimmune related conditions that may be treated by the methods herein include, but are not limited to, allergic conditions, allergic encephalomyelitis, allergic neuritis, allergic rhinitis, alopecia areata, ALS, anemia including aplastic anemia, Coombs positive anemia, Diamond Blackfan anemia, immune hemolytic anemia including autoimmune hemolytic anemia (AIHA), pernicious anemia, and pure red cell aplasia (PRCA), ankylosing spondylitis, antigen-antibody complex mediated diseases, anti-glomerular basement membrane disease, anti-phospholipid antibody syndrome, arthritis (e.g., rheumatoid arthritis, juvenile rheumatoid arthritis, juvenile arthritis, osteoarthritis, psoriatic arthritis), asthma, atherosclerosis, autoimmune diseases of the adrenal gland, autoimmune disease of the testis and ovary including autoimmune orchitis and oophoritis, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes also referred to as insulin-dependent diabetes mellitus (IDDM) and Sheehan's syndrome, autoimmune hepatitis, autoimmune myocarditis, autoimmune neutropenia, autoimmune polyendocrinopathies, autoimmune thrombocytopenia, Behcet's disease, Berger's Disease (IgA nephropathy), bronchiolitis obliterans (non-transplant), cardiomyopathy including coronary artery disease, Castleman's syndrome, celiac sprue (gluten enteropathy), chronic autoimmune urticaria, chronic fatigue immune dysfunction syndrome (CFIDS), chronic inflammatory demyelinating polyneuropathy, CNS inflammatory disorders, cold agglutinin disease, colitis, conditions involving infiltration of T cells and chronic inflammatory responses, cryoglobulinemia, cutaneous lupus erythematosus, dermatitis including atopic dermatitis, diseases involving leukocyte diapedesis, eczema, encephalitis, essential mixed cryoglobulinemia, Factor VIII deficiency, fibromyalgia-fibromyositis, glomerulonephritis, Goodpasture's Syndrome, graft versus host disease (GVHD), granulomatosis including Wegener's granulomatosis and agranulocytosis, Guillain-Barre Syndrome, hemophilia A, idiopathic pulmonary fibrosis, idiopathic thrombocytopenic purpura (ITP), IgA nephropathy, IgM polyneuropathies, IgA neuropathy, and IgM mediated neuropathy, immune complex nephritis, immune responses associated with acute and delayed hypersensitivity mediated by cytokines and T-lymphocytes, juvenile onset diabetes, Lambert-Eaton Myasthenic Syndrome, leukocyte adhesion deficiency, leukopenia, lichen planus, lupus (including nephritis, non-renal, discoid, alopecia), lymphoid interstitial pneumonitis (HIV), Ménière's disease, meningitis, mixed connective tissue disease, multiple organ injury syndrome, multiple sclerosis, myasthenia gravis, nonspecific interstitial pneumonia (NSIP), pancytopenia, pemphigoid (e.g., bullous pemphigoid and cicatricial pemphigoid), pemphigus (e.g., vulgaris, foliaceus, and paraneoplastic pemphigus), polychrondritis, polymyalgia rheumatica, polymyositis and dermatomyositis, primary agammaglobulinemia, primary biliary cirrhosis, primary hypothyroidism, psoriasis, Rapidly Progressive Glomerulonephritis, Reiter's disease, respiratory distress syndrome including adult respiratory distress syndrome (ARDS), responses associated with inflammatory bowel disease (IBD) (e.g., Crohn's disease, ulcerative colitis), Reynaud's phenomenon, sarcoidosis, Sjögren's syndrome, solid organ transplant rejection (including pretreatment for high panel reactive antibody titers, IgA deposit in tissues, etc), Stevens-Johnson syndrome, stiff-man syndrome, systemic lupus erythematosus (SLE), scleroderma including systemic scleroderma, CREST syndrome and sclerosis, thrombotic throbocytopenic purpura (TTP), toxic epidermal necrolysis, tuberculosis, uveitis, vasculitides such as dermatitis herpetiformis vasculitis, ANCA-associated vasculitides (AAV), large vessel vasculitis (including polymyalgia rheumatica, Giant Cell arteritis, and Takayasu's arteritis), medium vessel vasculitis (including Kawasaki Disease, Wegener's granulomatosis, and polyarteritis nodosa), and small vessel vasculitis (including Churg-Strauss arteritis, microscopic polyarteritis/polyangiitis, hypersensitivity/allergic vasculitis, Henoch-Schonlein purpura, and essential cryoglobulinemic vasculitis), and vitiligo. In a preferred embodiment, the autoimmune disorder is selected from the group consisting of Crohn's disease, multiple sclerosis, psoriasis, rheumatoid arthritis, systemic lupus erythematosus, type 1 diabetes, and vasculitis.

Non-limiting examples of inflammatory disorders treatable by the methods herein include immune-mediated inflammatory disorders (IMIDs), which are inflammatory conditions caused and sustained by an antigen-specific, pathological immune response. Among these disorders are various types of allergic diseases, such as asthma, hay fever, and urticaria, arthritis, such as osteoarthritis and rheumatoid arthritis, chronic inflammation, chronic obstructive pulmonary disease (COPD), connective tissue disorders, eczema and atopic dermatitis, fibrosis, graft rejection and graft-versus host-disease, inflammatory bowel disease (e.g., Crohn's disease and ulcerative colitis), inflammatory osteolysis, insulin-dependent diabetes, pulmonary fibrosis, retinitis, undifferentiated arthropathy, undifferentitated spondyloarthropathy, and uveitis. Molecules of the invention comprising at least one epitope binding domain specific for FcγRIIB and/or a variant Fc domain with an enhanced affinity for FcγRIIB and a decreased affinity for FcγRIIIA can also be used to prevent the rejection of transplants. In a preferred embodiment, the IMID is selected from the group consisting of of asthma, eczema and atopic dermatitis, fibrosis, graft rejection, graft-versus-host-disease, and inflammatory bowel disease.

The anti-inflammatory polypeptides of the present invention will preferably reduce inflammation in an animal by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the inflammation in an animal that does not receive such polypeptides.

In certain embodiments, the polypeptides of the invention are toxic to an infectious agent, enhance immune response against said agent or enhance effector function against said agent, relative to the immune response in the absence of said molecule. Infectious diseases that can be treated or prevented by the molecules of the invention are caused by infectious agents including but not limited to bacteria, fungi, protozoans, and viruses.

Non-limiting exemplary bacterial diseases include those caused by Bacillus anthracis (anthrax), Borrelia burgdorferi (Lyme disease), Candida, chlamydia, cholera, diptheria, E. coli, Enterococcus faecals, Heliobacter pylori, Kleb-

siella pneumonia, Streptococcal pneumonia, Klebsiella pneumonia; Hemophilus influenza; Mycoplasma pneumonia; Legionella pneumonia; Chlamydia pneumonia; Pneumocystis carinii pneumonia, legionella, mycobacterium, mycoplasma, Neisseria, Clostridium difficile (pertussis), Pasteruralla pestis (plague), Proteus vulgaris, Pseudomonas aeruginosa, S. pneumonia, Salmonella, staphylococcus, streptococcus, and Clostridium tetani (tetanus). In a preferred embodiment, the bacterial disease is selected from the group consisting of anthrax, bacterial meningitis, cholera, infection, Lyme disease, plague, pneumonia, streptococcus infection, tetanus, tuberculosis, and tularemia.

Non-limiting examples of viral diseases include those caused by adenovirus, arbovirus, coronavirus, coxsackie virus, cytomegalovirus, ebola, echinovirus, echovirus, endotoxin (LPS), enterovirus, Epstein Barr virus, hepatitis virus (e.g., hepatitis type A, hepatitis type B, hepatitis type C, murine hepatitis), herpes virus (e.g., herpes simplex type I (HSV-I), herpes simplex type II (HSV-II), murine gamma herpes virus), human immunodeficiency virus type I (HIV-I), human immunodeficiency virus type II (HIV-II), huntavirus, influenza, leukemia virus (e.g., murine leukemia, feline leukemia, etc.); measles virus, mumps virus, papilloma virus, papova virus, polio virus, respiratory syncytial virus, retrovirus, rhinovirus, rinderpest, rotavirus, rubella virus, small pox, T-cell lymphotropic virus 1, vaccinia, varicella, and agents of viral diseases such as viral meningitis, encephalitis, or dengue. In a preferred embodiment, the viral disease is selected from the group consisting of dengue fever, encephalitis, hemorrhagic fever, hepatitis, herpes, human papillomavirus, influenza, polio, rabies, smallpox, viral meningitis, West Nile fever, and yellow fever.

Non-limiting examples of protozoal diseases include those caused by amoebae, helminths, and other parasites, such as Acanthamoeba, Babesia, Balantidium, Cryptosporidium, Cyclospora, Entamoeba, Giardia, Leishmania, Microsporidia, Naegleria, Plasmodium, Toxoplasma, Trichomonas, and Trypanosoma. In a preferred embodiment, the protozoal disease is malaria. Non-limiting examples of fungal diseases include aspergillosis, Candida infection, cryptococcosis, fungal meningitis, fungal pneumonia, histoplasmosis, mucormycosis, Pneumocystis infection, sporotrichosis, and Valley fever.

## C2. Formulations

The pharmaceutical compositions can be formulated according to known methods for preparing pharmaceutically useful compositions, and may include a pharmaceutically acceptable carrier and/or an excipient. The compositions can be in any suitable form, for example tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders, to name just a few non-limiting alternatives. Such compositions may be prepared by any known method, for example by admixing the active ingredient with the carrier(s) or excipient(s) under sterile conditions.

The active ingredients can also be formulated so as to provide quick, sustained or delayed release of the active ingredient after administration to the patient by employing procedures known in the art. The physical and chemical characteristics of the compositions of the invention may be modified or optimized according to the skill in the art, depending on the mode of administration and the particular disease or disorder to be treated. The compositions may be provided in unit dosage form, a sealed container, or as part of a kit, which may include instructions for use and/or a plurality of unit dosage forms.

In particular embodiments, the therapeutic agents can be incorporated into a composition, by, e.g., encapsulation in liposomes, microparticles, microcapsules, recombinant cells capable of expressing the antibody or fusion protein, receptor-mediated endocytosis (See, e.g., Wu and Wu (1987) J. Biol. Chem. 262:4429-4432), construction of a nucleic acid as part of a retroviral or other vector, etc. In another particular embodiment, the therapeutic agents are supplied as a dry sterilized lyophilized powder or water free concentrate in a hermetically sealed container and can be reconstituted, e.g., with water or saline to the appropriate concentration for administration to a subject.

Preferably, the therapeutic agent is supplied as a dry sterile lyophilized powder in a hermetically sealed container at a unit dosage of at least 5 mg, more preferably at least 10 mg, at least 15 mg, at least 25 mg, at least 35 mg, at least 45 mg, at least 50 mg, or at least 75 mg. The lyophilized powder should be stored at between 2 and 8°C in its original container and the molecules should be parenterally administered within 12 hours, preferably within 6 hours, within 5 hours, within 3 hours, or within 1 hour after being reconstituted. In an alternative embodiment, the therapeutic agents are supplied in liquid form in a hermetically sealed container indicating the quantity and concentration of the therapeutic agent. Preferably, the liquid form is supplied in a hermetically sealed container at least 1 mg/ml, more preferably at least 2.5 mg/ml, at least 5 mg/ml, at least 8 mg/ml, at least 10 mg/ml, at least 15 mg/kg, at least 25 mg/ml, at least 50 mg/ml, at least 100 mg/ml, at least 150 mg/ml, at least 200 mg/ml of the molecules.

## C3. Kits

The compositions may also be included in a kit. The kit can include, in non-limiting aspects, a pharmaceutical composition comprising a therapeutic agent, instructions for administration and/or other components. In preferred embodiments, the kit can include a composition ready for administration. Containers of the kits can include a bottle, dispenser, package, compartment, or other types of containers, into which a component may be placed. The container can include indicia on its surface. The indicia, for example, can be a word, a phrase, an abbreviation, a picture, or a symbol. The containers can dispense a pre-determined amount of the component (*e.g.* compositions of the present invention). The composition can be dispensed in a spray, an aerosol, or in a liquid form or semi-solid form. The containers can have spray, pump, or squeeze mechanisms. In certain aspects, the kit can include a syringe for administering the compositions of the present invention.

Where there is more than one component in the kit (they may be packaged together), the kit also will generally contain a second, third or other additional containers into which the additional components may be separately placed. The kits also can include a container housing the components in close confinement for commercial sale. Such containers may include injection or blow-molded plastic containers into which the desired bottles, dispensers, or packages are retained. A kit can also include instructions for employing the kit compoinents as well the use of any other compositions, compounds, agents, active ingredients, or objects not included in the kit. Instructions may include variations that can be implemented. The instructions can include an explanation of how to apply, use, and maintain the products or compositions, for example.

## C4. Administration and Dosage

A variety of administration routes for the compositions of the present invention are available. The particular mode selected will depend, of course, upon the particular therapeutic agent selected, whether the administration is for prevention, diagnosis, or treatment of disease, the severity of the medical disorder being treated and dosage required for therapeutic efficacy. The methods of this invention may be practiced using any mode of administration that is medically acceptable, and produces effective levels of the active compounds without causing clinically unacceptable adverse effects. Such modes of administration include, but are not limited to, oral, buccal, sublingual, inhalation, mucosal, rectal, intranasal, topical, ocular, periocular, intraocular, transdermal, subcutaneous, intra-arterial, intravenous, intramuscular, parenteral, or infusion methodologies. In a specific embodiment, it may be desirable to administer the pharmaceutical compositions of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers.

As used herein, the term "therapeutically effective amount" means the total amount of each active component of the pharmaceutical composition or method that is sufficient to show a meaningful patient benefit, *i.e.,* healing or amelioration of chronic conditions, a reduction in symptoms, an increase in rate of healing of such conditions, or a detectable change in the levels of a substance in the treated or surrounding tissue. When applied to an individual active ingredient, administered alone, the term refers to that ingredient alone. When applied to a combination, the term refers to combined amounts of the active ingredients that result in the therapeutic effect, whether administered in combination, serially, or simultaneously.

The dosage schedule and amounts effective for therapeutic and prophylactic uses, i.e., the "dosing regimen", will depend upon a variety of factors, including the stage of the disease or condition, the severity of the disease or condition, the general state of the patient's health, the patient's physical status, age and the like. Therapeutic efficacy and toxicity of the compositions may be determined by standard pharmaceutical, pharmacological, and toxicological procedures in cell cultures or experimental animals. For example, numerous methods of determining $ED_{50}$ (the dose therapeutically effective in 50 percent of the population) and $LD_{50}$ (the dose lethal of 50 percent of the population) exist. The dose ratio between therapeutic and toxic effects is the therapeutic index, and it can be expressed as the ratio $ED_{50}/LD_{50}$. Compositions exhibiting high therapeutic indices are preferred. The data obtained from cell culture assays or animal studies may be used in formulating a range of dosages for human use. The dosage is preferably within a range of concentrations that includes the $ED_{50}$ with little or no toxicity, and may vary within this range depending on the dosage form employed, sensitivity of the patient, and the route of administration.

The dosage regimen also takes into consideration pharmacokinetics parameters well known in the art, i.e., the rate of absorption, bioavailability, metabolism, clearance, and the like (see, e.g., Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84:1144-1146; Rohatagi (1995) Pharmazie 50:610-613; Brophy (1983) Eur. J. Clin. Pharmacol. 24:103-108; the latest Remington, supra). The state of the art allows the clinician to determine the dosage regimen for each individual patient, therapeutic agent and disease or condition treated. Single or multiple administrations of the compositions of the present invention can be administered depending on the dosage and frequency as required and tolerated by the patient. The duration of prophylactic and therapeutic treatment will vary depending on the particular disease or condition being treated. Some diseases lend themselves to acute treatment whereas others require long-term therapy. If administration is not on a daily basis, for example if injections are given every few days, every few weeks, or every few months, then more therapeutic agent may be included in each administration, so that daily release of the agent is adequate to meet therapeutic needs.

The therapeutic agents of the invention may be administered in metronomic dosing regimens, either by continuous infusion or frequent administration without extended rest periods. Such metronomic administration can involve dosing at constant intervals without rest periods. Typically the therapeutic agents, in particular cytotoxic agents, are used at lower doses. Such dosing regimens encompass the chronic daily administration of relatively low doses for extended periods of time, which can minimize toxic side effects and eliminate rest periods. Kamat et al. (2007) Cancer Research 67:281-88. In certain embodiments, the therapeutic agents are delivered by chronic low-dose or continuous infusion ranging from about 24 hours to about 2 days, to about 1 week, to about 2 weeks, to about 3 weeks to about 1 month to about 2 months, to about 3 months, to about 4 months, to about 5 months, to about 6 months. The scheduling of such dose regimens can be optimized by the skilled oncologist.

For antibodies encompassed by the invention, the dosage administered to a patient is typically 0.0001 mg/kg to 100 mg/kg of the patient's body weight. Preferably, the dosage administered to a patient is between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. The dosage and frequency of administration may be reduced or altered by enhancing uptake and tissue penetration of the antibodies by modifications such as, for example, lipidation. In one embodiment, the dosage of the antibodies administered to a patient is 0.01 mg to 1000 mg/day, when used as single agent therapy. In another embodiment the antibodies are used in combination with other therapeutic compositions and the dosage intended to be administered to a patient are lower than when said molecules are used as a single agent therapy. In a preferred example, a subject is treated with antibodies in the range of between about 0.1 to 30 mg/kg body weight, one time per week for between about 1 to 10 weeks, preferably between 2 to 8 weeks, more preferably between about 3 to 7 weeks, and even more preferably for about 4, 5, or 6 weeks.

## C5. Combination Therapies

Also described is administering the antibodies or polypeptides of the invention in combination with other therapies known to those skilled in the art for the treatment or prevention of cancer, autoimmune disease, inflammation, or infectious disease, including but not limited to, current standard and experimental chemotherapies, hormonal therapies, biological therapies, immunotherapies, radiation therapies, or surgery. In some embodiments, the antibodies or polypeptides of the invention may be administered in combination with a therapeutically or prophylactically effective amount of one or more therapeutic agents known to those skilled in the art for the treatment and/or prevention of cancer, autoimmune disease, infectious disease or intoxication.

As used herein, the term "combination" refers to the use of more than one therapeutic agent. The use of the term "combination" does not restrict the order in which therapeutic agents are administered to a subject with a disorder, nor does it mean that the agents are administered at exactly the same time, but rather it is meant that an antibody or polypeptide of the invention and the other agent are administered to a mammal in a sequence and within a time interval such that the antibody or polypeptide of the invention can act together with the other agent to provide an increased benefit than if they were administered otherwise. For example, each therapeutic agent (e.g., chemotherapy, radiation therapy, hormonal therapy or biological therapy) may be administered at the same time or sequentially in any order at different points in time; however, if not administered at the same time, they should be administered sufficiently close in time so as to provide the desired therapeutic or prophylactic effect. Each therapeutic agent can be administered separately, in any appropriate form and by any suitable route, e.g., one by the oral route and one parenterally.

In various embodiments, a first therapeutic agent can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second (or subsequent) therapeutic agent to a subject with a disorder. In preferred embodiments, two or more agents are administered within the same patient visit, or no more than 12 hours apart, no more than 24 hours apart, or no more than 48 hours apart.

In certain embodiments, the therapeutic agents can be cyclically administered to a subject. Cycling therapy involves the administration of a first agent for a period of time, followed by the administration of a second agent and/or third agent for a period of time and repeating this sequential administration. Cycling therapy can reduce the development of resistance to one or more of the therapies, avoid or reduce the side effects of one of the therapies, and/or improves the efficacy of the treatment. Exemplary cycles are about once every week, about once every 10 days, about once every two weeks, and about once every three weeks. Each cycle can comprise at least 1 week of rest, at least 2 weeks of rest, at least 3 weeks of rest. The number of cycles to be administered is from about 1 to about 12 cycles, more typically from about 2 to about 10 cycles, and more typically from about 2 to about 8 cycles.

In an embodiment for the treatment of a cell proliferative disorder, an antibody or polypeptide of the present invention can be conjugated to, or administered in combination with, another therapeutic agent, such as, but not limited to, an alkylating agent (e.g., mechlorethamine or cisplatin), angiogenesis inhibitor, anthracycline (e.g., daunorubicin/daunomycin or doxorubicin), antibiotic (e.g., dactinomycin, bleomycin, or anthramycin), antibody (e.g., an anti-VEGF antibody such as bevacizumab (sold as AVASTIN® by Genentech, Inc.), an anti-EGFR antibody such as panitumumab (sold as VECTIBIX™ by Amgen, Inc.), or an anti-integrin antibody such as natalizumab (sold as TYSABRI® by Biogen Idec and Elan Pharmaceuticals, Inc.)), an antimetabolite (e.g., methotrexate or 5-fluorouracil), an anti-mitotic agent (e.g., vincristine or paclitaxel), a cytotoxin (e.g., a cytostatic or cytocidal agent), a hormone therapy agent (e.g., a selective estrogen receptor modulator (e.g., tamoxifen or raloxifene), aromatase inhibitor, luteinizing hormone-releasing hormone analogue, progestational agent, adrenocorticosteroid, estrogen, androgen, anti-estrogen agent, androgen receptor blocking agent, 5-alpha reductase inhibitor, adrenal production inhibitor, etc.), a matrix metalloprotease inhibitor, a radioactive element (e.g., alpha-emitters, gamma-emitters, etc.), or any other chemotherapeutic agent.

Non-limiting examples of suitable angiogenesis inhibitors include ABT-627; angiostatin (plasminogen fragment); angiozyme; antiangiogenic antithrombin III; Bay 12-9566; benefin; bevacizumab; BMS-275291; bisphosphonates; cartilage-derived inhibitor (CDI); CAI; CD59 complement fragment; CEP-7055; Col 3; combretastatin A-4; endostatin (collagen XVIII fragment); farnesyl transferase inhibitors (FTI); fibronectin fragment; gro-beta; halofuginone; heparinases; heparin hexasaccharide fragment; HMV833; human chorionic gonadotropin (hCG); IM-862; interferon alpha/beta/gamma; interferon inducible protein (IP-10); interleukin-12; kringle 5 (plasminogen fragment); marimastat; metalloproteinase inhibitors (TIMPs); 2-methoxyestradiol; MMI 270 (CGS 27023A); MoAb IMC-1C11; neovastat; NM-3; panzem; PI-88; placental ribonuclease inhibitor; plasminogen activator inhibitor; platelet factor-4 (PF4); prinomastat; prolactin 16kDa fragment; proliferin-related protein (PRP); PTK 787/ZK 222594; retinoids; solimastat; squalamine; SS 3304; SU 5416; SU6668; SU11248; tetrahydrocortisol-S; tetrathiomolybdate; thalidomide; thrombospondin-1 (TSP-1); TNP-470; transforming growth factor-beta (TGF-b); vasculostatin; vasostatin (calreticulin fragment); ZD6126; and ZD 6474.

Non-limiting examples of additional antibodies for the treatment of a cell proliferative disorder include antibodies to 17-1A, $\alpha v \beta_3$, AFP, CD3, CD18, CD20, CD22, CD33, CD44, CD52, CEA, CTLA-4, DNA-associated proteins, EGF receptor, Ep-CAM, GD2-ganglioside, gp IIIb/IIIa, gp72, HER2, HLA-DR 10 beta, HLA-DR antigen, IgE, ganglioside GD3, MUC-1, nuC242, PEM antigen, SK-1 antigen, tumor antigen CA125, tumor antigen MUC1, VEGF, and VEGF-receptor.

An antibody or polypeptide of the present invention may be administered in combination with a therapeutic agent or agents for the treatment of an inflammatory disorder, such as, but not limited to, antibodies, anticholingeric agents, beta-agonists, methyl xanthines, non-steroidal anti-inflammatory drugs (NSAIDs) (e.g., aspirin, ibuprofen, celecoxib or diclofenac), and steroidal anti-inflammatory drugs (e.g., glucocorticoids, dexamethasone, cortisones, prednisone or eicosanoids). The additional antibodies may be any suitable antibody for the treatment of inflammatory disease, such as, but not limited to antibodies to alpha4beta7, beta2-integrin, CBL, CD2, CD3, CD4, CD11a, CD11/18, CD14, CD18, CD23, CD25, CD40L, CD64 (FcR), CD80, CD147, Complement (C5), E-selectin, Fact VII, gpIIbIIIa, ICAM-3, IgE, IL-4, IL-5, IL-8, TNF-alpha, and VLA-4.

An antibody or polypeptide of the present invention may be administered in combination with a therapeutic agent or agents for the treatment of an autoimmune disorder, such as, but not limited to, antibodies, brequinar, cyclophosphamide, cyclosporine A, cytokine receptor modulators, deoxyspergualin, leflunomide, macrolide antibiotics, malononitriloamindes (*e.g.,* leflunamide), methothrexate, methylprednisolone, mizoribine, mycophenolate mofetil, rapamycin (sirolimus), steroids, and T cell receptor modulators. The additional antibodies may be any suitable antibody for the treatment of an autoimmune disorder, and non-limiting examples include antibodies to a4b7 integrin receptor, CBL antigen, CD2, CD4, CD23, CD40, CD80, FcRI, Gamma Interferon, IL-8, inosine monophosphate dehydrogenase, ICE interleukin-1 beta, P38MAP kinase, and TNF.

An antibody or polypeptide of the present invention may be administered in combination with a therapeutic agent or agents for the treatment of an infectious disease, such as, but not limited to, an antibiotic, anti-fungal, or anti-viral agent. Antibiotics that can be used in combination with the molecules of the invention include, but are not limited to, 2,4 diaminopyrimidines (e.g., brodimoprim), aminoglycosides (e.g., apramycin, neomycin, or spectinomycin), amphenicols (e.g., chloramphenicol), amphomycins, ansamycins (e.g., rifamide and rifampin), bacitracins, carbacephems (e.g., loracarbef), carbapenems (e.g., biapenem and imipenem), cephalosporins (e.g., cephalexin or cefadroxil), cephamycins (e.g., cefbuperazone, cefmetazole, and cefminox), clarithromycins, erythromycins, lincosamides (e.g., clindamycin and lincomycin), macrolides (e.g., tobramycin), monobactams (e.g., carumonam), nitrofurans (e.g., furaltadone, and furazolium chloride), oxacephems (e.g., flomoxef and moxalactam), penicillins, quinolones (e.g., ofloxacin or ciprofloxacin), sulfonamides (e.g., benzylsulfamide, and sulfacytine), sulfones (e.g., diathymosulfone, glucosulfone sodium, and solasulfone), and tetracyclines (e.g., apicycline and chlortetracycline).

Antifungal agents that can be used in combination with the molecules of the invention include, but are not limited to, amphotericin B, butoconazole, ciclopirox, clotrimazole, econazole, fluconazole, flucytosine, griseofuldin, haloprogrin, intrathecal, itraconazole, ketoconazole, miconazole, naftifine, nystatin, terbinafine, terconazole, tioconazole, and undecylenate. Useful anti-viral agents that can be used in combination with the molecules of the invention include, but are not limited to, non-nucleoside reverse transcriptase inhibitors, nucleoside analogs, nucleoside reverse transcriptase inhibitors, and protease inhibitors. Non-limiting examples of such agents are acyclovir, adefovir, alpha interferons, amantadine, amprenavir, clevadine, entecavir, foscarnet, gangcyclovir, idoxuridine, indinavir, lopinavir, pleconaril, ribavirin, rimantadine, ritonavir, saquinavir, trifluridine, vidarabine, and zidovudine.

## C6. Demonstration of Therapeutic Utility

The pharmaceutical compositions, prophylactic, or therapeutic agents described herein are preferably tested in vitro, in a cell culture system, and in an animal model organism, such as a rodent animal model system, for the desired therapeutic activity prior to use in humans. For example, assays which can be used to determine whether administration of a specific pharmaceutical composition is desired, include cell culture assays in which a patient tissue sample is grown in culture, and exposed to or otherwise contacted with a pharmaceutical composition of the invention, and the effect of such composition upon the tissue sample is observed. The tissue sample can be obtained by biopsy from the patient. This test allows the identification of the therapeutically most effective prophylactic or therapeutic molecule(s) for each individual patient. In various specific embodiments, in vitro assays can be carried out with representative cells of cell types involved in an autoimmune or inflammatory disorder (e.g., T cells), to determine if a pharmaceutical composition of the invention has a desired effect upon such cell types.

Suitable animal model systems include, but are not limited to, rats, mice, chicken, cows, monkeys, pigs, dogs, rabbits, etc. Any animal system well-known in the art may be used. Combinations of prophylactic and/or therapeutic agents can be tested in a mouse model system. Preferred animal models for use are, for example, transgenic mice expressing human $Fc\gamma Rs$ on mouse effector cells, e.g., any mouse model described in U.S. 5,877,396 can be used.

Anti inflammatory activity can be determined by using various experimental and spontaneous animal models of inflammatory arthritis known in the art and described in Crofford L.J. and Wilder R.L., "Arthritis and Autoimmunity in Animals", in Arthritis and Allied Conditions: A Textbook of Rheumatology, McCarty et al.(eds.), Chapter 30 (Lee and Febiger, 1993). For example, adjuvant-induced arthritis models such as carrageenan-, xymosan-, or collagen-induced arthritis in rats, hamsters, rabbits, dogs and pigs, are useful in studying anti-inflammatory activity, and inhibition of carrageenan-induced paw edema in rats is a primary in vivo screen for the anti inflammatory activity of most NSAIDs, and is considered predictive of human efficacy. These models are described in, e.g., Winter et al. (1962) Proc. Soc. Exp. Biol Med. 111:544-47; and Hansra et al. (2000) Inflammation 24(2):141-155. Animal models for inflammatory bowel disease can also be used to assess the efficacy of therapies of the invention, for example the models described in, e.g., Strober (1985) Dig. Dis. Sci. 30(12 Suppl):3S-10S; Kim et al. (1992) Scand. J. Gastroentrol. 27:529-537). In these models, ulcerative cholitis and Crohn's disease can be induced in animals by oral administration of sulfated polysaccharides, dextran sulfate or chemical irritants.

Efficacy in treating autoimmune disorders may be assessed using animal models for autoimmune disorders such as type 1 diabetes, thyroid autoimmunity, systemic lupus eruthematosus, and glomerulonephritis, for example the models described in Flanders et al. (1999) Autoimmunity 29:235-246; Krogh et al. (1999) Biochimie 81:511-515; Foster (1999) Semin. Nephrol. 19:12-24, etc.

The anti-cancer activity of the therapeutic agents also can be determined by using various experimental animal models for the study of cancer such as the SCID mouse model, transgenic mice or nude mice with human xenografts, and other animal models such as hamsters, rabbits, etc. known in the art and described in Relevance of Tumor Models for Anticancer Drug Development (1999, eds. Fiebig and Burger); Contributions to Oncology (1999, Karger); The Nude Mouse in Oncology Research (1991, eds. Boven and Winograd); and Anticancer Drug Development Guide (1997 ed. Teicher). Preferred animal models are mouse xenograft models. Tumor cell lines that can be used as a source for xenograft tumors include but are not limited to, SKBR3 and MCF7 cells, which can be derived from patients with breast adenocarcinoma. These cells have both erbB2 and prolactin receptors. SKBR3 cells have been used routinely in the art as ADCC and xenograft tumor models. Alternatively, OVCAR3 cells derived from a human ovarian adenocarcinoma can be used as a source for xenograft tumors.

The therapeutic agents of the invention are preferably tested in vitro, and then in vivo, for the desired therapeutic or prophylactic activity, prior to use in humans. Therapeutic agents and methods may be screened using cells of a tumor or malignant cell line. Many assays standard in the art can be used to assess such survival and/or growth; for example, cell proliferation can be assayed by measuring $^3$H-thymidine incorporation, by direct cell count, by detecting changes in transcriptional activity of known genes such as proto-oncogenes (e.g., fos, myc) or cell cycle markers; cell viability can be assessed by trypan blue staining, differentiation can be assessed visually based on changes in morphology, decreased growth and/or colony formation in soft agar or tubular network formation in three-dimensional basement membrane or extracellular matrix preparation, etc.

The data obtained from the cell culture assays and animal studies can be used in formulating a range of dosage of the therapeutic agents for use in humans. The dosage of such agents lies preferably within a range of circulating concentrations that include the $ED_{50}$ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the $IC_{50}$ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

## D. OTHER METHODS

### D1. Gene Therapy

Nucleic acids comprising sequences encoding molecules of the invention, can be administered to treat, prevent or ameliorate one or more symptoms associated with a disease, disorder, or infection, by way of gene therapy. Gene therapy refers to therapy performed by the administration to a subject of an expressed or expressible nucleic acid. The nucleic acids produce their encoded antibody or fusion protein that mediates a therapeutic or prophylactic effect. Any methods for gene therapy available in the art may be used, for example the methods described in, e.g., Goldspiel et al. (1993) Clinical Pharmacy 12:488-505; Wu and Wu (1991) Biotherapy 3:87-95; Tolstoshev (1993) Ann. Rev. Pharmacol. Toxicol. 32:573-596; Mulligan (1993) Science 260:926-932; and Morgan and Anderson (1993) Ann. Rev. Biochem. 62:191-217.

In a preferred aspect, a composition of the invention comprises nucleic acids encoding an antibody, diabody, or fusion protein of the invention, said nucleic acids being part of an expression vector that expresses the antibody in a suitable host. In particular, such nucleic acids have promoters, preferably heterologous promoters, operably linked to the antibody coding region, said promoter being inducible or constitutive, and, optionally, tissue-specific. In another particular embodiment, nucleic acid molecules are used in which the antibody coding sequences and any other desired sequences are flanked by regions that promote homologous recombination at a desired site in the genome, thus providing for intrachromosomal expression of the antibody encoding nucleic acids, as described in Koller and Smithies (1989) Proc. Natl. Acad. Sci. (U.S.A.) 86:8932-35; and Zijlstra et al. (1989) Nature 342:435-438.

Delivery of the nucleic acids into a subject may be either direct, in which case the subject is directly exposed to the nucleic acid or nucleic acid-carrying vectors, or indirect, in which case, cells are first transformed with the nucleic acids in vitro, then transplanted into the subject. These two approaches are known, respectively, as in vivo or ex vivo gene therapy.

A polynucleotide encoding a polypeptide of the present invention can be administered in vivo, where it is expressed to produce the encoded polypeptide. This can be accomplished by any of numerous methods, such as by infection using retroviral or other viral vectors (as described in, e.g., U.S. Patent No. 4,980,286; Miller et al. (1993) Meth. Enzymol. 217:581-599; Salmons and Gunzberg (1993) Human Gene Therapy 4:129-141; Grossman and Wilson (1993) Curr. Opin. in Genetics and Devel. 3:110-114; Kozarsky and Wilson (1993) Current Op. in Genetics and Dev. 3:499-503; Walsh et al. (1993) Proc. Soc. Exp. Biol. Med. 204:289-300; Bout et al. (1994) Human Gene Therapy 5:3-10; Boesen et al. (1994) Biotherapy 6:291-302; Clowes et al. (1994) J. Clin. Invest. 93:644-651; Klein et al. (1994) Blood 83:1467-1473; and U.S. Patent No. 5,436,146), by direct injection of naked DNA, by use of microparticle bombardment (e.g., a gene gun), or coating with lipids or cell-surface receptors or transfecting agents, encapsulation in liposomes, microparticles, or microcapsules, or by administering them in linkage to a peptide which is known to enter the nucleus or in linkage to an antigen subject to receptor-mediated endocytosis (as described in, e.g., Wu and Wu (1987) J. Biol. Chem. 262:4429-4432; Joliot et al. (1991) Proc. Natl. Acad. Sci. (U.S.A.) 88:1864-1868; WO 92/06180; WO 92/22635; WO92/20316; WO93/14188; WO 93/20221) (which can be used to target cell types specifically expressing the receptors).

A nucleic acid may be introduced into a cell prior to administration in vivo of the resulting recombinant cell, for example as described in WO 94/08598; Rheinwald (1980) Meth. Cell Bio. 21A:229; Pittelkow and Scott (1986) Mayo Clinic Proc. 61:771; Stemple and Anderson (1992) Cell 7 1:973-985. The resulting recombinant cells can be delivered to a subject by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art. Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc. In a preferred embodiment, the cell used for gene therapy is autologous to the subject.

**D2. Vaccine Therapy**

The antibodies of the invention may be used to induce an immune response against an antigenic or immunogenic agent, including but not limited to cancer antigens and infectious disease antigens. The vaccine compositions comprise one or more antigenic or immunogenic agents to which an immune response is desired, wherein the one or more antigenic or immunogenic agents is coated with an antibody of the invention. The vaccine compositions are particularly effective in eliciting an immune response, preferably a protective immune response against the antigenic or immunogenic agent, which may be a virus against which an immune response is desired, or an antigen derived from other viral or non-viral pathogens.

Also described are pathogenic cells or viruses, preferably attenuated viruses, which express the antibody on their surface. Also described are methods to induce tolerance in a subject by administering a composition of the invention. Preferably a composition suitable for inducing tolerance in a subject, comprises an antigenic or immunogenic agent coated with an antibody of the invention.

**D3. Targeting Liposomes or Other Microcarriers and Nanocarriers**

In some embodiments, the antibodies of the invention can be used to prepare targeted liposomes for delivery of a desired therapeutic composition (e.g., anti-cancer agents) to a target cell. The preparation and use of immunoliposomes for targeted delivery of antitumor drugs is reviewed in Mastrobattista et al. (1999) Advanced Drug Delivery Reviews 40:103-127. Liposomes are vesicular structures based on lipid bilayers. They can be as small as 20 nm and as large as 10 $\mu$m in diameter. They can be unilamellar (only one bilayer surrounds an aqueous core) or multilamellar (two or more bilayers concentrically oriented around an aqueous core). Targeting of liposomes using a variety of targeting agents (e.g., antibodies of the invention) is well known in the art. See, e.g., U.S. Patent Nos. 4,957,773 and 4,603,044. Standard methods for coupling targeting agents to liposomes can be used. Antibody targeted liposomes can be constructed using, for instance, liposomes which incorporate protein A. (Renneisen et al. (1990) J. Biol. Chem. 265:16337-16342; and Leonetti et al. (1990) Proc. Natl. Acad. Sci. (U.S.A.) 87:2448-2451).

In a preferred embodiment, the liposomes are formed from standard vesicle-forming lipids, which generally include neutral and negatively charged phospholipids and a sterol, such as cholesterol. The selection of lipids is generally guided by consideration of, e.g., liposome size and stability of the liposomes in the bloodstream. A variety of methods are available for preparing liposomes, as described in, e.g., Szoka, et al. (1980) Ann. Rev. Biophys. Bioeng. 9:467; U.S. Patent Nos. 4,235,871; 4,501,728; and 4,837,028. One method produces multilamellar vesicles of heterogeneous sizes. In this method, the vesicle forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous solution of the targeted drug and the targeting component (antibody) and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

**D4. Immunoassays**

The antibodies of the invention can be used to detect the BCR complex, BCR, CD79a, CD79b, or cells expressing such molecules. Any of a number of methods may be used to achieve such detection. For example, immunological binding assays may be used (see, e.g., U.S. Patent Nos. 4,366,241; 4,376,110; 4,517,288; and 4,837,168). For a review of the general immunoassays, see also Asai (ed. 1993) Methods in Cell Biology Vol. 37, Academic Press, New York; Stites & Terr (eds. 1991) Basic and Clinical Immunology 7th Ed.

Thus, also described are methods of detecting cells that express BCR and associated proteins. In one method, a biopsy is performed on the subject and the collected tissue is tested in vitro. The tissue or cells from the tissue is then contacted, with an antibody of the invention. Any immune complexes which result indicate the presence of a target protein in the biopsied sample. To facilitate such detection, the antibody can be radiolabeled or coupled to an effector molecule which is a detectable label, such as a radiolabel. In another method, the cells can be detected in vivo using typical imaging systems. Then, the localization of the label is determined by any of the known methods for detecting the label. A conventional method for visualizing diagnostic imaging can be used. For example, paramagnetic isotopes can be used for MRI. Internalization of the antibody may be important to extend the life within the organism beyond that provided by extracellular binding, which will be susceptible to clearance by the extracellular enzymatic environment coupled with circulatory clearance. BCR proteins can also be detected using immunoassay methods and the antibodies of the invention. Standard methods include, for example, radioimmunoassay, immunochromatographic methods, sandwich immunoassays (including ELISA), immunofluorescence assays, Western blot, affinity chromatography (affinity ligand bound to a solid phase), and in situ detection with labeled antibodies.

Application of the teachings of the present invention to a specific problem or environment is within the capabilities of one having ordinary skill in the art in light of the teachings contained herein. Having now generally described the invention, the same will be more readily understood through reference to the following examples, which are provided by way of illustration and are not intended to be limiting of the present invention unless specified.

## Example 1

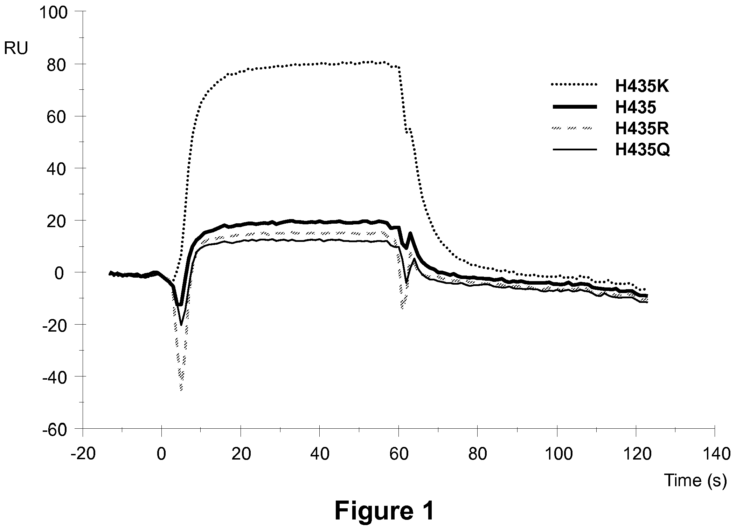

Surface plasmon resonance analysis on a Biocore 3000 instrument was performed to analyze the effect of different amino acid substitutions at the position 435 of ch4420 on the binding to hFcRn. Changes in real time binding responses to hFcRn were analyzed comparatively to wild type Fc of ch4420 antibody which was captured on protein-FITC surface at the level about 500 RU. Three variants (H435K, H435R, and H435Q) and wild-type ch4420 antibodies were analyzed. Injection of supernatants containing ch4420 variants was followed by injection of hFcRn at concentration 500 nM in Na acetate buffer containing 150 mM NaCl and 0.005% P-20, pH 6.0 at flow rate 10 μl/min. The protein-FITC surface was regenerated by 10 mM Glycine pH 1.5 in between two different mutants. Results, shown in Figure 1, indicate that mutant Fcs with substitutions of His to R and Q retained close to wild-type binding to hFcRn, but that the substitution of His to K significantly increased binding response (up to 4-fold).

## Example 2

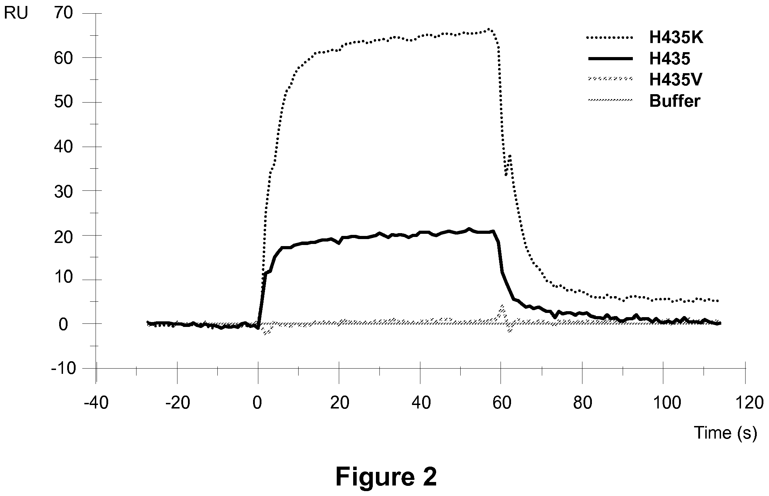

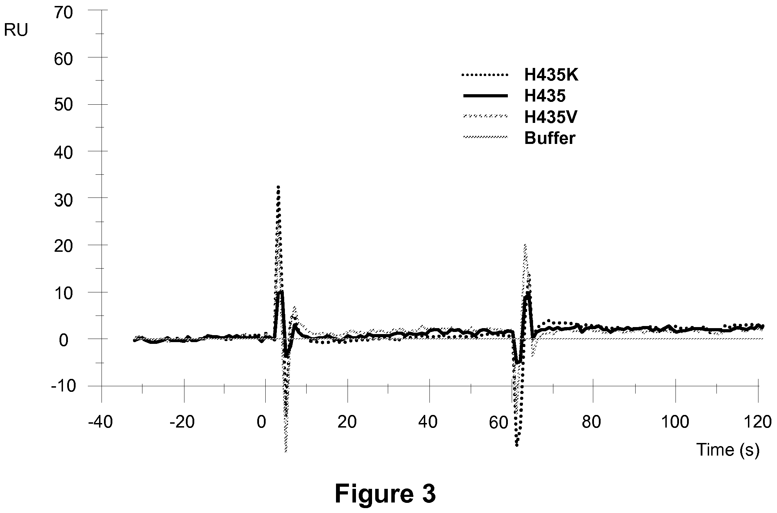

Surface plasmon resonance analysis on a Biocore 3000 instrument was performed to analyze the effect of different amino acid substitutions at the position 435 of ch4420 on the pH dependency of hFcRn binding to mutant ch4420 captured on protein-FITC surface. pH dependency of binding to hFcRn was demonstrated in two separate experiments performed at similar conditions in sodium acetate buffer (pH 6.0) and HEPES buffer (pH 7.4). Injection of supernatants containing ch4420 variants was followed by injection of hFcRn at concentration 500 nM in 20 mM Na acetate buffer containing 150 mM NaCl and 0.005% P-20 pH 6.0 or 20 mM HEPES buffer containing 150 mM NaCl and 0.005% P-20 pH 7.4 at flow rate 10 μl/min. The protein-FITC surface was regenerated by 10 mM Glycine pH 1.5 between capturing of two different mutants. The buffer injection curve was subtracted as a blank. Results, shown in **Figure 2** and **Figure 3,** indicate that mutant Fc with substitution of His to K exhibited enhanced binding to FcRn at pH below 6.5 (pH was 6.0), and that binding to FcRn was completely abrogated at pH 7.4 to the level of non-binding mutants with substitution V. **Figure 2** depicts binding at pH 6.0, and **Figure 3** depicts binding at pH 7.4.

## Example 3

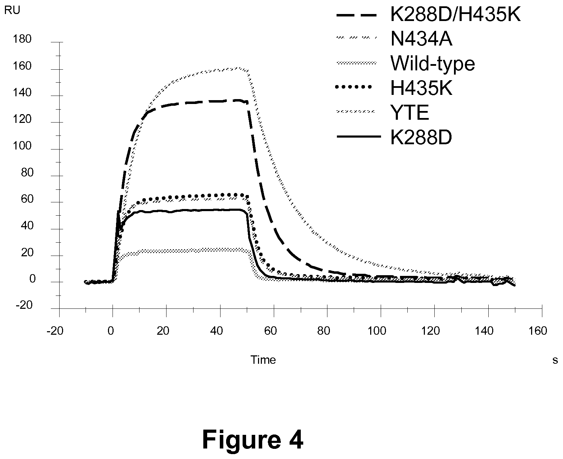

Surface plasmon resonance analysis on a Biocore 3000 instrument was performed to analyze the effect of the H435K amino acid substitution in ch4420 on the binding to soluble human FcRn (shFcRn). Wild-type Fc and mutant Fc ch4420 antibodies were captured on the surface with immobilized mIgG1-Flrscn, and shFcRn was injected at concentration of 400 nM. Four variants (K288D, N434A, H435K, K288D and H435K), a YTE mutant (triple substitution of M252Y, S254T, and T256E; Dall'Acqua, W.F. et al. (2002) "Increasing The Affinity Of A Human Igg1 For The Neonatal Fc Receptor: Biological Consequences," J. Immunol. 169(9):5171-5180; Petkova, S.B. et al. (Epub 2006 Oct 31) "Enhanced Half-Life Of Genetically Engineered Human Igg1 Antibodies In A Humanized Fcrn Mouse Model: Potential Application In Humorally Mediated Autoimmune Disease," Int. Immunol. 18(12):1759-1769), and wild-type ch4420 antibodies were analyzed. **Figure 4** depicts an SPR analysis of binding of shFcRn (400nM) to mutant Fc ch4420 captured on the surface with immobilized mIgG1-Flrscn. Five variants (K288D/H435K; H435K; K288D; N434A and the YTE mutant (triple substitution of M252Y, S254T, and T256E), and wild-type ch4420 antibodies were analyzed. The results shown in **Figure 4** indicate that Fc mutants having the H435K substitution and the combination of K288D and H435K substitutions had significantly improved binding as compared to wild-type binding with the K288D/H435K and YTE variants having the best binding.

## Example 4

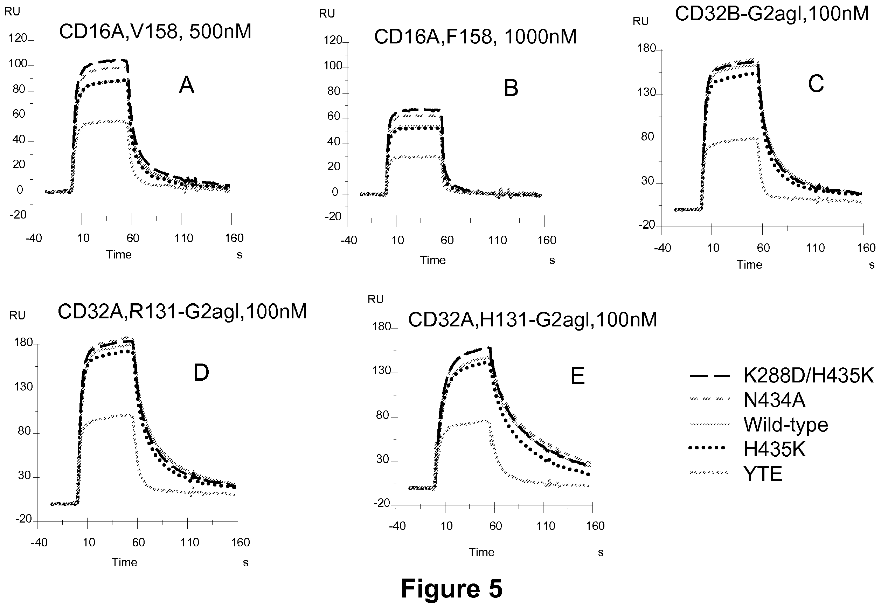

Surface plasmon resonance analysis on a Biocore 3000 instrument was performed to analyze the effect of different amino acid substitutions on the ability of ch4420 to bind different FcγRs (FcγRIIIA (CD16A); FcγRIIA (CD32A); FcγRIIB (CD32B)), captured on BSA-Flrscn surface (bovine serum albumin (BSA) was used as a reference. Four variants (K288D / H435K; N434A; H435K; and the YTE mutant (triple substitution of M252Y, S254T, and T256E), and wild-type ch4420 antibodies were analyzed. Mutant ch4420 antibodies were captured at the level 700-1000Ru on the surface with immobilized BSA-Flrscn. FcγRs were injected at a flow rate of 20 μl/min for 60 sec, followed by a pulse of regeneration solution (1M ethanol amine pH 8.5) for complete dissociation of receptor. The BSA-Flrscn surface was regenerated by a pulse injection of 10mM Glycine pH 1.5. Binding responses for each set of FcγRs were normalized to the captured level of wt antibody. The results of the analysis, shown in **Figure 5, Panels A-E,** indicate that the K288D / H435K Fc variant and the N434A Fc variant each exhibited enhanced binding to CD16A and substantially equivalent binding to CD32A and CD32B (compared to wild-type Fc ch4420). Fc variant H435K exhibited binding to CD16 and to CD32A and CD32B that was equivalent to that of wild-type Fc ch4420. The YTE mutant (triple substitution of M252Y, S254T, and T256E) exhibited decreased binding to CD16, CD32A and CD32B compared with wild-type Fc ch4420.

**Example 5**

Surface plasmon resonance analysis on a Biocore 3000 instrument was performed to analyze the ability of three different ch4420 mutants to bind to CD16A, CD32A and CD32B:

- MGFc316 - a 4D5 Fc variant having substitutions F243L, R292P, Y300L, V305I, and P396L in addition to H435K;
- MGFc317 - a 4D5 Fc variant having substitutions F243L, R292P, Y300L, V305I, and P396L in addition to both K288D and H435K; and
- MGFc318 - a 4D5 Fc variant having substitutions F243L, R292P and Y300L in addition to both K288D and H435K.

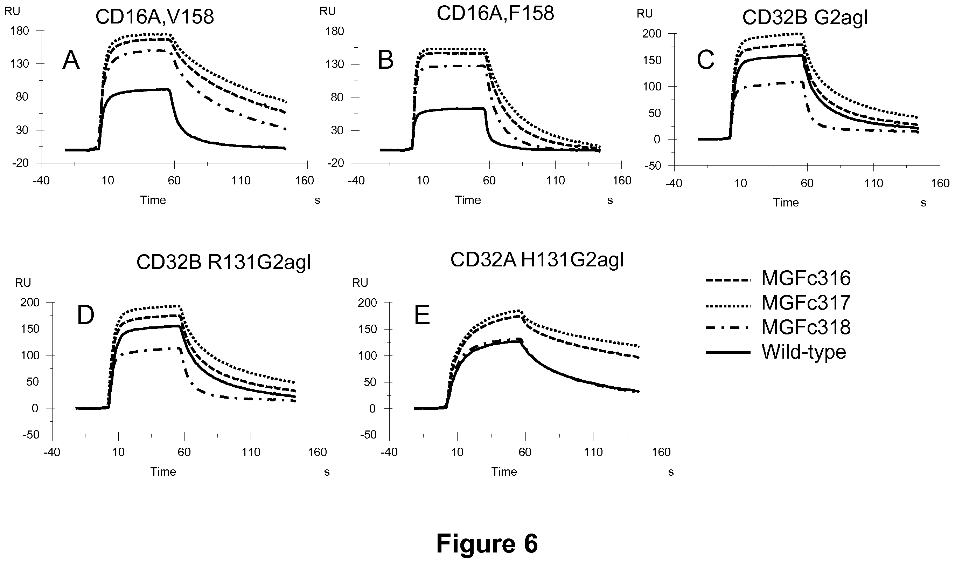

The results of the analysis, shown in **Figure 6, Panels A-E,** indicate that all three Fc variants exhibited enhanced binding to CD16A. MGFc316 and MGFc317 were found to exhibit enhanced binding to CD32A and CD32B. MGFc318 was found to exhibit decreased binding to CD32B and CD32A R131G2agl, and equivalent binding to CD32B H131G2agl.

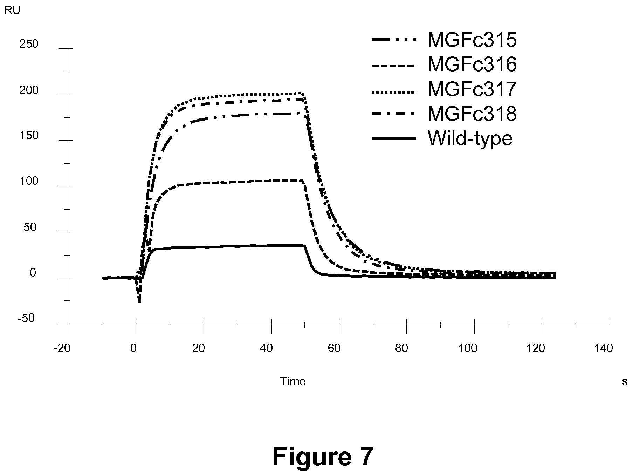

**Figure 7** depicts an SPR analysis of binding of these ch4420 mutants (and MGFc315 (K288D/H435K) to FcRn. ch4420 mutants are captured on a surface with immobilized protein-Flrscn; the SPR responses at pH 6.0 are normalized to same level of antibody. The results show that all four of the tested mutants exhibited enhanced binding to FcRn.

**Example 6**

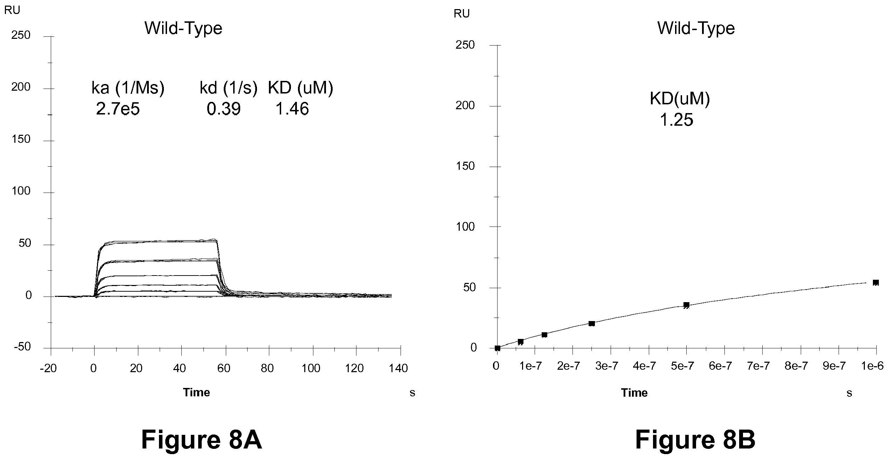

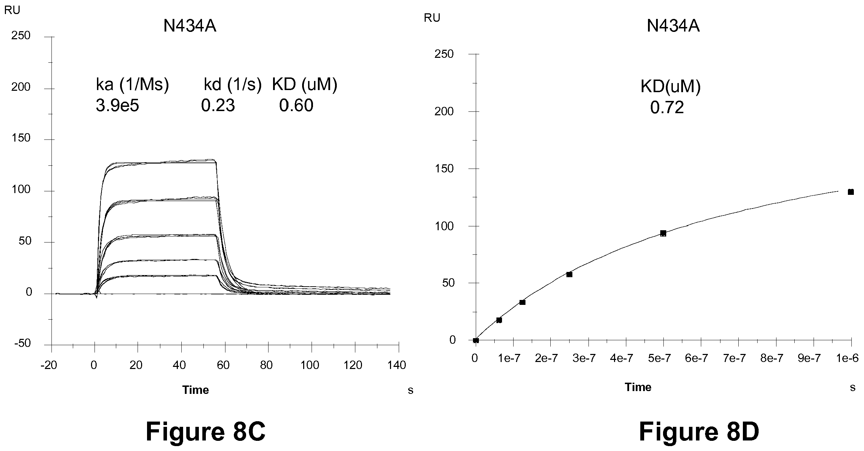

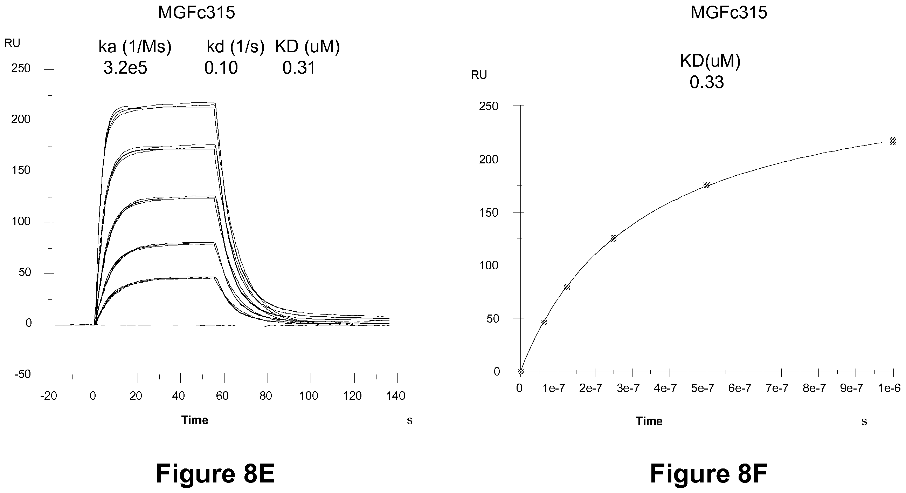

The kinetics of binding between FcRn and different ch4420 mutants was investigated. Human sFcRn was injected over ch4420 variants N434A and MGFc315 (K288D/H435K) captured at the level of approximately 1000 RU on immobilized mIgG1-Flrscn at a concentration range 0.63-1.00 μM. Buffer injection was subtracted as a blank. Binding responses were normalized to the same level of captured antibody and analyzed by Langmuir 1:1 (upper panel) and Steady state affinity (lower panel) models. The results of this investigation are shown for wild-type in **Figure 8A** (Langmuir 1:1 model) and **Figure 8B** (steady state affinity model), for ch4420 variant N434A in **Figure 8C** (Langmuir 1:1 model) and **Figure 8D** (steady state affinity model), and for MGFc315 in **Figure 8E** (Langmuir 1:1 model) and **Figure 8F** (steady state affinity model). The results show that the ch4420 variant MGFc315 had a lower KD than either N434A or wild-type Fc.

**Example 7**

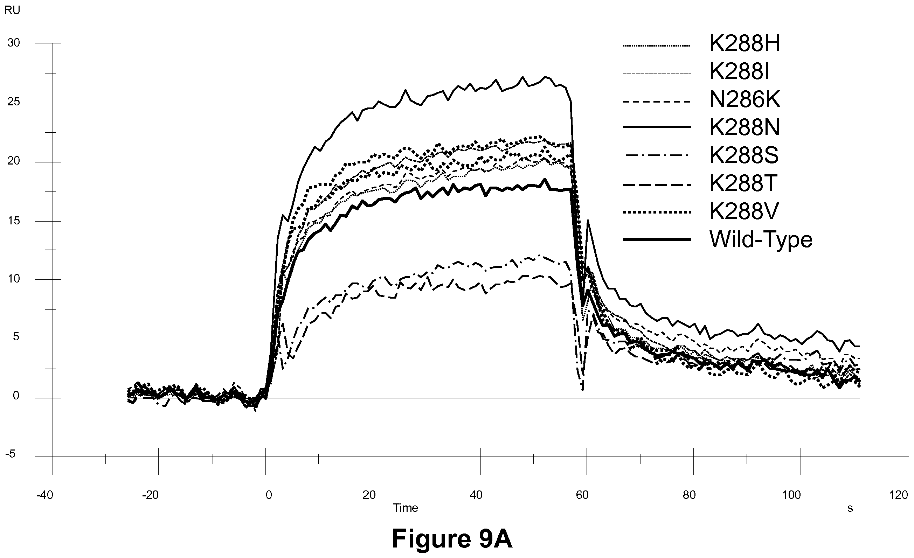

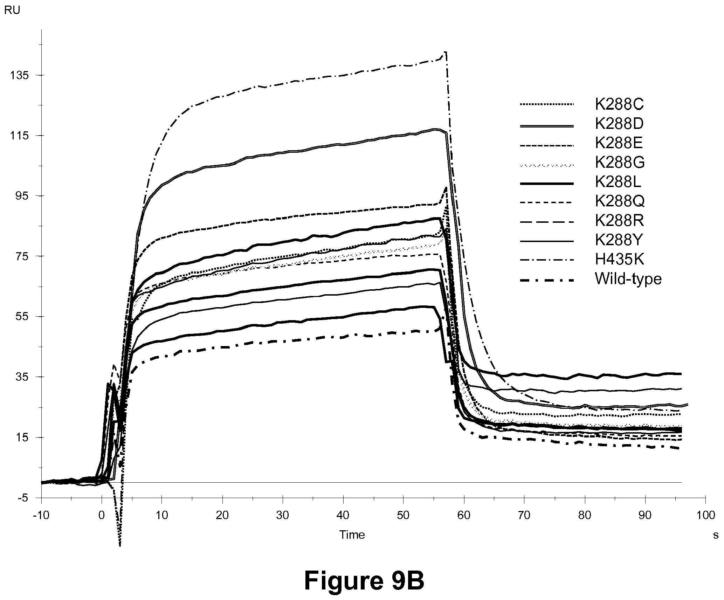

The ability of different K288 ch4420 variants to bind to FcRn was investigated. Variants in which K288 had been replaced by C, D, E, F, G, H, I, L, N, Q, R, S, T, V, W and Y were captured on a surface with immobilized protein-Flrscn and contacted with 500 nM hFcRn. The SPR binding analysis of these ch4420 mutants and of N286K, H435K and wild-type are shown in **Figure 9A** and **Figure 9B.**

**Claims**

1. A polypeptide comprising a variant Fc domain, wherein said variant Fc domain possesses an amino acid sequence that differs from the amino acid sequence of a wild-type Fc domain by comprising no more than 15 amino acid residue modifications relative to said wild-type Fc domain, said amino acid residue modifications comprising a substitution of a lysine at Kabat residue 435 and a substitution of aspartic acid at Kabat residue 288, wherein:

   i) said variant Fc domain is a variant human IgG1 Fc domain and said wild-type Fc domain is a human IgG1 Fc domain;
   ii) said variant Fc domain is a variant human IgG2 Fc domain and said wild-type Fc domain is a human IgG2 Fc domain;
   iii) said variant Fc domain is a variant human IgG3 Fc domain and said wild-type Fc domain is a human IgG3 Fc domain; or
   iv) said variant Fc domain is a variant human IgG4 Fc domain and said wild-type Fc domain is a human IgG4 Fc domain;

and wherein said variant Fc domain exhibits:

(A) enhanced binding affinity of said Fc domain of said polypeptide to human FcRn at least 2-fold relative to the binding affinity exhibited by a comparable molecule to human FcRn if comprising said wild-type Fc domain; or
(B) enhanced serum half-life of said polypeptide in humans of at least 1.5 times compared to the serum half-life of such polypeptide in humans if comprising said wild-type Fc domain.

2. The polypeptide of claim 1, wherein said variant Fc domain of said polypeptide:

(A) exhibits enhanced binding to human FcRn at a pH below 6.5, as compared to said wild-type Fc domain;
(B) exhibits higher binding affinity to human FcRn, as compared to said wild-type Fc domain; or
(C) enhances the serum half-life of said polypeptide.

3. The polypeptide of claim 1 or claim 2, wherein said polypeptide is an IgG1 heavy chain.

4. The polypeptide of any one of claims 1-3, wherein said variant Fc domain is a chimeric, humanized or variant human Fc domain.

5. The polypeptide of any one of claims 1-4, wherein said variant Fc domain of said polypeptide comprises at least one amino acid modification in addition to a lysine at Kabat residue 435 and an aspartic acid at Kabat residue 288.

6. The polypeptide of claim 5, wherein said additional modification comprises:

(A) at least one substitution selected from the group consisting of F243L, D270E, R292P, S298N, Y300L, V305I, A330V, and P396L;
(B) at least two substitutions selected from the group consisting of F243L and P396L; F243L and R292P; and R292P and V305I;
(C) at least three substitutions selected from the group consisting of F243L, R292P and Y300L; F243L, R292P and V305I; F243L, R292P and P396L; and R292P, V305I and P396L;
(D) at least four substitutions selected from the group consisting of F243L, R292P, Y300L and P396L; and F243L, R292P, V305I and P396L; or
(E) at least F243L, R292P, Y300L, V305I and P396L substitutions.

7. The polypeptide of any one of claims 1-6, wherein said amino acid modifications of said variant Fc domain alter effector function mediated by said Fc domain.

8. The polypeptide of claim 7, wherein the altered effector function is an enhanced antibody-dependent cell-mediated cytotoxicity (ADCC) function or an enhanced complement-dependent cytotoxicity (CDC) function.

9. The polypeptide of any one of claims 5-8, wherein said amino acid modifications to said variant Fc domain:

(A) increase binding of said Fc domain to an activating FcγR;
(B) increase binding of said Fc domain to FcγRIIB; or
(C) decrease binding of said Fc domain to FcγRIIB.

10. The polypeptide of any one of claims 1-9, wherein said polypeptide additionally comprises a therapeutic agent.

11. The polypeptide of claim 10, wherein said polypeptide comprises:

(A) a single chain antibody;
(B) a diabody; or
(C) a polypeptide chain of an antibody, or of an F(ab')$_2$ fragment or F(ab) fragment of an antibody.

12. The polypeptide of any one of claims 1-11, wherein said polypeptide binds a tumor antigen or a pathogen-related antigen.

13. The polypeptide of claim 12, wherein the tumor antigen is selected from the group consisting of 17-1A, αvβ3, AFP, BCR complex, CA125, CD3, CD18, CD20, CD22, CD33, CD44, CD52, CEA, CTLA-4, DNA-associated proteins, EGF receptor, Ep-CAM, GD2-ganglioside, gp IIIb/IIIa, gp72, HER2/neu, HLA-DR 10 beta, HLA-DR antigen, IgE, ganglioside GD3, MUC-1, nuC242, PEM antigen, SK-1 antigen, tumor antigen CA125, tumor antigen MUC1, VEGF, and VEGF-receptor; or wherein the pathogen-related antigen is a smallpox antigen, a West Nile Virus antigen, an anthrax antigen, a bacterial meningitis antigen, a cholera antigen, a *Clostridium difficile* antigen, a Lyme disease antigen, a *Pasteurella pestis* antigen, a pneumococcal antigen , a streptococcal antigen, a *Clostridium tetani* antigen, a micrococcal antigen, or a tularemia antigen.

14. A polynucleotide encoding the polypeptide of any one of claims 1-13.

15. Use of the polypeptide of claim 13 for the manufacture of a medicament for the treatment of cancer, wherein said polypeptide binds a tumor antigen.

16. The polypeptide of claim 13 for use in treating cancer, wherein said polypeptide binds a tumor antigen.

17. Use of the polypeptide of claim 13 for the manufacture of a medicament for the treatment of an infectious disease, wherein said polypeptide binds a pathogen-related antigen.

18. The polypeptide of claim 13 for use in treating or preventing an infectious disease, wherein said polypeptide binds a pathogen-related antigen.

19. A pharmaceutical composition comprising the polypeptide of any one of claims 1-13, and a pharmaceutically acceptable carrier.

**Patentansprüche**

1. Polypeptid, das eine abweichende Fc-Domäne umfasst, wobei die abweichende Fc-Domäne eine Aminosäuresequenz aufweist, die sich von der Aminosäuresequenz einer Wildtyp-Fc-Domäne unterscheidet, indem sie nicht mehr als 15 Aminosäurerest-Modifikationen verglichen mit der Wildtyp-Fc-Domäne umfasst, wobei die Aminosäurerest-Modifikationen eine Substitution eines Lysins an Kabat-Rest 435 und eine Substitution einer Asparaginsäure an Kabat-Rest 288 umfassen, wobei:

   i) es sich bei der abweichenden Fc-Domäne um eine abweichende menschliche IgG1-Fc-Domäne handelt und es sich bei der Wildtyp-Fc-Domäne um eine menschliche IgG1-Fc-Domäne handelt;
   ii) es sich bei der abweichenden Fc-Domäne um eine abweichende menschliche IgG2-Fc-Domäne handelt und es sich bei der Wildtyp-Fc-Domäne um eine menschliche IgG2-Fc-Domäne handelt;
   iii) es sich bei der abweichenden Fc-Domäne um eine abweichende menschliche IgG3-Fc-Domäne handelt und es sich bei der Wildtyp-Fc-Domäne um eine menschliche IgG3-Fc-Domäne handelt; oder
   iv) es sich bei der abweichenden Fc-Domäne um eine abweichende menschliche IgG4-Fc-Domäne handelt und es sich bei der Wildtyp-Fc-Domäne um eine menschliche IgG4-Fc-Domäne handelt;

   und wobei die abweichende Fc-Domäne zeigt:

   (A) eine wenigstens 2-fach erhöhte Bindungsaffinität der Fc-Domäne des Polypeptids für menschlichen FcRn, verglichen mit der Bindungsaffinität, die ein vergleichbares Molekül für menschlichen FcRn zeigt, wenn es die Wildtyp-Fc-Domäne umfasst; oder
   (B) eine wenigstens 1,5-fach erhöhte Serum-Halbwertszeit des Polypeptids bei Menschen, verglichen mit der Serum-Halbwertszeit eines solchen Polypeptids bei Menschen, wenn es die Wildtyp-Fc-Domäne umfasst.

2. Polypeptid nach Anspruch 1, wobei die abweichende Fc-Domäne des Polypeptids:

   (A) bei einem pH unterhalb von 6,5 eine gesteigerte Bindung an menschlichen FcRn zeigt, verglichen mit der Wildtyp-Fc-Domäne;
   (B) eine höhere Bindungsaffinität für menschlichen FcRn zeigt, verglichen mit der Wildtyp-Fc-Domäne; oder
   (C) die Serum-Halbwertszeit des Polypeptids erhöht.

3. Polypeptid nach Anspruch 1 oder 2, wobei es sich bei dem Polypeptid um eine IgG1-schwere-Kette handelt.

4. Polypeptid nach einem der Ansprüche 1-3, wobei es sich bei der abweichenden Fc-Domäne um eine chimäre, humanisierte oder abweichende menschliche Fc-Domäne handelt.

5. Polypeptid nach einem der Ansprüche 1-4, wobei die abweichende Fc-Domäne des Polypeptids wenigstens eine Aminosäuremodifikation zusätzlich zu einem Lysin an Kabat-Rest 435 und einer Asparaginsäure an Kabat-Rest 288 umfasst.

6. Polypeptid nach Anspruch 5, wobei die zusätzliche Modifikation umfasst:

   (A) wenigstens eine Substitution ausgewählt aus der Gruppe bestehend aus F243L, D270E, R292P, S298N, Y300L, V305I, A330V, und P396L;
   (B) wenigstens zwei Substitutionen ausgewählt aus der Gruppe bestehend aus F243L und P396L; F243L und R292P; sowie R292P und V305I;
   (C) wenigstens drei Substitutionen ausgewählt aus der Gruppe bestehend aus F243L, R292P und Y300L; F243L, R292P und V305I; F243L, R292P und P396L; sowie R292P, V305I und P396L;
   (D) wenigstens vier Substitutionen ausgewählt aus der Gruppe bestehend aus F243L, R292P, Y300L und P396L; sowie F243L, R292P, V305I und P396L; oder
   (E) wenigstens die Substitutionen F243L, R292P, Y300L, V305I und P396L.

7. Polypeptid nach einem der Ansprüche 1-6, wobei die Aminosäuremodifikationen der abweichenden Fc-Domäne die von der Fc-Domäne vermittelte Effektorfunktion verändern.

8. Polypeptid nach Anspruch 7, wobei es sich bei der veränderten Effektorfunktion um eine erhöhte Antikörperabhängige-zellvermittelte-Zytotoxizität(ADCC)-Funktion oder eine erhöhte Komplementabhängig-Zytotoxizität(CDC)-Funktion handelt.

9. Polypeptid nach einem der Ansprüche 5-8, wobei die Aminosäuremodifikationen der abweichenden Fc-Domäne:

   (A) die Bindung der Fc-Domäne an einen aktivierenden FcγR erhöhen;
   (B) die Bindung der Fc-Domäne an einen aktivierenden FcγRIIB erhöhen;
   (C) die Bindung der Fc-Domäne an einen aktivierenden FcγRIIB verringern.

10. Polypeptid nach einem der Ansprüche 1-9, wobei das Polypeptid zusätzlich ein Therapeutikum umfasst.

11. Polypeptid nach Anspruch 10, wobei das Polypeptid umfasst:

    (A) einen Einzelketten-Antikörper;
    (B) einen Diabody; oder
    (C) eine Polypeptidkette eines Antikörpers, oder eines F(ab')$_2$-Fragments oder F(ab)-Fragments eines Antikörpers.

12. Polypeptid nach einem der Ansprüche 1-11, wobei das Polypeptid ein Tumorantigen oder ein pathogenassoziiertes Antigen bindet.

13. Polypeptid nach Anspruch 12, wobei das Tumorantigen aus der Gruppe ausgewählt ist, die aus 17-1A, αvβ3, AFP, BCR-Komplex, CA125, CD3, CD18, CD20, CD22, CD33, CD44, CD52, CEA, CTLA-4, DNA-assoziierten Proteinen, EGF-Rezeptor, Ep-CAM, GD2-Gangliosid, gpIIb/IIIa, gp72, HER2/neu, HLA-DR 10-beta, HLA-DR-Antigen, IgE, Gangliosid GD3, MUC-1, nuC242, PEM-Antigen, SK-1-Antigen, Tumorantigen CA125, Tumorantigen MUC1, VEGF, und VEGF-Rezeptor besteht; oder wobei es sich bei dem pathogenassoziierten Antigen um ein Pockenantigen, ein West-Nil-Virus-Antigen, ein Anthrax-Antigen, ein Bakterielle-Meningitis-Antigen, ein Cholera-Antigen, ein *Clostridium-difficile-Antigen,* ein Lyme-Krankheit-Antigen, ein *Pasteurella-pestis*-Antigen, ein Pneumokokken-Antigen, ein Streptokokken-Antigen, ein *Clostridium-tetani*-Antigen, ein Mikrokokken-Antigen, oder ein Tularämie-Antigen handelt.

14. Polynukleotid, welches das Polypeptid nach einem der Ansprüche 1-13 codiert.

15. Verwendung des Polypeptids nach Anspruch 13 zur Herstellung eines Arzneimittels zur Behandlung von Krebs, wobei das Polypeptid ein Tumorantigen bindet.

**16.** Polypeptid nach Anspruch 13 zur Behandlung von Krebs, wobei das Polypeptid ein Tumorantigen bindet.

**17.** Verwendung des Polypeptids nach Anspruch 13 zur Herstellung eines Arzneimittels zur Behandlung einer Infektionskrankheit, wobei das Polypeptid ein pathogenassoziiertes Antigen bindet.

**18.** Polypeptid nach Anspruch 13 zur Behandlung einer Infektionskrankheit, wobei das Polypeptid ein pathogenassoziiertes Antigen bindet.

**19.** Pharmazeutische Zusammensetzung, die das Polypeptid nach einem der Ansprüche 1-13 umfasst, sowie einen pharmazeutisch unbedenklichen Trägerstoff.

**Revendications**

**1.** Polypeptide comprenant un domaine Fc variant, dans lequel ledit domaine Fc variant possède une séquence d'acides aminés qui diffère de la séquence d'acides aminés d'un domaine Fc de type sauvage en ce qu'elle ne comprend pas plus de 15 modifications de résidu d'acide aminé par rapport audit domaine Fc de type sauvage, lesdites modifications de résidu d'acide aminé comprenant une substitution d'une lysine au résidu Kabat 435 et une substitution d'acide aspartique au résidu Kabat 288, dans lequel :

i) ledit domaine Fc variant est un domaine Fc d'IgG1 humain variant et ledit domaine Fc de type sauvage est un domaine Fc d'IgG1 humain ;
ii) ledit domaine Fc variant est un domaine Fc d'IgG2 humain variant et ledit domaine Fc de type sauvage est un domaine Fc d'IgG2 humain ;
iii) ledit domaine Fc variant est un domaine Fc d'IgG3 humain variant et ledit domaine Fc de type sauvage est un domaine Fc d'IgG3 humain ; ou
iv) ledit domaine Fc variant est un domaine Fc d'IgG4 humain variant et ledit domaine Fc de type sauvage est un domaine Fc d'IgG4 humain ;

et dans lequel ledit domaine Fc variant présente :

(A) une affinité de liaison augmentée dudit domaine Fc dudit polypeptide pour FcRn humain d'au moins 2 fois par rapport à l'affinité de liaison présentée par une molécule comparable à FcRn humain si elle comprend ledit domaine Fc de type sauvage ; ou
(B) une demi-vie sérique augmentée dudit polypeptide chez des humains d'au moins 1,5 fois par rapport à la demi-vie sérique d'un tel polypeptide chez des humains s'il comprend ledit domaine Fc de type sauvage.

**2.** Polypeptide de la revendication 1, dans lequel ledit domaine Fc variant dudit polypeptide :

(A) présente une liaison augmentée à FcRn humain à un pH inférieur à 6,5, par comparaison avec ledit domaine Fc de type sauvage ;
(B) présente une affinité de liaison à FcRn humain plus élevée, par comparaison avec ledit domaine Fc de type sauvage ; ou
(C) augmente la demi-vie sérique dudit polypeptide.

**3.** Polypeptide de la revendication 1 ou la revendication 2, ledit polypeptide étant une chaîne lourde d'IgG1.

**4.** Polypeptide de l'une quelconque des revendications 1 à 3, dans lequel ledit domaine Fc variant est un domaine Fc chimérique, humanisé ou humain variant.

**5.** Polypeptide de l'une quelconque des revendications 1 à 4, dans lequel ledit domaine Fc variant dudit polypeptide comprend au moins une modification d'acide aminé en plus d'une lysine au résidu Kabat 435 et un acide aspartique au résidu Kabat 288.

**6.** Polypeptide de la revendication 5, dans lequel ladite modification supplémentaire comprend :

(A) au moins une substitution choisie dans le groupe constitué de F243L, D270E, R292P, S298N, Y300L, V305I, A330V et P396L ;

(B) au moins deux substitutions choisies dans le groupe constitué de F243L et P396L ; F243L et R292P ; et R292P et V305I ;

(C) au moins trois substitutions choisies dans le groupe constitué de F243L, R292P et Y300L ; F243L, R292P et V305I ; F243L, R292P et P396L ; et R292P, V305I et P396L ;

(D) au moins quatre substitutions choisies dans le groupe constitué de F243L, R292P, Y300L et P396L ; et F243L, R292P, V305I et P396L ; ou

(E) au moins les substitutions F243L, R292P, Y300L, V305I et P396L.

7. Polypeptide de l'une quelconque des revendications 1 à 6, dans lequel lesdites modifications d'acide aminé dudit domaine Fc variant modifient la fonction effectrice médiée par ledit domaine Fc.

8. Polypeptide de la revendication 7, dans lequel la fonction effectrice modifiée est une cytotoxicité à médiation cellulaire dépendante des anticorps (ADCC) augmentée ou une fonction de cytotoxicité dépendante du complément (CDC) augmentée.

9. Polypeptide de l'une quelconque des revendications 5 à 8, dans lequel lesdites modifications d'acide aminé dudit domaine Fc variant :

(A) augmentent la liaison dudit domaine Fc à un FcγR activateur ;

(B) augmentent la liaison dudit domaine Fc à FcγRIIB; ou

(C) diminuent la liaison dudit domaine Fc à FcγRIIB.

10. Polypeptide de l'une quelconque des revendications 1 à 9, ledit polypeptide comprenant en outre un agent thérapeutique.

11. Polypeptide de la revendication 10, ledit polypeptide comprenant :

(A) un anticorps monocaténaire ;

(B) un anticorps dimérique (diabody) ; ou

(C) une chaîne polypeptidique d'un anticorps, ou un fragment F(ab')$_2$ ou un fragment F(ab) d'un anticorps.

12. Polypeptide de l'une quelconque des revendications 1 à 11, ledit polypeptide se liant à un antigène tumoral ou un antigène associé à un agent pathogène.

13. Polypeptide de la revendication 12, dans lequel l'antigène tumoral est choisi dans le groupe constitué des 17-1A, αvβ3, AFP, complexe BCR, CA125, CD3, CD18, CD20, CD22, CD33, CD44, CD52, CEA, CTLA-4, protéines associées à l'ADN, récepteur d'EGF, Ep-CAM, ganglioside GD2, gp IIIb/IIIa, gp72, HER2/neu, HLA-DR 10-bêta, antigène HLA-DR, IgE, ganglioside GD3, MUC-1, nuC242, antigène PEM, antigène SK-1, antigène tumoral CA125, antigène tumoral MUC1, VEGF et récepteur de VEGF ; ou l'antigène associé à un agent pathogène est un antigène de la variole, un antigène du virus du Nil occidental, un antigène de l'anthrax, un antigène de la méningite bactérienne, un antigène du choléra, un antigène de *Clostridium difficile,* un antigène de la maladie de Lyme, un antigène de *Pasteurella pestis,* un antigène pneumococcique, un antigène streptococcique, un antigène de *Clostridium tetani,* un antigène micrococcique ou un antigène de la tularémie.

14. Polynucléotide codant pour le polypeptide de l'une quelconque des revendications 1 à 13.

15. Utilisation du polypeptide de la revendication 13 pour la fabrication d'un médicament pour le traitement du cancer, dans laquelle ledit polypeptide se lie à un antigène tumoral.

16. Polypeptide de la revendication 13 pour utilisation dans le traitement du cancer, ledit polypeptide se liant à un antigène tumoral.

17. Utilisation du polypeptide de la revendication 13 pour la fabrication d'un médicament pour le traitement d'une maladie infectieuse, dans laquelle ledit polypeptide se lie à un antigène associé à un agent pathogène.

18. Polypeptide de la revendication 13 pour utilisation dans le traitement ou la prévention d'une maladie infectieuse, ledit polypeptide se liant à un antigène associé à un agent pathogène.

19. Composition pharmaceutique comprenant le polypeptide de l'une quelconque des revendications 1 à 13, et un véhicule pharmaceutiquement acceptable.

**Figure 1**

EP 2 282 770 B1

**Figure 2**

EP 2 282 770 B1

**Figure 3**

**Figure 4**

Figure 5

Figure 6

**Figure 7**

**Figure 8A**

**Figure 8B**

Figure 8C

Figure 8D

EP 2 282 770 B1

**Figure 8E**

**Figure 8F**

**Figure 9A**

EP 2 282 770 B1

Figure 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1958615 A
- FR 2894982 A1
- US 952568 A
- US 20040185045 A
- US 20040197347 A
- US 20040197866 A
- US 20050037000 A
- US 20050064514 A
- US 20050215767 A
- US 20050260213 A
- US 20060013810 A
- US 20060134709 A
- US 20060177439 A
- US 20070004909 A
- US 20070036799 A
- US 20070037216 A
- US 20070077246 A
- US 20070244303 A
- US 20080044417 A
- US 20080044429 A
- US 20080050371 A
- US 20080095766 A
- US 20080112961 A
- US 7112439 B
- US 7351803 B
- US 7355008 B
- WO 05115452 A
- WO 05110474 A
- WO 06113665 A
- WO 04063351 A
- WO 06088494 A
- WO 06066078 A
- WO 04016750 A
- WO 05018669 A
- WO 07021841 A
- WO 08019199 A
- WO 08002933 A
- WO 2007106707 A
- WO 2008105886 A
- WO 2008140603 A
- US 5739277 A
- US 5624821 A
- US 5885573 A
- US 6194551 B
- US 7276586 B
- US 7317091 B
- WO 0042072 A
- WO 9958572 A
- US 6472511 B
- US 6218149 B
- US 20030115614 A
- US 20020028486 A
- EP 0359096 B1
- WO 03035835 A
- US 6602684 B
- US 20030157108 A
- US 20030003097 A
- WO 02311140 A
- WO 0230954 A
- WO 01292246 A
- WO 0061739 A
- WO 9002809 A
- WO 9110737 A
- WO 9201047 A
- WO 9218619 A
- WO 9311236 A
- WO 9515982 A
- WO 9520401 A
- US 5698426 A
- US 5223409 A
- US 5403484 A
- US 5580717 A
- US 5427908 A
- US 5750753 A
- US 5821047 A
- US 5571698 A
- US 5516637 A
- US 5780225 A
- US 5658727 A
- US 5733743 A
- US 5969108 A
- US 4816567 A
- WO 9308829 A
- US 5633425 A
- US 4347935 A
- US 5464581 A
- US 5483469 A
- US 5602039 A
- US 5643796 A
- US 6211477 B
- US 6373577 B
- US 6289286 B
- US 5322798 A
- US 5341215 A
- US 6268125 B
- US 5877396 A
- US 4980286 A
- US 5436146 A

- WO 9206180 A
- WO 9222635 A
- WO 9220316 A
- WO 9314188 A
- WO 9320221 A
- WO 9408598 A
- US 4957773 A
- US 4603044 A

- US 4235871 A
- US 4501728 A
- US 4837028 A
- US 4366241 A
- US 4376110 A
- US 4517288 A
- US 4837168 A

**Non-patent literature cited in the description**

- **LEACH et al.** *J. Immunology,* 1996, vol. 157, 3317
- **ISRAEL et al.** *Immunology,* 1997, vol. 92, 69
- **KOBAYASHI et al.** *Renal Physiol.,* 2002, vol. 282, F358
- **SPIEKERMAN et al.** *J. Exp. Med.,* 2002, vol. 196 (3), 303-310
- **YOSHIDA et al.** *Immunity,* 2004, vol. 20, 769-783
- **BITONTI et al.** *Proc. Natl. Acad. Sci. USA,* 2004, vol. 101, 9763-9768
- **WARD et al.** *International Immunology,* 2002, vol. 15 (2), 187
- **GHETIE et al.** *Eur. J. Immunology,* 1996, vol. 26, 690
- **JUNGHANS ; ANDERSON.** *Proc. Natl. Acad. Sci. USA,* 1996, vol. 93, 5512
- **ROOPENIAN et al.** *J. Immunology,* 2003, vol. 170, 3528
- **GHETIE ; WARD.** *Annu. Rev. Immunol.,* 2000, vol. 18, 739-66
- **RAGHAVAN et al.** *Immunity,* 1994, vol. 1 (4), 303-15
- **RAGHAVAN et al.** *Biochemistry,* 1995, vol. 34 (45), 14649-57
- **STORY et al.** *J. Exp. Med.,* 1994, vol. 180, 2377
- **GHETIE ; WARD.** *Immunology Today,* 1997, vol. 18 (12), 592-8
- **BURMEISTER et al.** *Nature,* 1994, vol. 372, 336
- **SIMISTER ; MOSTOV.** *Nature,* 1989, vol. 337, 184-7
- **RAGHAVAN et al.** *Immunity,* 1994, vol. 1, 303-15
- **MARTIN et al.** *Mol Cell,* 2001, vol. 7, 867-877
- **SAUER-ERIKSSON et al.** *Structure,* 1995, vol. 3, 265-278
- **TASHIRO et al.** *Curr Opin Struct Biol,* 1995, vol. 5, 471-481
- **BURMEISTER et al.** *Nature,* 1994, vol. 372, 336-379
- **KIM et al.** *Eur. J. Immunol.,* 1994, vol. 24, 2429-2434
- **MEDESAN et al.** *Eur. J. Immunol.,* 1996, vol. 26, 2533

- **MEDESAN et al.** *J. Immunol.,* 1997, vol. 158, 2211-2217
- **POPOV et al.** *Mol. Immunol.,* 1996, vol. 33, 493-502
- **GHETIE et al.** *Eur. J. Immunol.,* 1996, vol. 26, 690-696
- **JUNGHANS et al.** *Proc. Natl. Acad. Sci. USA,* 1996, vol. 93, 5512-55166
- **ISRAEL et al.** *Immunol.,* 1996, vol. 89, 573-578
- **BILLADEAU et al.** *J. Clin. Investigat.,* 2002, vol. 2 (109), 161-81
- **GERBER et al.** *Microbes Infection,* 2001, vol. 3, 131-139
- **RAVETCH et al.** *Annu. Rev. Immunol.,* 2001, vol. 19, 275-90
- **RAVETCH et al.** *Science,* 2000, vol. 290, 84-89
- **RAVETCH.** *Cell,* 1994, vol. 78 (4), 553-560
- **RAVETCH et al.** *Annu. Rev. Immunol.,* 1991, vol. 9, 457-492
- Immunobiology: The Immune System in Health and Disease. Elsevier Science Ltd/Garland Publishing
- **HULETT ; HOGARTH.** *Adv Immunol,* 1994, vol. 57, 1-127
- **OTT.** *J. Immunol.,* 2002, vol. 162 (9), 4430-4439
- **YAMANSHI et al.** *Cell,* 1997, vol. 88, 205
- **CARPINO et al.** *Cell,* 1997, vol. 88, 197
- **TRIDANDAPANI et al.** *J. Biol. Chem.,* 2002, vol. 277 (7), 5082-89
- **LONG.** *Annu Rev. Immunol,* 1999, vol. 17, 875
- **METCALFE et al.** *Physiol. Rev.,* 1997, vol. 77, 1033
- **BRAUWEILER et al.** *Journal of Immunology,* 2001, vol. 167 (1), 204-211
- **HIRANO et al.** *Nature Immunology,* 2007, vol. 8, 762-771
- **NIMMERJAHN et al.** *Immunity,* 2005, vol. 23, 41-51
- **MECHETINA et al.** *Immunogenetics,* 2002, vol. 54, 463-468
- **DAVIS et al.** *Immunol Rev,* 2002, vol. 190, 23-36

- **SHIELDS et al.** High resolution mapping of the binding site on human IgG1 for FcgammaRI, FcgammaRII, FcgammaRIII and FcRn and design of IgG1 variants with improved binding to the FcgammaR. *J. Biol. Chem., Am. Soc. Biochem. Biologist,* 2001, vol. 276 (9), 6591-6604
- Current Protocols in Molecular Biology. John Wiley & Sons, March 2008
- **SAMBROOK ; RUSSELL.** Molecular Cloning: A Laboratory Manual. 2001
- Single-Molecule Techniques: A Laboratory Manual. Cold Spring Harbor Press, 2008
- Current Protocols in Nucleic Acid Chemistry. John Wiley & Sons, Inc, 2000
- Current Protocols in Immunology. John Wiley & Sons
- Making and Using Antibodies: A Practical Handbook. CRC, 2006
- **HARLOW ; LANE.** Using Antibodies: A Laboratory Manual. Cold Spring Harbor Press, 1999
- **GOODRICH ; KUGEL.** Binding and Kinetics for Molecular Biologists. Cold Spring Harbor Press, 2007
- Current Protocols in Pharmacology. John Wiley & Sons, March 2008
- **GOODMAN ; GILMAN.** The Pharmacological Basis of Therapeutics. 2006
- Remington: The Science and Practice of Pharmacy. Lippincott Williams & Wilkins, 2005
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. (U.S.A.),* 1988, vol. 85, 2444
- **ALTSCHUL et al.** *J. Mol. Biol.,* 1990, vol. 215, 403-10
- **KABAT.** Sequences Of Proteins Of Immunological Interest. National Institutes of Health, 1987
- **CUNNINGHAM ; WELLS.** *Science,* 1989, vol. 244, 1081-1085
- **FLESCH ; NEPPERT.** *J. Clin. Lab. Anal.,* 1999, vol. 14, 141-156
- **CHAPPEL et al.** *J. Biol. Chem.,* 1993, vol. 33, 25124-25131
- **CHAPPEL et al.** *Proc. Natl. Acad. Sci. (U.S.A.),* 1991, vol. 88, 9036-9040
- **BRÜGGEMANN et al.** *J. Exp. Med,* 1987, vol. 166, 1351-1361
- **SONDERMANN et al.** *Nature,* 2000, vol. 406, 267-73
- **JEFFERIS et al.** *Immunol Lett,* 2002, vol. 82, 57-65
- **PRESTA et al.** *Biochem Soc Trans,* 2002, vol. 30, 487-90
- **IDUSOGIE et al.** *J Immunol,* 2001, vol. 166, 2571-75
- **SHIELDS et al.** *J Biol Chem,* 2001, vol. 276, 6591-6604
- **IDUSOGIE et al.** *J Immunol,* 2000, vol. 164, 4178-84
- **REDDY et al.** *J Immunol,* 2000, vol. 164, 1925-33
- **XU et al.** *Cell Immunol,* 2000, vol. 200, 16-26
- **ARMOUR et al.** *Eur J Immunol,* 1999, vol. 29, 2613-24
- **JEFFERIS et al.** *Immunol Lett,* 1996, vol. 54, 101-04
- **LUND et al.** *J Immunol,* 1996, vol. 157, 4963-69
- **HUTCHINS et al.** *Proc. Natl. Acad. Sci. (U.S.A.),* 1995, vol. 92, 11980-84
- **JEFFERIS et al.** *Immunol Lett.,* 1995, vol. 44, 111-17
- **LUND et al.** *FASEB J,* 1995, vol. 9, 115-19
- **ALEGRE et al.** *Transplantation,* 1994, vol. 57, 1537-43
- **LUND et al.** *Mol Immunol,* 1992, vol. 29, 53-59
- **LUND et al.** *J. Immunol,* 1991, vol. 147, 2657-62
- **DUNCAN et al.** *Nature,* 1988, vol. 332, 563-64
- **CARON et al.** *J. Exp Med.,* 1992, vol. 176, 1191-1195
- **B. SHOPES.** *J. Immunol.,* 1992, vol. 148, 2918-2922
- **WOLFF et al.** *Cancer Research,* 1993, vol. 53, 2560-65
- **STEVENSON et al.** *Anti-Cancer Drug Design,* 1989, vol. 3, 219-230
- **WANG et al.** *Chem. Comm.,* 2002, vol. 1, 1-11
- **WANG et al.** *Science,* 2001, vol. 292, 498-500
- **VAN HEST et al.** *Chem. Comm.,* 2001, vol. 19, 1897-1904
- **TANG et al.** *J. Am. Chem.,* 2001, vol. 123 (44), 11089-11090
- **KIICK et al.** *FEBS Lett.,* 2001, vol. 505 (3), 465
- **OKAZAKI et al.** *JMB,* 2004, vol. 336, 1239-1249
- **SHINKAWA et al.** *J Biol Chem,* 2003, vol. 278, 3466-3473
- **SHIELDS et al.** *J Biol Chem,* 2002, vol. 277, 26733-26740
- **DAVIES et al.** *Biotechnol Bioeng,* 2001, vol. 74, 288-294
- **UMANA et al.** *Nat. Biotechnol,* 1999, vol. 17, 176-180
- *Methods in Enzymology,* vol. 44, 1976
- Antibodies For Drug Delivery. **HELLSTROM et al.** Controlled Drug Delivery. Marcel Dekker, Inc, 1987, 623-53
- **MARVIN et al.** *Acta Pharmacol. Sin.,* 2005, vol. 26, 649-658
- **OLAFSEN et al.** *Prot. Engr. Des. Sel.,* 2004, vol. 17, 21-27

- **HOLLIGER et al.** *Proc. Natl. Acad. Sci. (U.S.A.),* 1993, vol. 90, 6444-6448
- **FITZGERALD et al.** *Protein Eng.,* 1997, vol. 10, 1221
- **STAERZ et al.** *Nature,* 1985, vol. 314, 628-31
- **HOLLIGER et al.** *Protein Eng.,* 1996, vol. 9, 299-305
- **HOLLIGER et al.** *Cancer Res.,* 1999, vol. 59, 2909-2916
- **CAO ; LAM.** *Adv. Drug. Deliv. Rev.,* 2003, vol. 55, 171-97
- **DAWSON et al.** *Ann. Rev Biochem.,* 2000, vol. 69, 923-960
- **WILKEN et al.** *Curr. Opin. Biotechnol.,* 1998, vol. 9 (4), 412-426
- **KOCHENDOERFER et al.** *Curr. Opin. Chem. Biol.,* 1999, vol. 3 (6), 665-671
- **WANG et al.** *IDrugs,* 2007, vol. 10 (8), 562-565
- **HAGEMEYER et al.** *Semin. Thromb. Hemost.,* 2007, vol. 33 (2), 185-195
- **RASMUSSEN et al.** *Biotechnol. Lett.,* 2007, vol. 29 (6), 845-852
- **GASSER et al.** *Biotechnol. Lett.,* 2007, vol. 29 (2), 201-212
- **AUBREY et al.** *J. Soc. Biol.,* 2006, vol. 200 (4), 345-354
- **LAFFLY et al.** *J. Soc. Biol.,* 2006, vol. 200 (4), 325-343
- **JEFFERIS.** *Biotechnol Prog.,* 2005, vol. 21 (1), 11-16
- **SMITH et al.** *J. Clin. Pathol.,* 2004, vol. 57 (9), 912-917
- **KIPRIYANOV et al.** *Mol Biotechnol.,* 2004, vol. 26 (1), 39-60
- **FISCHER et al.** *Vaccine,* 2003, vol. 21 (7-8), 820-825
- **MAYNARD et al.** *Ann. Rev. Biomed. Eng.,* 2000, vol. 2, 339-376
- **YOUNG et al.** *Res. Immunol.,* 1998, vol. 149 (6), 609-610
- **HUDSON.** *Curr. Opin. Biotechnol.,* 1998, vol. 9 (4), 395-402
- **ALDINGTON et al.** *J. Chromatogr. B Analyt. Technol. Biomed. Life Sci.,* 2007, vol. 848 (1), 64-78
- **S.S. FARID.** *J. Chromatogr. B Analyt. Technol. Biomed. Life Sci.,* 2006, vol. 848 (1), 8-18
- **BIRCH et al.** *Adv. Drug Deliv. Rev.,* 2006, vol. 58 (5-6), 671-685
- **EVEN et al.** *Trends Biotechnol.,* 2006, vol. 24 (3), 105-108
- **GRAUMANN et al.** *Biotechnol. J.,* 2006, vol. 1 (2), 164-86
- **BRINKMAN et al.** *J. Immunol. Methods,* 1995, vol. 182, 41-50
- **AMES et al.** *J. Immunol. Methods,* 1995, vol. 184, 177-86
- **KETTLEBOROUGH et al.** *Eur. J. Immunol.,* 1994, vol. 24, 952-58
- **PERSIC et al.** *Gene,* 1997, vol. 187, 9-18
- **BURTON et al.** *Advances in Immunology,* 1994, vol. 57, 191-280
- **GLASER et al.** *J. Immunology,* 1992, vol. 149, 3903
- **WU et al.** *Proc. Natl. Acad. Sci. (U.S.A.),* 1998, vol. 95, 6037
- **YELTON et al.** *J. Immunology,* 1995, vol. 155, 1994
- **SCHIER et al.** *J. Mol. Bio.,* 1996, vol. 263, 551
- **KOHLER et al.** *Nature,* 1975, vol. 256, 495
- **KOZBOR et al.** *Immunology Today,* 1983, vol. 4, 72
- **COLE et al.** Monoclonal Antibodies and Cancer Therapy. Alan R. Liss, Inc, 1985, 77-96
- **CLACKSON et al.** *Nature,* 1991, vol. 352, 624-628
- **MARKS et al.** *J. Mol. Biol.,* 1991, vol. 222, 581-597
- **MILSTEIN et al.** *Nature,* 1983, vol. 305, 537-39
- **TRAUNECKER et al.** *EMBO J.,* vol. 10, 3655-59
- **LONBERG ; HUSZAR.** *Int. Rev. Immunol.,* 1995, vol. 13, 65-93
- **HOOGENBOOM ; WINTER.** *J. Mol. Biol.,* 1991, vol. 227, 381
- **MARKS et al.** *J. Mol. Biol.,* 1991, vol. 222, 581
- **JESPERS et al.** *Biotechnology,* 1994, vol. 12, 899-903
- **HOUGHTEN.** *Bio/Techniques,* 1992, vol. 13, 412-421
- **LAM.** *Nature,* 1991, vol. 354, 82-84
- **FODOR.** *Nature,* 1993, vol. 364, 555-556
- **CULL et al.** *Proc. Natl. Acad. Sci. (U.S.A.),* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci. (U.S.A.),* 1990, vol. 87, 6378-6382
- **FELICI.** *J. Mol. Biol.,* 1991, vol. 222, 301-310
- **AUSUBEL et al.** *Current Protocols in Molecular Biology,* 2008
- **DONG et al.** *Review in Mol. Biotech.,* 2002, vol. 82, 303-323
- **MULLET et al.** *Methods,* 2000, vol. 22, 77-91
- **RICH et al.** *Current Opinion in Biotechnology,* 2000, vol. 11, 54-61
- **FIVASH et al.** *Current Opinion in Biotechnology,* 1998, vol. 9, 97-101
- **MYSZKA.** *Current Opinion in Biotechnology,* 1997, vol. 8, 50-57
- **O'SHANNESSY et al.** *Analytical Biochemistry,* 1996, vol. 236, 275-283

- **MORTON et al.** *Analytical Biochemistry,* 1995, vol. 227, 176-185
- **FISHER et al.** *Current Opinion in Biotechnology,* 1994, vol. 5, 389-95
- **O'SHANNESSY.** *Current Opinion in Biotechnology,* 1994, vol. 5, 65-71
- **CHAIKEN et al.** *Analytical Biochemistry,* 1992, vol. 201, 197-210
- **ABDUL-MAJID et al.** *Scand. J. Immunol.,* 2002, vol. 55, 70-81
- **PERUSSIA et al.** *Methods Mol. Biol.,* 2000, vol. 121, 179-192
- **LEHMANN et al.** *J. Immunol. Methods,* 2000, vol. 243 (1-2), 229-242
- **DING et al.** *Immunity,* 1998, vol. 8, 403-411
- **BAGGIOLINI et al.** *Experientia,* 1998, vol. 44 (10), 841-848
- **BROWN.** *Methods Cell Biol.,* 1994, vol. 45, 147-164
- **MUNN et al.** *J. Exp. Med.,* 1990, vol. 172, 231-237
- **TRIDANDAPANI et al.** *J. Biol. Chem.,* 2000, vol. 275, 20480-20487
- **BEDZYK et al.** *J. Biol. Chem.,* 1989, vol. 264 (3), 1565-1569
- **GAZZANO-SANTORO et al.** *J. Immunol. Methods,* 1996, vol. 202, 163
- **WENG et al.** *J. Clin. Oncol.,* 2003, vol. 21, 3940-3947
- **BLOMBERG et al.** *Journal of Immunological Methods,* 1996, vol. 193, 199-206
- **TREMP et al.** *Cancer Res.,* 1976, 33-41
- **EPSTEIN et al.** *J. Natl. Cancer Inst.,* 1965, vol. 34, 231-240
- **KLEIN et al.** *Cancer Res.,* 1968, vol. 28, 1300-1310
- **CARTON et al.** *Blood,* 2002, vol. 99, 754-758
- **WENG et al.** *J Clin Oncol.,* 2003, vol. 21 (21), 3940-3947
- **WU ; WU.** *J. Biol. Chem.,* 1987, vol. 262, 4429-4432
- **HIDALGO-ARAGONES.** *J. Steroid Biochem. Mol. Biol.,* 1996, vol. 58, 611-617
- **GRONING.** *Pharmazie,* 1996, vol. 51, 337-341
- **FOTHERBY.** *Contraception,* 1996, vol. 54, 59-69
- **JOHNSON.** *J. Pharm. Sci.,* 1995, vol. 84, 1144-1146
- **ROHATAGI.** *Pharmazie,* 1995, vol. 50, 610-613
- **BROPHY.** *Eur. J. Clin. Pharmacol.,* 1983, vol. 24, 103-108
- **KAMAT et al.** *Cancer Research,* 2007, vol. 67, 281-88
- Arthritis and Autoimmunity in Animals. **CROFFORD L.J. ; WILDER R.L. et al.** Arthritis and Allied Conditions: A Textbook of Rheumatology. Lee and Febiger, 1993
- **WINTER et al.** *Proc. Soc. Exp. Biol Med.,* 1962, vol. 111, 544-47
- **HANSRA et al.** *Inflammation,* 2000, vol. 24 (2), 141-155
- **STROBER.** *Dig. Dis. Sci.,* 1985, vol. 30 (12), 3S-10S
- **KIM et al.** *Scand. J. Gastroentrol.,* 1992, vol. 27, 529-537
- **FLANDERS et al.** *Autoimmunity,* 1999, vol. 29, 235-246
- **KROGH et al.** *Biochimie,* 1999, vol. 81, 511-515
- **FOSTER.** *Semin. Nephrol.,* 1999, vol. 19, 12-24
- Relevance of Tumor Models for Anticancer Drug Development. 1999
- **KARGER.** *Contributions to Oncology,* 1999
- The Nude Mouse in Oncology Research. 1991
- Anticancer Drug Development Guide. 1997
- **GOLDSPIEL et al.** *Clinical Pharmacy,* 1993, vol. 12, 488-505
- **WU ; WU.** *Biotherapy,* 1991, vol. 3, 87-95
- **TOLSTOSHEV.** *Ann. Rev. Pharmacol. Toxicol.,* 1993, vol. 32, 573-596
- **MULLIGAN.** *Science,* 1993, vol. 260, 926-932
- **MORGAN ; ANDERSON.** *Ann. Rev. Biochem.,* 1993, vol. 62, 191-217
- **KOLLER ; SMITHIES.** *Proc. Natl. Acad. Sci. (U.S.A.),* 1989, vol. 86, 8932-35
- **ZIJLSTRA et al.** *Nature,* 1989, vol. 342, 435-438
- **MILLER et al.** *Meth. Enzymol.,* 1993, vol. 217, 581-599
- **SALMONS ; GUNZBERG.** *Human Gene Therapy,* 1993, vol. 4, 129-141
- **GROSSMAN ; WILSON.** *Curr. Opin. in Genetics and Devel.,* 1993, vol. 3, 110-114
- **KOZARSKY ; WILSON.** *Current Op. in Genetics and Dev.,* 1993, vol. 3, 499-503
- **WALSH et al.** *Proc. Soc. Exp. Biol. Med.,* 1993, vol. 204, 289-300
- **BOUT et al.** *Human Gene Therapy,* 1994, vol. 5, 3-10
- **BOESEN et al.** *Biotherapy,* 1994, vol. 6, 291-302
- **CLOWES et al.** *J. Clin. Invest.,* 1994, vol. 93, 644-651
- **KLEIN et al.** *Blood,* 1994, vol. 83, 1467-1473
- **JOLIOT et al.** *Proc. Natl. Acad. Sci. (U.S.A.),* 1991, vol. 88, 1864-1868
- **RHEINWALD.** *Meth. Cell Bio.,* 1980, vol. 21A, 229
- **PITTELKOW ; SCOTT.** *Mayo Clinic Proc.,* 1986, vol. 61, 771

- **STEMPLE ; ANDERSON.** *Cell,* 1992, vol. 7 (1), 973-985
- **MASTROBATTISTA et al.** *Advanced Drug Delivery Reviews,* 1999, vol. 40, 103-127
- **RENNEISEN et al.** *J. Biol. Chem.,* 1990, vol. 265, 16337-16342
- **LEONETTI et al.** *Proc. Natl. Acad. Sci. (U.S.A.),* 1990, vol. 87, 2448-2451
- **SZOKA et al.** *Ann. Rev. Biophys. Bioeng.,* 1980, vol. 9, 467
- Methods in Cell Biology. Academic Press, 1993, vol. 37
- Basic and Clinical Immunology. 1991
- **W.F. et al.** Increasing The Affinity Of A Human Igg1 For The Neonatal Fc Receptor: Biological Consequences. *J. Immunol.,* 2002, vol. 169 (9), 5171-5180
- **PETKOVA, S.B. et al.** Enhanced Half-Life Of Genetically Engineered Human Igg1 Antibodies In A Humanized Fcrn Mouse Model: Potential Application In Humorally Mediated Autoimmune Disease. *Int. Immunol.,* 31 October 2006, vol. 18 (12), 1759-1769